# EUROPEAN PATENT APPLICATION

(11) **EP 2 567 969 A1**
(43) Date of publication of application: **13.03.2013**
(21) Application number: 12182424.7
(22) Date of filing: 22.09.2008
(51) Int. Cl.: C07K 14/20

(54) **Immunogenic proteins from genome-derived outer membrane of Leptospira and compositions and methods based thereon**

(30) Priority: 24.09.2007 US 974818 P; 28.09.2007 US 976088 P
(62) Divisional of application: 08834647.3
(71) Applicant: Cornell University, Ithaca, NY 14850 (US)
(72) Inventor: Chang, Yung-fu, Ithaca, NY New York 14850 (US)
(74) Representative: Huebner, Stefan Rolf

(57) **Abstract**

*Leptospira* outer membrane protein (OMP) MCEII is provided. The OMP can be used as a tool for developing effective vaccines or diagnostic methods for leptospirosis. Expression vectors for the OMP gene are further provided. The antigenic properties of the *Leptospira* OMP can be used to create, manufacture or improve vaccines. Vaccines, including but not limited to DNA vaccines, recombinant vaccines, and T-cell epitope vaccines based on the foregoing OMP are also provided. Methods for producing such vaccines are also provided. Also provided are methods for using *Leptospira* OMP genes, proteins and antibodies for therapeutic treatment and serological diagnosis techniques.

## Description

### Cross-Reference to Related Applications

This application claims priority to and the benefit of co-pending U.S. provisional patent applications Serial No. 60/974,818 (filed September 24,2007) and Serial No. 60/976,088 (filed September 28,2007), both of which are incorporated herein by reference in their entireties.

### Statement Regarding Federally Sponsored Research or Development

Not applicable

### Reference to Appendix

Not applicable

### 1. TECHNICAL FIEND

The present invention relates to *Leptospira* outer membrane proteins (OMPs) and antibodies directed against them. The invention also relates to methods and compositions for the treatment of diseases or disorders caused by a blood-borne immunogenic *Leptospira* pathogen, comprising administering antibodies that bind to OMPs. The invention further relates to methods for using *Leptospira* OMPs as vaccines or diagnostic antigens.

### 2. BACKGROUND

*Leptospira* species are a gram negative spirochete, the causative agent of leptospirosis, a zoonotic disease of mammals characterized by renal and liver failure, abortion, uveitis, reproductive failure, myocarditis, mastitis and pulmonary hemorrhage (Kishimoto, et al. 2004. Leptospirosis misdiagnosed as pulmonary-renal syndrome. Am. J. Med. Sci. 328:116-120; Marotto, et al. 1999. Acute lung injury in leptospirosis: clinical and laboratory features, outcome, and factors associated with mortality. Clin. Infect. Dis. 29:1561-1563; Seijo, et al, 2002. Lethal leptospiral pulmonary hemorrhage: an emerging disease in Buenos Aires, Argentina. Emerg. Infect. Dis. 8:1004-1005; Trevejo, et al. 1998. Epidemic leptospirosis associated with pulmonary hemorrhage-Nicaragua. 1995. J. Infect. Dis. 178:1457-1463; Vinetz, J. M. 2001. Leptospirosis. Curr. Opin. Infect, Dis. 14:527-538).

Although there are 21 genomospecies of *Leptospira*, the most common cause of leptospirosis among cattle, especially in dairy cows in the United States, is *L. borgpeiersenii* serovar Hardjo, and *L. interrogans* serovar Pomona (Talpada, M. D., N. Garvey, R. Sprowls, A. K. Eugster, and J. M. Vinetz. 2003. Prevalence of leptospiral infection in Texas cattle: implications for transmission to humans. Vector Borne Zoonotic Dis. 3:141-147). Cattle act as the maintenance host for this infection and in chronically infected cattle, leptospires persist in the kidneys and genital tract, leading to abortion (Ellis, W. A., and S. W. Michna. 1976. Bovine leptospirosis: demonstration of leptospires of the Hebdomadis serogroup in aborted fetuses and a premature calf. Vet. Rec. 99:430-432; Ellis, W. A., J. J. O'Brien, D. G. Bryson, and D. P.Mackie. 1985. Bovine leptospirosis: some clinical features of serovar Hardjo infection. Vet. Rec. 117:101-104) and renal failure (Masri, S. A., P. T. Nguyen, S. P. Gale, C. J. Howard, and S. C. Jung. 1997. A polymerase chain reaction assay for the detection of Leptospira spp. in bovine semen. Can. J. Vet. Res. 61:15-20). *Leptospira* species also infects pigs, horses, dogs and humans (Palaniappan, R.U.M.; Ramanujam, S.; Chang, Y-F. (2007) Leptospirosis: pathogenesis, immunity, and diagnosis. Curr. Opin. Infect. Dis. 20(3): 284-292). Infected animals may also survive asymptomtatically by excreting the spirochetes intermittently in the urine, eventually affecting the other healthy animals.

Diagnosis of leptospirosis depends on a standard serological test, the microscopic agglutination test (MAT). However, MAT is time consuming, laborious and lack sensitivity to detect the infection at an early stage. Serological tests tor diagnosis such as dipstick assay, indirect hemagglutination assay and enzyme linked immunosorbent assay (ELISA) are alternative methods for rapid diagnosis but only few assays have been systematically evaluated based on the MAT titers (Palaniappan, et al. 2004. Expression of leptospiral immunoglobulin-like protein by Leptospira interrogans and evaluation of its diagnostic potential in a kinetic ELISA. J. Med. Microbiol. 53:975-984). This information is crucial for many diagnostic laboratories to choose the suitable assay for diagnosis of leptospirosis.

Commercially available monovalent or pentavalent vaccines provide only short-term immunity and are ineffective against increasing serovars of *Leptospira.* Recently, a new vaccine containing whole cell proteins of *L. borgpetersenii* serovar Hardjo has been found to protect even the transmission of infection from mother to fetus in cattle (Ribeiro, M. A., C. C. Souza, and S. H. Almeida. 1995. Dot-ELISA for human leptospirosis employing immunodominant antigen. J. Trop. Med. Hyg. 98:452-456; Smith, C. R., P. J. Ketterer, M. R. McGowan, and B. G. Corney. 1994. A review of laboratory techniques and their use in the diagnosis of Leptospira interrogans serovar Hardjo infection in cattle. Aust. Vet. J. 71:290-294). Notably, these vaccines increase the antibody titer against leptospiral antigens in animals, and this increase cannot be differentiated by the standard microscopic agglutination test (MAT). Furthermore, if these vaccinated animals are also subjected to infection from other serovars of *Leptospira,* distinguishing vaccination from infection using MAT is impossible.

There is therefore a need in the art for a vaccine for leptospirosis. There is also a need for distinguishing leptospirosis vaccination from infection.

Citation or identification of any reference in Section 2, or in any other section of this application, shall not be considered an admission that such reference is available as prior art to the present invention.

### 3. SUMMARY OF THE INVENTION

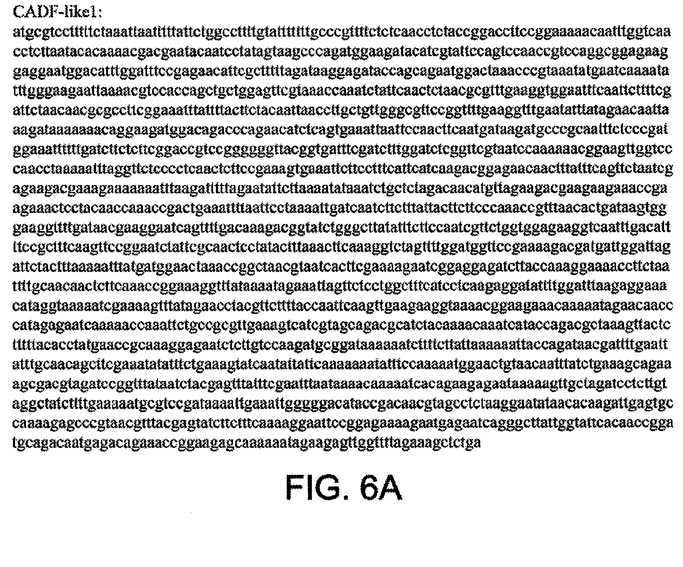

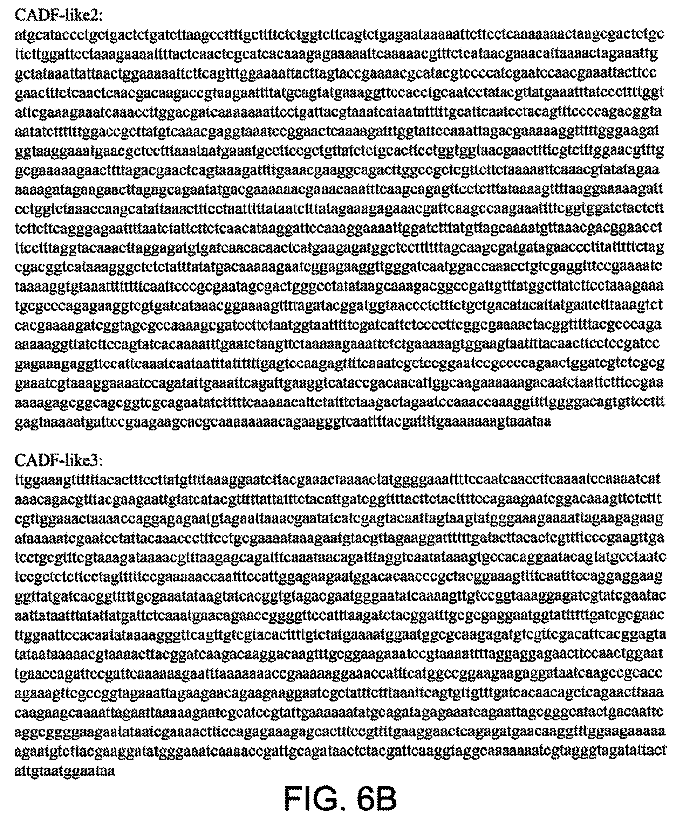

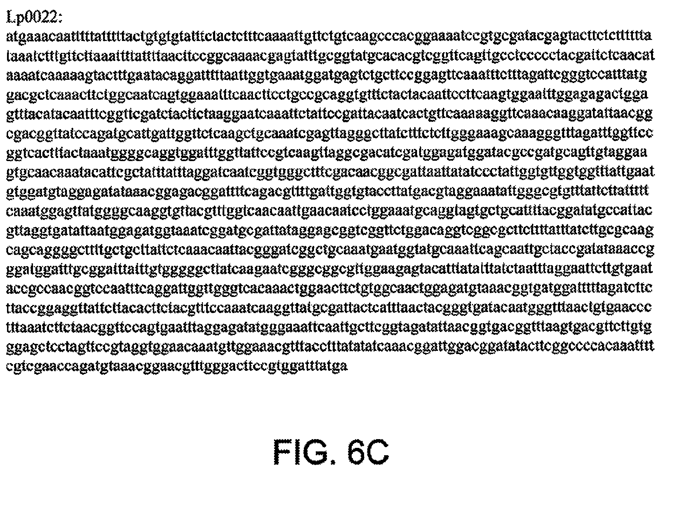

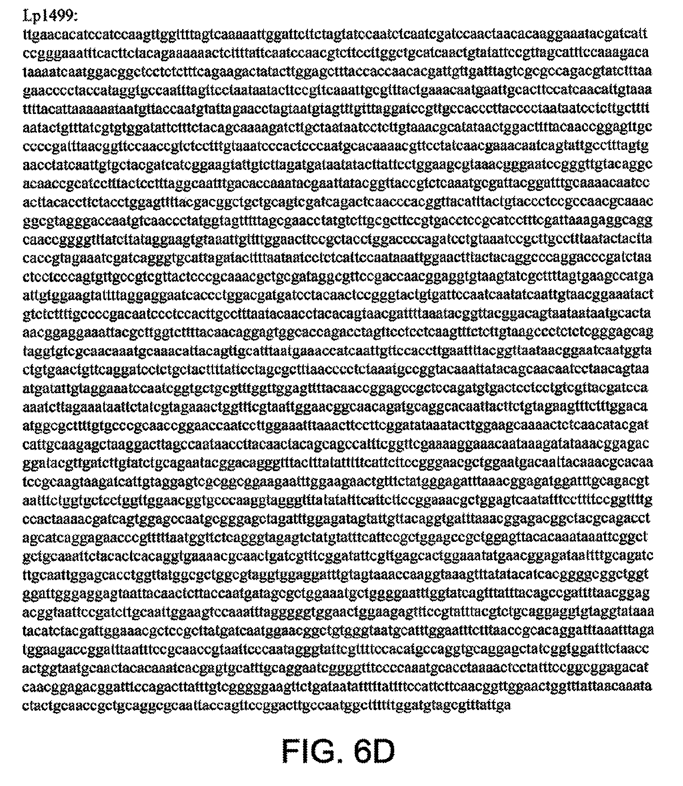

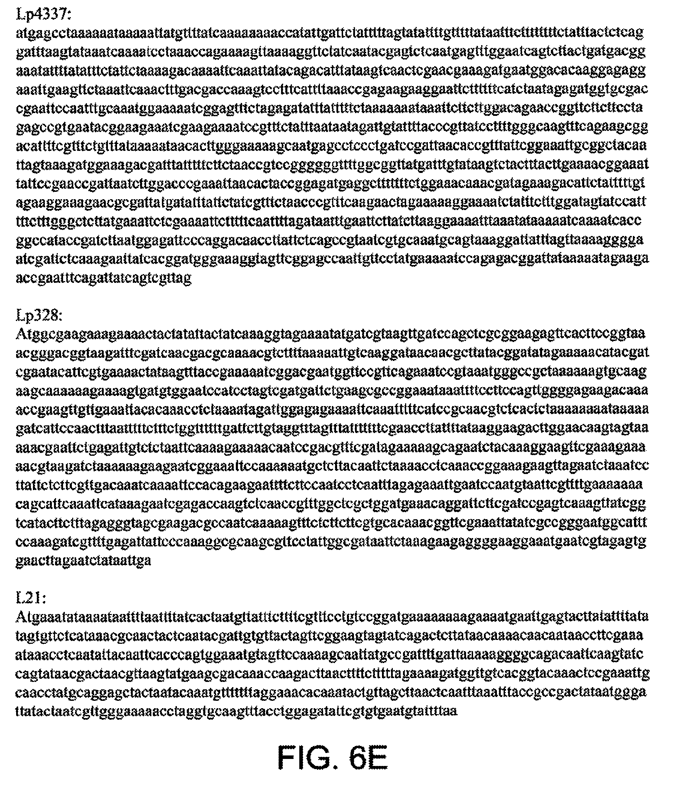

*Leptospira* outer membrane proteins (OMPs) rLP1454 (SEQ ID NOS: 1-2), rLP1118 (SEQ ID NOS: 3-4), rLP1939 (SEQ ID NOS: 5-6), rMCEII (Lp0607; SEQ ID NOS: 7-8), CADF-like1. (SEQ ID NOS: 52-53; FIG. 6A), CADF-like2 (SEQ ID NOS: 54-55; FIG. 6B), CADF-like3 (SEQ ID NOS: 56-57; FIG. 6B), Lp0022 (SEQ ID NOS_{:} 58-59; FIG. 6C), Lp1499 (SEQ ID NOS: 60-61; FIG. 6D), Lp4337 (SEQ ID NOS: 62-63; FIG. 6E), Lp328 (SEQ ID NOS: 64-65; FIG. 6E) and L21 (SEQ ID NOS: 66-67; FIG. 6E) are provided that can serve as tools for developing effective vaccines or diagnostic methods for leptospirosis. In one embodiment, protein fragments of at least 10%, 20%, 30%, 40%, 50%, 60% 70%, 80%, 90% or 95% of the length of the amino acid sequence of any of the above are provided.

The antigenic properties of the *Leptospira* LP1454, LP1118*,* LP1939, MCEII, CADF-like1, CADF-like2, CADF-like3, Lp0022, Lp1499, Lp4337, Lp328 or L21 OMPs can be used to create, manufacture or improve vaccines. Vaccines, including but not limited to DNA vaccines, recombinant vaccines, and T-cell epitope vaccines based on the foregoing OMPs are further provided. Methods for producing such vaccines are also provided.

In one embodiment the vaccine is based on a *Leptospira* outer membrane protein (OMP) selected from the group consisting of rLP1454 (SEQ ID NOS: 1-2), rLP1118 (SEQ ID NOS: 3-4), rLP1939 (SEQ ID NOS: 5-6), rMCEII (Lp0607: SEQ ID NOS: 7-8), CADF-like1 (SEQ ID) NOS: 52-53; FIG. 6A), CADF-like2 (SEQ ID NOS: 54-55; FIG. 6B), CADF-like3 (SEQ ID NOS: 56-57; FIG. 6B), Lp0022 (SEQ ID NOS: 58-59; FIG. 6C), Lp1499 (SEQ ID NOS: 60-61; FIG. 6D), Lp4337 (SEQ ID) NOS: 62-63, FIG. 6E). Lp328 (SEQ ID NOS: 64-65; FIG. 6E) and L21 (SEQ ID NOS: 66-67; FIG. 6E) OMP.

Methods are provided for using *Leptospira* OMP genes, proteins and antibodies for serological diagnosis techniques.

A plasmid is also provided comprising a recombinant DNA encoding a LP1454, LP1118, LP1939. MCEII, CADF-like1, CADF-like2, CADF-like3, Lp0022, Lp1499, Lp4337, Lp328 or L21 OMP or an epitope of a conserved variant thereof, or an immunologically reactive peptide fragment thereof.

An expression vector is also provided.

Immunogenic epitopes of *Leptospira* LP1454, LP1118, LP1939, MCEII, CADF-like1, CADF-like2, CADF-lik;3 , Lp0022, Lp1499, Lp4337, Lp328 and L21 OMPs, or an epitope of a conserved variant thereof, or an immunologically reactive peptide fragment thereof are also provided.

Methods for using the immunogenic epitope (e.g., a T-cell epitope) as a vaccine (or to improve a vaccine) for mammals, including but not limited to cow, pig, dog, sheep and horse, are provided.

Methods for using the immunogenic epitope (e.g., a T-cell epitope) as a serological diagnostic antigen are also provided. In one aspect, the epitope is specifically recognized by an antibody.

Vaccine formulations for pharmacological administration are also provided.

Vaccine formulations can be DNA vaccine formulations, recombinant vaccine formulations, or T-cell epitope vaccine formulations.

Isolated and purified antibodies to *Leptospira* OMPs LP1454. LP1118, LP1939, MCEII, CADF-like1, CADF-like2, CADF-like3, Lp0022, Lp1499. Lp4337, Lp328 and L21, or to an immunogenic epitope (e.g., a T-cell epitope), epitope of a conserved variant, or an immunologically reactive peptide fragment thereof are also provided. Such antibodies include, but are not limited to, polyclonal antibodies, monoclonal antibodies (mAbs), human, humanized or chimeric antibodies, single chain antibodies, Fab fragments, F(ab')₂ fragments, fragments produced by a Fab expression library, anti-idiotypic (anti-Id) antibodies and epitope-binding fragments of any of the above.

In one embodiment, an isolated and purified antibody of the invention can specifically recognize one or more epitopes of *Leptospira* LP1454, LP1118, LP1939, MCEII, CADF-like1, CADF-like2, CADF-like3, Lp0022, Lp1499, Lp4337, Lp328 and L21, or epitopes of conserved variants or immunologically reactive peptide fragments thereof.

The isolated and purified antibody to the foregoing *Leptospira* OMPs can be conjugated to moieties including but not limited to a targeting agent, a label, a carrier, a drug, a toxin and a solid support.

A hybridoma or immortalized cell line is also provided that secretes a monoclonal antibody that can specifically recognize one or more epitopes of *Leptospira* LP1454, LP1118, LP1939, MCEII, CADF-like1, CADF-like2, CADF-like, Lp0022, Lp1499, Lp4337, Lp328 and L21. or an epitope of a conserved variant or immunologically reactive peptide fragment thereof.

Methods are provided for producing antibodies that specifically recognize one or more epitopes of *Leptospira* LP1454, LP1118, LP1939, MCEII, CADF-like1, CADF-like2, CADF-like3, Lp0022, Lp1499, Lp4337, Lp328 and L21, or an epitope of a conserved variant or immunologically reactive peptide fragment thereof.

In one embodiment, the method can comprise immunizing a host animal by injection with an OMP protein or peptide, wherein the OMP protein is an *Leptospira* LP1454, LP1118, LP1939, MCEII, CADF-like1, CADF-like2, CADF-like3, Lp0022, Lp1499, Lp4337, Lp328 and L21 peptide (*e.g*., one corresponding to a functional domain of the protein), a truncated polypeptide thereof (in which one or more domains has been deleted), a functional equivalent thereof, or a mutant thereof.

In another embodiment, the method can comprise producing a hybridoma or immortalized cell line that produces an antibody that specifically recognize one or more epitopes of *Leptospira* LP1454, LP1118, LP1939, MCEII, CADF-like1, CADF-like2, CADF-like3, Lp0022, Lp1499, Lp4337, Lp328 and L21 OMPs, or epitopes of conserved variants thereof, or immunologically reactive peptide fragments thereof.

Methods are provided for preventing a *Leptospira*-related disorder in an animal or human subject in need thereof, comprising administering an amount of a vaccine or an antibody of the invention sufficient to confer immunity to the *Leptospira*-related disorder to the subject.

A method is provided for treating a *Leptospira-*related disorder in an animal or human subject in need thereof, comprising administering an amount of an antibody of the invention sufficient to inhibit or decrease the activity of a *Leptospira* pathogen.

In one embodiment, the method comprises administering an amount of a vaccine of the invention sufficient to inhibit or decrease the activity of the *Leptospira* pathogen.

In another embodiment, the amount of the vaccine of the invention administered is sufficient to confer immunity to *Leptospira* infection or a *Leptospira-*related disorder to the subject.

In another embodiment, the method for treating the *Leptospira-*related disorder may not involve administration of antibodies or a vaccine of the invention to a subject. For example, antibodies of the invention can be used to kill infections organisms *in vitro* where eventual intended use is to combat infection in animals or humans.

A pharmaceutical composition is provided comprising a therapeutically effective amount of an antibody that immunospecifically binds to *a Leptospira* LP1454, LP1118, LP1939, MCEII, CADF-like1, CADF-like2, CADF-like3, Lp0022, Lp1499, Lp4337, Lp328 and L21 OMP; and a pharmaceutically acceptable carrier.

A pharmaceutical composition is also provided comprising a therapeutically elective amount of a fragment or derivative of an antibody that immunospecifically binds to a *Leptospira* LP1454, LP1118, LP1939, MCEII, CADF-like1, CADF-like2, CADF-like3, Lp0022, Lp1499, Lp4337, Lp328 and L21 OMP; the fragment or derivative containing the binding domain of the antibody; and a pharmaceutically acceptable carrier.

Methods are provided for assaying for the presence or activity of a LP1454, LP1118, LP1939, MCEII, CADF-like1, CADF-like2, CADF-like3, Lp0022, Lp1499, Lp4337, Lp328 or L21 protein using antibodies to *Leptospira* outer membrane proteins (OMPs) LP1454, LP1118, LP1939, MCEII, CADF-like1, CADF-like2, CADF-like3, Lp0022, Lp1499, Lp4337, Lp328 or L21 or fragments thereof.

Methods are provided for diagnosing a *Leptospira-*related disorder in a subject comprising:

providing one or more antibodies to a *Leptospira* LP1454, LP1118, LP1939, MCEII, CADF-like1, CADF-like2, CADF-like3, Lp0022, Lp1499, Lp4337, Lp328 or L21 protein or a fragment thereof;

collecting a cell or tissue sample, e.g., a blood sample, from the subject,

contacting the sample with the antibodies; and

assaying for the presence or activity of a LP1454, LP1118, LP1939, MCEII, CADF-like1, CADF-like2, CADF-like3, Lp0022, Lp1499, Lp4337, Lp328 or L21 protein in the sample using antibodies to *Leptospira* LP1454, LP1118, LP1939, MCEII, CADF-like1, CADF-like2, CADF-like3, Lp0022, Lp1499, Lp4337, Lp328 or L21 or fragments thereof;

wherein presence or activity of a LP1454, LP1118, LP1939, MCEII, CADF-like1, CADF-like2, CADF-like3, Lp0022, Lp1499, Lp4337, Lp328 or L21 protein is indicative of the presence of the *Leptospira-*related disorder in the subject.

Methods are provided for diagnosing or screening for the presence of a *Leptospira*-related disorder in a subject comprising measuring the level of a LP1454, LP1118, LP1939, MCEII, CADF-like1, CADF-like2, CADF-like3, Lp0022, Lp1499, Lp4337, Lp328 or L21 protein functional activity in a sample derived from the subject, in which a decrease in the level of LP1454, LP1118, LP1939, MCEII, CADF-like1, CADF-like2, CADF-like3, Lp0022, Lp1499, Lp4337, Lp328 or L21 protein or LP1454, LP1118, LP1939, MCEIL CADF-like1, CADF-like2, CADF-like3, Lp0022, Lp1499, Lp4337, Lp328 or L21 protein functional activity in the sample, relative to the level of LP1454, LP1118, LP1939, MCEII, CADF-like1, CADF-like2, CADF-like3, Lp0022, Lp1499, Lp4337, Lp328 or L21 protein or LP1454, LP1118, LP1939, MCEII, CADF-like1, CADF-like2, CADF-like3, Lp0022, Lp1499, Lp4337, Lp328 or L21 I protein functional activity found in an analogous sample not having the disorder, indicates the presence of the disease or disorder or a predisposition for developing the disorder.

Methods are also provided for monitoring therapy using antibodies to *Leptospira* LP1454, LP1118, LP1939, MCEII, CADF-like1, CADF-like2, CADF-like3, Lp0022, Lp1499, Lp4337, Lp328 or L21 OMPs or fragments thereof.

Methods for using recombinant LP1454, LP1118, LP1939, MCEII, CADF-like1, CADF-like2, CADF-like3, Lp0022, Lp1499, Lp4337, Lp328 or L21 proteins as diagnostic agents are also provided. In one embodiment, a LP1454, LP1118, LP1939, MCEII, CADF-like1, CADF-like2, CADF-like3, Lp0022, Lp1499, Lp4337, Lp328 or L21 protein is used in a kinetic enzyme-linked immunosorbent assay (KELA ELISA).

A kit is provided comprising in one or more containers an antibody of the invention that immunospecifically binds to a *Leptospira* LP1454, LP1118, LP1939, MCEII, CADF-like1, CADF-like2, CADF-like3, Lp0022, Lp1499, Lp4337, Lp328 or L21 protein.

A kit is provided comprising in one or more containers an expression vector comprising a plasmid, wherein the plasmid comprises a recombinant DNA encoding a LP1454, LP1118, LP1939, MCEII, CADF-like1, CADF-like2, CADF-like3, Lp0022, Lp1499, Lp4337, Lp328 or L21 OMP or an epitope of a conserved variant or an immunologically reactive peptide fragment thereof.

### 4. BRIEF DESCRIPTION OF THE DRAWINGS

The present invention is described herein with reference to the accompanying drawings, in which similar reference characters denote similar elements throughout the several views. It is to be understood that in some instances, various aspects of the invention may be shown exaggerated or enlarged to facilitate an understanding of the invention.

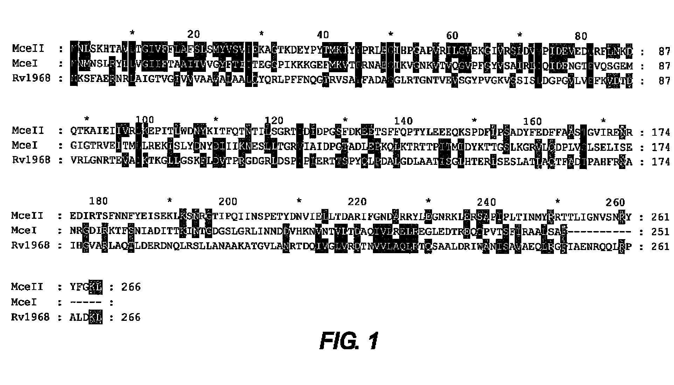

FIG. 1. Homology of hypothesized mammalian cell invasion proteins of *Leptospira.* The top sequence is MceII, a hypothetical MceI-like protein of L. *interrogans* serovar Pomona. This protein was renamed "MceII" (Lp0607, SEQ ID NOs: 7-8) to distinguish it from the MceI of L*. interrogans* serovar Lai (middle sequence). The middle sequence is MceI of L*. interrogans* serovar Lai (GenBank Accession number AAN49254; SEQ ID NO: 12). The bottom sequence is amino acids 31-293 of Rv1968, a Mce of *Mycobacterium tuberculosis* CDC1551 (GenBank accession number AE000516, SEQID NOS: 13-14). The top sequence, MeeII, has approximately 30% homology with the middle sequence, MeeI of *L*. *interrogans* serovar Lai, and approximately 25-30% homology with the bottom sequence, Rv1968, Mce of *Mycobacterium tuberculosis*. See Section 6.1.3 for details.

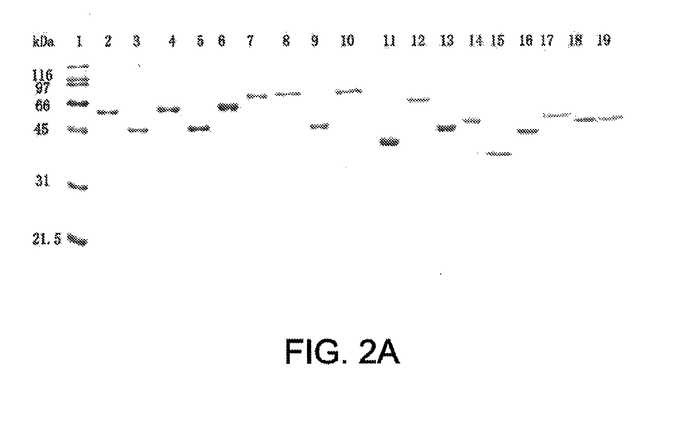

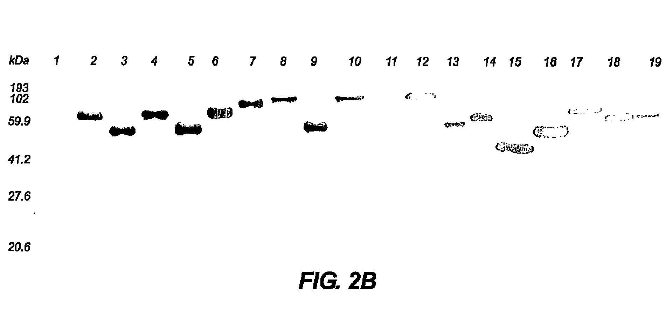

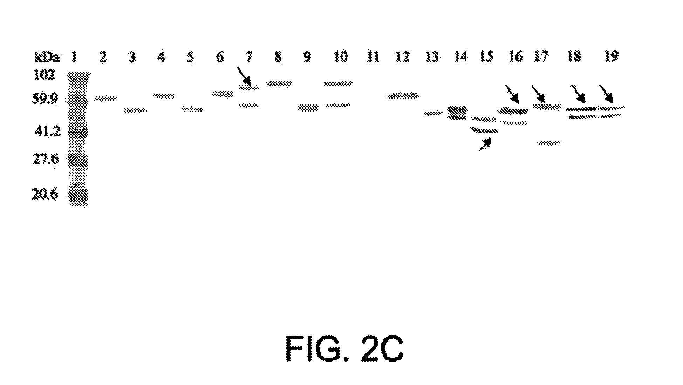

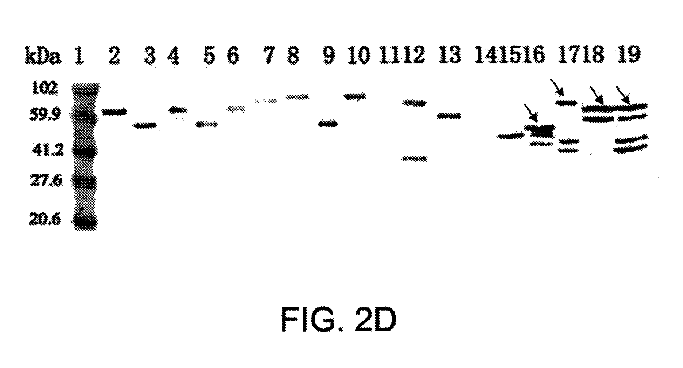

FIGS. 2A-2D. SDS-PAGE and immunoblot analysis of 18 purified recombinant outer membrane proteins of L*. interrogans* serovar Pomona. FIG. 2A represents purified GST fusion proteins of 18 recombinant proteins of *Leptospira* in SDS-PAGE gels that were stained with Coomassie brilliant blue R-250. FIGS. 2B, C, and D indicate immunoblots of purified recombinant proteins probes with polyclonal antibodies to GST, bovine sera from experimental infection and natural infection, respectively. Size standards (in kilodaltons) are indicated on the left. Lanes: 1, SDS-PAGE protein molecular weight standard marker (Bio-Rad); 2, Lp1118; 3, Lp1228; 4, Lp1404; 5, MCEI; 6, Lp2268; 7, LigBVT; 8, Lp1332; 9, Lp1965; 10, Lp1192; 11, Lp1947; 12, LigCon; 13, LIPI32; 14, Lp1495; 15, Lp1939; 16, MCEII; 17, Lp2471; 18, Lp1931; 19, Lp1454. See Section 6.1.3 for details.

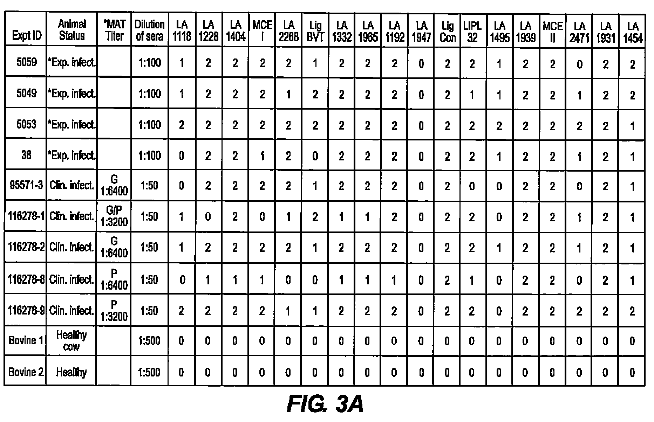

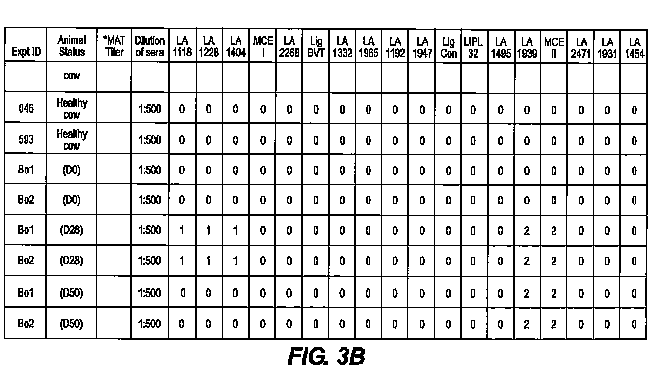

FIGS. 3A-B. Immunogenicity of recombinant outer membrane proteins (OMPs) *of Leptospira* to bovine sera from experimental and natural infection. *^{a}L*. *interrogans* serovar Grippotyphosa ;^{b}*L*. *interrogans* serovar Canicola; ^{c}*L*. *interrogans* serovar Copenhagenii; ^{d}*L*. *borgpetersenii* serovar Hardjo. 0=negative, 1=weak, 2=strong. See Section 6.1 for details.

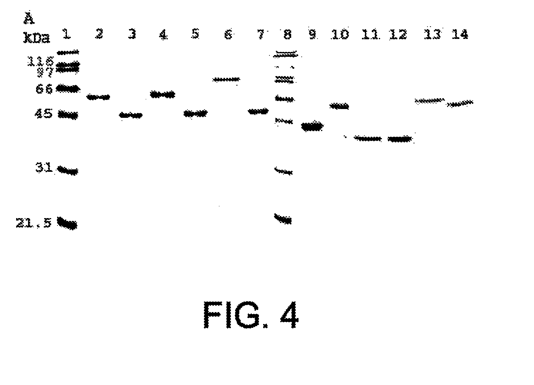

FIG. 4. Expression of recombinant OMPs of *Leptospira.* OMPs were cloned, expressed and purified as recombinant proteins. Purified recombinant OMPs (1.5-2 µg) were subjected to SDS-PAGE electrophoresis. Size standards (in kilodaltons) are indicated on the left. Lanes: 1 and 11, SDS-PAGE protein molecular weight standard marker (Bio-Rad); 2, LP1118; 3, LP1228; 4, LP1404; 5, MCEI; 6, LP1965; 7, LP1332; 9, LP1947; 10, 2471; 11, LP1939; 12, MCEII; 13, LP1932; 14, LP1454. See Section 6.2.3 for details.

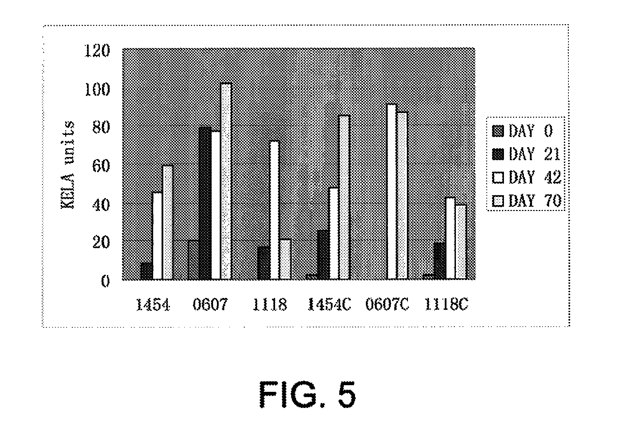

FIG. 5. Serum IgG antibody response of rMCE II, rLP1454 and rLP1118 - immunized hamsters. See Section 6.2.3 for details.

FIGS. 6A-E. Gene sequences *for Leptospira* CADF-like1, CADF-like2, CADF-like3, Lp0022, Lp1499, Lp4337, Lp328 and L21 OMPs.

### 5. DETAILED DESCRIPTION OF THE INVENTION

*Leptospira* outer membrane proteins (OMPs) LP1454, LP1118, LP1939, MCEII, CADF-like1, CADF-like2, CADF-like3, Lp0022, Lp1499, Lp4337, Lp328 or L21 are provided that can serve as tools for developing effective vaccines or diagnostic methods for leptospirosis. The antigenic properties of the *Leptospira* LP1454, LP1118, LP1939, MCEII, CADF-like1, CADF-like2, CADF-like3, Lp0022, Lp1499, Lp4337, Lp328 or L21. OMPs can be used to create, manufacture or improve vaccines. Vaccines, including but not limited to DNA vaccines, recombinant vaccines, and T cell epitope vaccines based on the foregoing OMPs are further provided. Methods for producing such vaccines are also provided. Also provided are methods for using *Leptospira* OMP genes, proteins and antibodies for serological diagnosis techniques.

For clarity of disclosure, and not by way of limitation, the detailed description of the invention is divided into the subsections set forth below.

**5.1 Identification of immunogenic proteins from genome-derived putative outer membrane proteins (OMPs) of *Leptospira***

Immunogenic proteins from genome-derived putative OMPs of *Leptospira* can be identified using standard methods known in the art. For example, experimentally infected animal sera, e.g., bovine or canine sera can be prepared according to standard methods (e.g., Surujballi, et al. 1997. Development and initial evaluation of an indirect enzyme-linked immunosorbent assay for the detection of Leptospira interrogans serovar hardjo antibodies in bovine sera. Can. J. Vet. Res. 61:260-266).

Infected animals can be identified by their exhibition of clinical symptoms or by a Microscopic Agglutination Test (MAT) using art-known methods. MAT can be carried out as previously described (Cole, J. R., Jr., H. C. Ellinghausen, and H. L. Rubin. 1979. Laboratory diagnosis of leptospirosis of domestic animals. Proc. Annu. Meet. U S Anim. Health Assoc: 189-195) with the whole cell antigens of serovars of interest.

Sera can be collected using standard methods, e.g., from the jugular vein, and stored at -20°C until use.

Gnomic DNA of a *leptovpira interrogans* serovar, e.g., *L. interrogans* serovar Pomona, can be prepared using standard methods known in the art, e.g., a DNAeasy kit (Qiagen, Valencia, CA). Nucleotide sequences encoding a rLP1454, rLP1118, rLP1939 or rMCEII, rCADF-like1, rCADF-like2, rCADF-like3, rLp0022, rLp1499, rLp4337, rLp328 or rL21 OMPs gene or portions thereof may be obtained by PCR amplification *of Leptospira* genomic DNA. Useful DNA sources include DNA preparations and cloned DNA in DNA libraries. Outer membrane DNA preparations or libraries are particularly preferred.

Genomic DNA can be amplified using standard PCR amplification techniques known in the art. Primers for amplifying and cloning putative outer membrane protein genes, for example, *L*. *interrogans* serovar specific sequences, can be designed without the signal sequences using standard methods well known in the art. A selection of primers suitable for use in identifying putative OMP genes is set forth in Example 1, Table 1.

PCR amplification can be carried out using standard methods, e.g., by use of a standard automated PCR thermal cycler and a Taq polymerase such as Accuprime Taq polymerase (Invitrogen, CA), One can choose to synthesize appropriate primers for use in the PCR reactions. It also is possible to vary the stringency of hybridization conditions used in priming the PCR reactions, to allow for greater or lesser degrees of nucleotide sequence similarity between the degenerate primers and the corresponding sequences in the cDNA library. One of ordinary skill in the art will know that the appropriate amplification conditions and parameters depend, in part, on the length and base composition of the primers and that such condition may be determined using standard formulae. Protocols for executing all PCR procedures discussed herein are well known to those skilled in the art, and may be found in references such as Gelfand, 1989, PCR Technology, Principles and Applications for DNA Amplification, H.A. Erlich, ed., Stockton Press, New York; and Current Protocols In Molecular Biology, Vol. 2, Ch. 15, Ausubel et al., eds. 1988, New York, Wiley & Sons, Inc.

The amplification may use, as the 5'-primer (*i.e*., forward primer), a degenerate oligonucleotide that corresponds to a segment of a known LP1454, LP1118, LP1939, MCEII, CADF-like1, CADF-like2, CADF-like3, Lp0022, Lp1499, Lp4337, Lp328 or L21 amino acid sequence (or other known putative or actual OMP amino acid sequence), preferably from the amino-terminal region. The 3'-primer (*i.e*., reverse primer) may be a degenerate oligonucleolide that corresponds to a distal segment of the same known LP1454, LP1118, LP1939, MCEII, CADF-like1, CADF-like2, CADP-like3, Lp0022, Lp1499, Lp4337, Lp328 or L21 amino acid sequence (i.e., carboxyl to the sequence that corresponds to the 5'-primer).

For example, the amino acid sequence of the LP1454 (SEQ ID NOS: 1-2; GenBank /NCBI Entrez Protein Database Accession No. AAN48653; National Center for Biotechnology Information, National Library of Medicine, Bethesda, MD 20894), LP 1118 (SEQ ID NOS: 3-4, GenBank / NCBI Accession No. AAN48316), LP1939 (SEQ ID NOS: 5-6; GenBank/NCBI Accession No. AAN49138) MCEII (Lp0607; SEQ ID NOS; 7-8), CADF-like1, CADF-like2, CADF-like3, Lp0022, Lp1499, Lp4337, Lp328 or L21 protein may be used to design useful 5' and 3' primers. Preferably, the primers correspond to segments in the N-terminal of the protein (see Section 6.1, Table 1).

The sequence of the optimal degenerate oligonucleotide probe corresponding to a known amino acid sequence may be determined by standard algorithms known in the art. See for example, Sambrook et a]., Molecular Cloning: A laboratory Manual, 2nd ed., Cold Spring Harbor Laboratory Press, Cold Spring Harbor, NY, Vol 2 (1989).

PCR products can be run, using standard methods known in the art, in a 1% agarose gel and visualized by elhidium bromide staining. PCR products can be eluted from the gel using a gel elution kit (e.g., from Qiagen, Valencia, CA).

A PCR-amplified sequence may be molecularly cloned and sequenced. For example, the PCR-amplified can be cloned into a vector, e.g., a TOPO TA vector, as described by the manufacturer (Inivitrogen, CA).

The amplified sequence can be utilized as a probe to isolate genomic (or cDNA) clones of a LP1454, LP1118, LP1939, MCEII, CADF-like1, CADF-like2, CADF-like3, Lp0022, Lp1499, Lp4337, Lp328 or L21 gene.

DNA sequencing can be performed with a standard automated nucleic acid sequencer, e.g., an ABI model 377 automated nucleic acid sequencer. Homology searches can be performed using standard searching methods e.g., BLAST, in the NCBI databases (Altschul, S. F., W. Gish, W. Miller, E. W. Myers, and D. J. Lipman. 1990. Basic local alignment search tool. J. Mol. Biol. 215:403-410).

The genomic DNA can be inserted into suitable vectors, including, but not limited to, plasmids, cosmids, bacteriophages lambda or T₄, and yeast artificial chromosome (YAC) or attenuated viruses (such as Herpes virus). See, for example, Sambrook et al., Molecular Cloning: A Laboratory Manual, 2d ed., Cold Spring Harbor Laboratory Press, Cold Spring Harbor, New York (1989); D. Glover (ed.), DNA Cloning: A Practical Approach, IRL Press, Ltd., Oxford, U.K., Vols. I and II (1985).

The identity of a cloned or amplified putative OMP gene sequence can be verified by comparing the amino acid sequences of its open reading frames with the amino acid sequence of the gene *(e.g., Leptospira* LP1454, LP1118, LP1939. MCEII, CADF-like1, CADF-like2, CADF-like3, Lp0022, Lp1499, Lp4337, Lp328 or L21polypeptide whose amino acid sequence is substantially similar to that of a LP1454, LP1118, LP1939, MCEII CADF-like1, CADF-like2, CADF-like3, Lp0022, Lp1499, Lp4337, Lp328 or L21 protein or polypeptide. The identity of the cloned or amplified OMP gene sequence may be further verified by examining its expression pattern, which should show highly localized expression in the outer membrane of the *Leptospira* species from which the gene sequence (e.g., LP1454, LP1118, LP1939, MCEII, CADF-like1, CADF-like2, CADF-like3, Lp0022, Lp1499, Lp4337, Lp328 or L21 gene sequence) was isolated.

Homology searches can be performed using standard search methods in the NCBI. database and BLAST (Altschul, S. F., W. Gish. W. Miller, E. W. Myers, and D. J. Lipman. 1990. Basic local alignment search tool. J. Mol. Biot. 215:403-410).

Although the polynucleotide sequences encoding LP1454, LP1118, LP1939, MCEII, CADF-like1, CADF-like2, CADF-like3, Lp0022, Lp1499, Lp4337, Lp328 or L21 OMP proteins described herein can be derived *L. interrogans* serovar Pomona, the sequences can be derived from another organism, derived from any recombinant source, synthesized in *vitro* or by chemical synthesis. The nucleotides in the polynucleotide sequences may be DNA or RNA and may exist in a double-stranded, single-stranded or partially double-stranded form.

Nucleic acids can also be prepared by any conventional means typically used to prepare nucleic acids in large quantity. For example, DNAs and RNAs may be chemically synthesized using commercially available reagents and synthesizers by methods that are well-known in the art (*see*, *e.g,*, Gait, 1985, Oligonucleotide Synthesis: A Practical Approach, IRL Press, Oxford, England). RNAs may be produce in high yield via *in vitro* transcription using plasmids such as SP65 (Promega Corporation, Madison, WI).

The nucleic acids may be purified by any suitable means, as are well known in the art. For example, the nucleic acids can be purified by reverse phase or ion exchange HPLC, size exclusion chromatography or gel electrophoresis. Of course, the skilled artisan will recognize that the method of purification will depend in part on the size of the DNA to be purified.

**5.2 Expression Systems**

Using standard methods, inserts can be sub-eloned into expression vectors, e.g., pGFX-KG (Stratagene), pRSETA (InVitroGen, CA), attenuated herpes virus vector, etc., using standard methods known in the art, and transformed into *E. coli* (BL21 DE3 or CQ21).

In one embodiment, a vector capable of expressing a recombinant DNA is provided. The recombinant DNA can be selected from the group consisting of:
a recombinant DNA that encodes a protein having the amino acid sequence as shown in SEQ ID NO: 2 (LP1454),
a recombinant DNA that encodes a protein having the amino acid sequence as shown in SEQ ID NO: 4 (LP1118),
a recombinant DNA that encodes a protein having the amino acid sequence as shown in SEQ ID NO: 6 (LP1939),
a recombinant DNA that encodes a protein having the amino acid sequence as shown in SEQ ID NO: 8 (MCEII),
a recombinant DNA that encodes a protein having the amino acid sequence as shown in SEQ ID NO: 53 (CADF-like1),
a recombinant DNA that encodes a protein having the amino acid sequence as shown in SEQ ID NO: 55 (CADF-like2),
a recombinant DNA that encodes a protein having the amino acid sequence as shown in SEQ ID NO: 57 (CADF-like3),
a recombinant DNA that encodes a protein having the amino acid sequence as shown in SEQ ID NO: 59 (Lp0022),
a recombinant DNA that encodes a protein having the amino acid sequence as shown in SEQ ID NO: 61 (Lp1499),
a recombinant DNA that encodes a protein having the amino acid sequence as shown in SEQ ID NO: 63 (Lp4337),
a recombinant DNA that encodes a protein having the amino acid sequence as shown in SEQ ID NO: 65 (Lp328),
a recombinant DNA that encodes a protein having an amino acid sequence as shown in SEQ ID NO: 67 (L21),
a recombinant DNA that encodes an immunogenic epitope or immunologically active fragment of any of the above, and
a recombinant DNA that encodes a protein fragment of at least 10%, 20%, 30%, 40%, 50%. 60% 70%, 80%, 90% or 95% of the length of the amino acid sequence of any of the above,
and wherein the recombinant DNA is inserted into the vector such that a recombinant protein is expressed when the vector is provided in an appropriate host.

In bacterial systems a number of expression vectors may be advantageously selected depending upon the use intended for the expressed OMPs, such as LP1454, LP1118, LP1939, MCEII CADF-like1, CADF-like2, CADF-like3, Lp0022, Lp1499, Lp4337, Lp328 or L21 product.

For example, when large quantities of LP1454, LP1118, LP1939, MCEII, CADF-like1 , CADF-like2, CADF-like3, Lp0022, Lp1499, Lp4337, Lp328 or L21 protein are to be produced for the generation of antibodies, screening peptide libraries or formulating pharmaceutical compositions, vectors that direct the expression of high levels of fusion protein products that are readily purified may be desirable. Such vectors include but are not limited to the *E. coli* expression vector pUR278 (Ruther et al., 1983, EMBO J. 2:1791), in which the LP1454, LP1118, LP1939, MCEII, CADF-like1, CADF-like2, CADF-like3, Lp0022, Lp1499, Lp4337, Lp328 or L21coding sequence may be ligated into the vector in frame with the *lacZ.* coding region so that a hybrid protein is produced; pIN vectors (Inouye & Inouye, 1985, Nucleic acids Res. 13:3101-3109; Van Heeke & Schuster, 1989, J. Biol. Chem. 264:5503-5509); and the like. pGEX vectors may also be used to express foreign polypeptides as fusion proteins with glulathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned polypeptide of interest can be released from the GST moiety.

In one such embodiment of a bacterial system, full length DNA sequences are appended with in-frame *Bam*HI sites at the amino terminus and *Eco*RI sites at the carboxyl terminus using standard PCR methodologies (Innis et al., 1990, *supra*) and ligated into the pGEX-2TK vector (Pharmacia, Uppsala, Sweden). The resulting cDNA construct contains a kinase recognition site at the amino terminus for radioactive labeling and glutathione S-transferase sequences at the carboxyl terminus for affinity purification (Nilsson, et al., 1985, EMBO J. 4: 1075; Zabeau and Stanley, 1982, EMBO J. 1: 1217).

The recombinant constructs of the present invention may include a selectable marker for propagation of the construct. For example, a construct to be propagated in bacteria preferably contains an antibiotic resistance gene, such as one that confers resistance to kanamycin, tetracycline, streptomycin or chloramphenicol. Suitable vectors for propagating the construct include plasmids, cosmids, bacteriophages or viruses, to name but a few.

In addition, the recombinant constructs may include cell-expressible, selectable or screenable marker genes for isolating, identifying or tracking cells transformed by these constructs. Selectable markers include, but are not limited to, genes that encode visible markers such as green fluorescent protein (GFP) or those that confer antibiotic resistance, (e.g., resistance to kanamycin or hygromycin).

In yeast, a number of vectors containing constitutive or inducible promoters may be used (Current Protocols in Molecular Biology, Vol 2. 1988, Ed. Ausubel et al., Greene Publish. Assoc. & Wiley Interscience, Ch. 13; Grant et al., 1987, Expression and Secretion Vectors for Yeast, in Methods in Enzymology, Eds. Wu & Grossman, 1987, Acad. Press, N.Y., Vol. 153, pp. 516-544; Glover, 1986, DNA Cloning, Vol. II, IRL Press, Wash., D.C., Ch. 3; and Bitter, 1987, Heterologous Gene Expression in Yeast, Methods in Enzymology, Eds. Berger & Kimmel, Acad. Press, N.Y., Vol. 152, pp. 673-684; and The Molecular Biology of the Yeast Saccharomyces, 1982, Eds. Strathern et al., Cold Spring Harbor Press, Vols. I and II).

Another alternative expression system that can be used to express LP1454, LP1118, LP1939, MCEII, CADF-like1, CADF-like2, CADF-like3, Lp0022, Lp1499, Lp4337, Lp328 or L21 is an insect system. In one such system, *Autographa californica* nuclear polyhedrosis virus (AcNPV) is used as a vector to express foreign genes. The virus grows in *Spodoptera frugiperda cells*. The rLP1454, rLP1118, rLP1939, rMCEII, rCADF-like1, rCADF-like2, rCADF-like3, rLp0022, rLp1499, rLp4337, rLp328 or rL21 coding sequence may be cloned into non-essential regions (for example the polyhedron gene) of the virus and placed under control of an AcNPV promoter (for example the polyhedron promoter). Successful insertion of the rLP1454, rLP1118, rLP1939, rMCEII, rCADF-like1, rCADF-like2, rCADF-like3, rLp0022, rLp1499, rLp4337, rLp328 or rL21 coding sequence will result in inactivation of the polyhedron gene and production of non-occluded recombinant virus (*i.e*., virus lacking the proteinaceous coat coded for by the polyhedron gene). These recombinant viruses are then used to infect *Spodoptera frugiperda* cells in which the inserted gene is expressed (*e*.*g*., see Smith et al., 1983, J. Viol. 46:584; Smith, U.S. Patent No. 4,215,051).

In mammalian host cells, a number of viral based expression systems may be utilized, such as an attenuated herpes virus vector or an adenovirus.

In cases where an adenovirus is used as an expression vector, the LP1454, LP1118, LP1939, MCEII, CADF-like1, CADF-like2, CADF-like3, Lp0022, Lp1499, Lp4337, Lp328 or L21 coding sequence may be ligated to an adenovirus transcription/translation control complex, *e.g.,* the late promoter and tripartite leader sequence. This chimeric gene may then be inserted in the adenovirus genome by *in vitro* or in *vivo* recombination. Insertion in a non-essential region of the viral genome (*e.g*., regions E1 or E3) will result in a recombinant virus that is viable and capable of expressing LP1454, LP1118, LP1939, MCEII, CADF-like1, CADF-like2, CADF-like3, Lp0022, Lp1499, Lp4337, Lp328 or L21 in infected hosts (*e.g*., See Logan & Shenk, 1984, Proc. Natl. Acad. Sci. USA 81:3655.3659). Alternatively, a vector derived from vaccinia virus can be used, which would typically make use of the vaccinia 7.5K promoter (*See*, *e.g*., Mackett et al., 1982, Proc. Natl. Acad. Sci. USA 79:7415-7419; Mackett et al., 1984, J. Virol. 49:857-864; Panicali et al,, 1982, Proc. Natl. Acad. Sci. USA 79:4927-4931). Regulatable expression vectors such as the tetracycline repressible vectors may also be used to express the coding sequences in a controlled fashion.

Specific initiation signals may also be required for efficient translation of inserted LP1454, LP1118, LP1939, MCEII, CADF-like1, CADF-like2, CADF-like3, Lp0022, Lp1499, Lp4337, Lp328 or L21 coding sequences. These signals include the ATG initiation codon and adjacent sequences. In cases where the entire LP1454, LP1118, LP1939, MCEII, CADF-like1, CADF-like2, CADF-like3, Lp0022, Lp1499, Lp4337, Lp328 or L21 gene, including its own initiation codon and adjacent sequences, is inserted into the appropriate expression vector, no additional translational control signals may be needed. However, in cases where only a portion of the LP1454, LP1118, LP1939, MCEII, CADF-like1, CADF-like2
CADF-like3, Lp0022, Lp1499, Lp4337, Lp328 or L21 coding sequence is inserted, exogenous translational control signals, including the ATG initiation codon, must be provided. Furthermore, the initiation codon must be in phase with the reading frame of the LP1454, LP1118, LP1939, MCEII, CADF-like1, CADF-like2, CADF-like3. Lp0022, Lp1499, Lp4337, Lp328 or L21 coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (*see* Bittner et al., 1987, Methods in Enzymol. 153:516-544).

In addition, a host cell strain can be chosen that modulates the expression of the inserted sequences, or modifies and processes the gene product in the specific fashion desired. Such modifications (*e.g.,* glycosylation) and processing (*e.g*., cleavage) of protein products may be important for the function of the protein. Different host cells have characteristic and specific mechanism for the post-translational processing and modification of proteins. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product may be used.

For long-term, high-yield production of recombinant proteins, stable expression is preferred. For example, cell lines that stably express the LP1454, LP1118, LP1939, MCEII, CADF-likel, CADF-like2, CADF-like3, Lp0022, Lp1499, Lp4337, Lp328 or L21proteins may be engineered. Rather than using expression vectors that contain viral origins of replication, host cells can be transformed with the LP1454, LP1118, LP1939, MCEII, CADF-like1, CADF-like2, CADF-like3, Lp0022, Lp1499, Lp4337, Lp328 or L21coding sequence controlled by appropriate expression control elements (*e.g.*, promoter and/or enhancer sequences, transcription terminators, polyadenylation sites, etc.), and a selectable marker. Following the introduction of foreign DNA, genetically engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci, which in turn can be cloned and expanded into cell lines. This method may advantageously be used to engineer cell lines that express the LP1454, LP1118, LP1939, MCEII, CADF-like1, CADF-like2, CADF-like3, Lp0022, Lp1499, Lp4337, Lp328 or L21 protein. Such engineered cell lines are particularly useful in screening for molecules or drugs that affect LP1454, LP1118, LP1939, MCEII, CADF-like1, CADF-like2, CADF-like3, Lp0022, Lp1499, Lp4337, Lp328 or L21 function.

A number of selection systems may be used, including but not limited to, the herpes simplex virus thymidine kinase (Wigler, et al., 1977, Cell 11:223), hypoxanthineguanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for *dhfr*, which confers resistance to methotrexate (Wigler, et al., 1980, Proc. Natl. Acad. Sci. USA 77:3567; O'Hare, et al., 1981, Proc. Natl. Acad. Sci. USA 78:1527); *gpt*, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and *hygro,* which confers resistance to hygromycin (Santerre, et al., 1984, Gene 30:147) genes. Additional selectable genes include *trpB*, which allows cells to utilize indole in place of tryptophan; *hisD*, which allows cells to utilize histinol in place of histidine (Hartman & Mulligan, 1988, Proc. Natl. Acad. Sci. USA 85:8047); *ODC* (ornithine decarboxylase), which confers resistance to the ornithine decarboxylase inhibitor, 2-(difluoromethyl)-DL-ornithine, DFMO (McConlogue L., 1987, In: Current Communications in Molecular Biology, Cold Spring Harbor Laboratory ed.) and glutamine synthetase (Bebbington et al., 1992, Biotech 10:169).

The expression characteristics of an endogenous LP1454, LP1118, LP1939, MCEII, CADF-like1, CADF-like2, CADF-like3, Lp0022, Lp1499, Lp4337, Lp328 or L21 gene within a cell line, plant or microorganism may be modified by inserting a heterologous DNA regulatory element into the genome of a stable cell line, plant or cloned microorganism such that the inserted regulatory element is operatively linked with the endogenous LP1454, LP1118, LP1939, MCEII, CADF-like1, CADF-like2, CADF-like3, Lp0022, Lp1499, Lp4337, Lp328 or L21 gene. For example, an endogenous gene that is normally "transcriptionally silent", *i.e.*, an LP1454, LP1118, Lp1939, MCEII, CADF-like1, CADF-like2, CADF-like3, Lp0022, Lp1499, Lp4337, Lp328 or L21 gene that is normally not expressed, or is expressed only at very low levels in a cell line, plant or microorganism, may be activated by inserting a regulatory element that is capable of promoting the expression of a normally expressed gene product in that cell line, plant or microorganism. Alternatively, a transcriptionally silent, endogenous gene may be activated by insertion of a promiscuous regulatory element that works across cell types.

A heterologous regulatory element may be inserted into a stable cell line, plant or cloned microorganism, such that it is operatively linked with an endogenous LP1454, LP1118, LP1939, MCEII, CADF-likel, CADF-like2, CADF-like3, Lp0022, Lp1499, Lp4337, Lp328 or L21 gene, using techniques that are well known to those of skill in the art, such as targeted homologous recombination (*e.g.*, in Chappel, U.S. Patent No. 5,272,071; PCT publication No. WO 91/06667, published May 16. 1991).

**5.3 Production of *Leptospira* outer membrane proteins and polypeptides**

While leptospiral OMP polypeptides and peptides, such as LP1454, LP1118, Lp1939, MCEII, CADF-like1, CADF-like2, CADF-like3, Lp0022, Lp1499, Lp4337, Lp328 or L21 polypeptides and peptides, can be chemically synthesized (*e.g*., see Creighton, 1983, Proteins. Structures and Molecular Principles, W.H. Freeman & Co., N.Y.) large polypeptides derived from LP1434, LP1118, LP1939, MCEII, CADF-like1, CADF-like2, CADF-like3, Lp0022, Lp1499, Lp4337, Lp328 or L21 and full length proteins may advantageously be produced by recombinant DNA technology using techniques well known to those skilled in the art for expressing nucleic acid sequences.

Methods that are well known to those skilled in the art can be used to construct expression vectors containing the LP1454, LP1118, LP1939, MCEII, CADF-like1, CADF-like2, CADF-like3, Lp0022, Lp1499, Lp4337, Lp328 or L21coding sequence and appropriate transcriptional/translational control signals. These methods include *in vitro* recombinant DNA techniques, synthetic techniques and *in vivo* recombination/genetic recombination. (*See*, for example, the techniques described in Sambrook et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y.). RNA capable of encoding LP1454, LP1118, LP1939, MCEII, CADF-like1, CADF-like2, CADF-like3, Lp0022, Lp1499, Lp4337, Lp328 or L21 polypeptide may also be chemically synthesized (Gait, ed., 1984, Oligonucleotide Synthesis, IRL Press, Oxford).

A variety of host-expression vector systems may be utilized to express the gene products. Such host-expression systems represent vehicles by which the gene products of interest may be produced and subsequently recovered and/or purified from the culture or organism (using purification methods well known to those skilled in the art), but also represent cells that may, when transformed or transfected with the appropriate nucleotide coding sequences, exhibit the LP1454, LP1118, LP1939, MCEII, CADF-like1, CADF-like2, CADF-like3, Lp0022, Lp1499, Lp4337, Lp328 or L21protein *in situ*.

These include, but are not limited to, microorganisms such as bacteria (*e.g.*, *E*. *coli, B. subtilis*) transformed with recombinant bacteriophage DNA, plasmid DNA or cosmid DNA expression vectors containing LP1434, LP1118, LP1939, MCEII, CADF-like1 CADF-like2, CADF-like3, Lp0022, Lp1499, Lp4337, Lp328 or L21 protein coding sequences; yeast (*e.g.*, *Saccharomyces*, *Pichia*) transformed with recombinant yeast expression vectors containing the protein coding sequences; insect cell systems infected with Recombinant virus expression vectors (*e.g.,* baculovirus) containing the protein coding sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing the protein coding sequences: or mammalian cell systems (*e*.*g*., COS, CHO, BHK, 293, 3T3) harboring recombinant expression constructs containing promoters derived from the genome of mammalian cells (*e*.*g*., metallothionein promoter) or from mammalian viruses (*e*.*g*., the adenovirus late promoter; the vaccinia virus 7.5K promoter; the cytomegalovirus promoter/enhancer; etc.),

Depending on the host/vector system utilized, any of a number of suitable transcription and translation elements, including constitutive and inducible promoters, may be used in the expression vector. For example, when cloning in bacterial systems, inducible promoters such as pL of bacteriophage λ, plac, ptrp, ptac (ptrp-lac hybrid promoter; cytomegalovirus promoter) and the like may be used; when cloning in insect cell systems, promoters such as the baculovirus polyhedron promoter may be used; when cloning in plant cell systems, promoters derived from the genome of plant cells (*e*.*g*., heat shock promoters; the promoter for the small subunit of RUBISCO; the promoter for the chlorophyll α/β binding protein) or from plant viruses (*e*.*g*., the 35S RNA promoter of CaMV; the coat protein promoter of TMV) may be used; when cloning in mammalian cell systems, promoters derived from the genome of mammalian cells (*e*.*g*., metallothionein promoter) or from mammalian viruses (*e*.*g*., the adenovirus late promoter; the vaccinia virus 7.5K promoter) may be used; when generating cell lines that contain multiple copies of the LP1454, LP1118, LP1939, MCEII, CADF-like1, CADF-like2, CADF-like3, Lp0022, Lp1499, Lp4337, Lp328 or L21 coding sequence, SV40-, BPV- and EBV-based vectors may be used with an appropriate selectable marker.

A protein region can be considered "homologous" to a second protein region when the amino acid sequence of the first region is at least 30%, 40%, 50%, 60%, 70%, 75%, 80%, 90%, or 95% identical, when compared to any sequence in the second region of an equal number of amino acids as the number contained in the first region or when compared to an aligned sequence of the second region that has been aligned by a computer homology program known in the art *e*.*g*., the BLAST program described above.

**5.4 In situ expression of outer membrane proteins**

rLP1454, rLP1118, rLP1939, rMCEII, rCADF-like1, rCADF-like2, rCADF-like3, rLp0022, rLp1499, rLp4337, rLp328 and rL21 genes show highly localized expression in the *Leptospira* outer membrane. Such expression patterns may be ascertained by using standard art-known methods such as Northern hybridization, in situ hybridizations using antisense probes isolation of the OMPs from the outer membranes, or by standard methods of immunogold labeling (see, e.g., Matsunaga J, et al. Pathogenic Leptospira species express surface-exposed proteins belonging to the bacterial immunoglobulin superfamily. Mol Microbiol. 2003 Aug;49(4):929-45.

**5.5 Protein Identification and Purification**

Once a recombinant protein is expressed, it can be identified by assays based on the physical or functional properties of the product, including radioactive labeling of the product followed by analysis by gel electrophoresis, radioimmunoassay, ELISA, bioassays, etc.

Once the encoded protein is identified, it may be isolated and purified by standard methods including chromatography (*e*.*g*., high performance liquid chromatography, ion exchange, affinity, and sizing column chromatography), centrifugation, differential solubility, or by any other standard technique for the purification of proteins. The actual conditions used will depend, in part, on factors such as net charge, hydrophobicity, hydrophilicity, etc., and will be apparent to those having skill in the art. The functional properties may be evaluated using any suitable assay, *e.g.* an assay for the ability to after root formation. For the practice of the present invention, it is preferred that the polypeptide is at least 80% purified from other proteins. It is more preferred that they are at least 90% purified. For *in vivo* administration, it is preferred that it is greater than 95% purified, and more preferably greater than 99%. For example, recombinant proteins can be expressed and purified as follows. Exponentially growing cultures (OD₆₀₀=0.6) of *E. coli* (BL21 DE3) or CQ21 harboring recombinant plasmids can be induced to synthesize fusion proteins by standard methods using 1mM isopropy-β-D-thiogalactopyranoside (IPTG; Sigma, St. Louis, Mo.). For each recombinant protein, a pilot experiment can be performed with 3mL culture volume

The molecular weight of the expressed recombinant protein can be checked by SDS-PAGE analysis. SDS-PAGE can be performed using standard methods (e.g., Chang, Y. F., D. P. Ma, J. Shi, and M. M. Chengappa. 1993. Molecular characterization of a leukotoxin gene from a Pasteurelle haemolytica-like organism, encoding a new member of the RTX toxin family. Infect. Immun. 61:2089-2095). Recombinant proteins can be loaded into SDS-PAGE gel and stained with Coomassie brilliant blue R-250 followed by destaining.

Mass cultivation can be performed according to standard methods, e.g., in 500mL of LB broth with ampicillin (50 µg/mL). Bacteria can be harvested by standard methods, e.g., by centrifugation at 5,000 RPM for 30 min. The cell pellets can then be washed and suspended in PBS allowed by passing through a French pressure cell (e.g., American Instrument).

Lysates can be centrifuged using standard methods at 8,000 xg for 30 min and the inclusion bodies washed and purified using standard methods (e.g., Palaniappan, et al. 2004. Expression of leptospiral immunoglobulin-like protein by Leptospira interrogans and evaluation of its diagnostic potential in a kinetic ELISA. J. Med. Microbiol. 53:975-984, Palaniappan, et al. 2002. Cloning and molecular characterization of an immunogenic LigA protein of Leptospira interrogans. Infect Immun. 70:5924-5930) with model prep cell (BioRad, CA.).

Purified protein can be isolated by dialysis at 4°C with PBS by changing PBS for at least 4 times. The purified protein can be concentrated and lyophilized (Palaniappan, R. U., Y. F. Chang, F. Hassan, S. P. McDonough, M. Pough, S. C. Barr, K. W. Simpson., H. O. Mohammed, S. Shin, P. McDonough, R. L. Zuerner, J. Qu, and 8. Roe. 2004. Expression of leptospiral immunoglobulin-like protein by Leptospira interrogans and evaluation of its diagnostic potential in a kinetic ELISA. J. Med. Microbiol. 53:975-984). Lyophilized protein can be suspended with sterile PBS and quantified by the Bradford assay (Bio-Rad).

Recombinant proteins can be separated using standard methods, e.g., by running SDS-PAGE followed by transfer onto nitrocellulose membrane (Schleicher & Schuell Keene, NH) in a Trans Blot Cell (Bio-Rad Laboratories, Hercules, CA.) and immunoblotting (e.g., western blotting). For example, after transfer, the membrane can be blocked with TBS plus 1% bovine serum albumin (BSA) for 1h. Antisera can then be diluted in TBS-BSA and incubated with the blot for I h. After three washings in TBS plus 0.1% Tween 20, the blot can be incubated for 1 h with 1:2,000 Goat anti-bovine IgG conjugated with alkaline phosphatase (KPL). The blot can be washed three times as described above and developed with NBT/BCIP as described by Chang et al. (1995. Recombinant OspA protects dogs against infection and disease caused by Borrelia burgdorferi. Infect..Immun. 63:3543-3549).

The recombinant protein expression can also be assessed immunologically by immunoassays such as radioimmuno-precipitation, enzyme-linked immunoassays and the like. This can be achieved by using an anti-OMP antibody, e.g., an anti-LP1454, LP1118, LP1939, MCEII, CADF-like 1, CADF-like2. CADF-like3, Lp0022, Lp1499. Lp4337, Lp328 or L21 protein antibodies.

Expression of the protein product can also be assessed using analytical techniques such as amino acid sequencing, which can be accomplished by means of, for example, Edman degradation or tandem mass spectroscopy, or by analysis of the masses of peptide generated by partial hydrolysis of the protein product using mass spectroscopy. In the identification of a protein of interest (e.g., a LP1454, LP1118, LP1939, MCEII, CADF-like1, C-ADF-like2, CADF-like3, Lp0022, Lp1499, Lp4337, Lp328 or L21 protein) by mass spectroscopy, it will often be desirable to separate the protein of interest from other protein constituents of the cell by means of two-dimensional gel electrophoresis, partially hydrolyze the isolated protein using an amino acid specific protease (*e,g*_{.}, Lys-C, trypsin), and then determine the mass of the resulting peptide fragments using mass spectroscopy.

Determination of peptide mass can then be used to identify the protein as LP1454, LP118, LP1939, MCEII, CADF-like I, CADF-like2, CADF-like3, Lp0022, Lp1499, Lp4337, Lp328 or L21, or a variant thereof, using a database of the predicted masses of protein proteolysis products and analysis software such as Protein Prospector, which is publicly available on the internet at htlp://prospector.ucsf.edu.

**5.6 Antibodies to Outer Membrane Proteins and Polypeptides**

Antibodies that specifically recognize one or more epitopes of a *Leptosira* outer membrane protein, such as LP1454, LP1 118, LP1939, MCEII CADF-like1 , CADF-like2, CADF-like3. Lp0022, Lp1499, Lp4337, Lp328 or L21, or epitopes of conserved variants or peptide fragments thereof are also provided. Such antibodies include, but are not limited to, polyclonal antibodies, monoclonal antibodies (mAbs), human, humanized or chimeric antibodies, single chain antibodies. Fab fragments, F(ab')₂ fragments, fragments produced by a Fab expression library, anti-idiotypic (anti-Id) antibodies and epitope-binding fragments of any of the above.

A method for producing an antibody is also provided wherein the method comprises: (a) providing a microorganism in a culture containing a DNA encoding a fusion polypeptide comprising at least one epitope of a *Leptospira* OMP, wherein the *Leptospira* OMP is selected from the group consisting of LP1454, LP1118, LP1939, MCEII, CADF-like1. CADF-like2, CADF-like3, Lp0022, Lp1499, Lp4337, Lp328 and L21, linked to a polypeptide that facilitates isolation of the fusion polypeptide; (b) culturing the microorganism in a culture to produce the fusion polypeptide; (c) isolating the fusion polypeptide: (d) producing the antibody from the polypeptide. In a preferred embodiment, the polypeptide is removed from the antigen portion of the fusion polypeptide.

A method for producing a monoclonal antibody is also provided comprising: (a) providing a microorganism in a culture containing a DNA encoding a fusion polypeptide comprising a at least one epitope of a *Leptospira* OMP, wherein the *Leptospira* OMP is selected from the group consisting of LP1454, LP1118, LP1939, MCEII, CADF-likel CADF-like2, CADF-like3, Lp0022, Lp1499, Lp4337, Lp328 and L21. , linked to a polypeptide that facilitates isolation of the fusion polypeptide; (b) culturing the microorganism in a culture to produce the fusion polypeptide; (c) isolating the fusion polypeptide; and (d) producing the monoclonal antibody from the polypeptide. Preferably, the polypeptide is removed from the antigen portion of the fusion polypeptide.

In one embodiment, an isolated and purified antibody of the invention can specifically recognize one or more epitopes of a *Leptospira* LP1454, LP1118, LP1939, MCEII, CADF-like1, CADF-like2, CADF-like3, Lp0022. Lp1499, Lp4337, Lp328 or L21OMP, or epitopes of conserved variants or immunologically reactive peptide fragments thereof.

The isolated and purified antibody to the *Leptospira* LP1454, LP1118, LP1939, MCEII, CADF-like1, CADF-like2, CADF-like3, Lp0022, Lp1499, Lp4337, Lp328 or L21 OMP can be conjugated to moieties including but not limited to a targeting agent, a label, a carrier, a drug, a toxin and a solid support.

For the production of antibodies, various host animals may be immunized by injection with the OMP protein or peptide of interest, such as an LP1454, LP1118, LP1939, MCEII, CADF-like1, CADF-like2, CADF-like3, LP0022, Lp1499, Lp4337, Lp328 or L21 peptide (*e*.*g.*, one corresponding to a functional domain of the protein), a truncated polypeptide (in which one or more domains has been deleted), functional equivalents of the protein or mutants of the protein. Such proteins, polypeptides, peptides or fusion proteins can be prepared and obtained as described above. Host animals may include, but are not limited to, dogs, cows, sheep, horses, rabbits, rats and mice, to name but a few.

Various adjuvants may be used to increase the immunological response, depending on the host species, including, but not limited to, Freund's (complete and incomplete), mineral gels such as aluminum hydroxide, surface active substances such as lysolecithin, pluronic polyols, polyanions, peptides, oil emulsions, keyhole limpet hemocyanin, dinitrophenol and potentially useful human adjuvants such as BCG (bacille Calmette-Guerin) and *Corynebacterium parvum.*

Polyclonal antibodies are heterogeneous populations of antibody molecules derived from the sera of the immunized animals.

A hybridoma or immortalized cell line is also provided that secretes a monoclonal antibody of the invention. A hybridoma or immortalized cell line can be produced using methods known in the art that produces an antibody that specifically recognize one or more epitopes of *Leptospira* LP1434, LP1118, LP1939, MCEII, CADF-like1, CADF-like2, CADF-like3, Lp0022, Lp1499, Lp4337, Lp328 or L21 OMPs, or immunologically reactive peptide fragments thereof.

Monoclonal antibodies to *Leptospira* outer membrane proteins or polypeptides, such as LP1454, LP1118, LP1939, MCEII, CADF-like1, CADF-like2, CADF-like3, Lp0022., Lp1499, Lp4337, Lp328 or L21 proteins or polypeptides, may be prepared by using any technique that provides for the production of antibody molecules by continuous cell lines in culture. These include but are not limited to the hybridoma technique originally described by Kohler and Milstein, (Nature, 1975, 256:495-4.9-7.), the human B-cell hybridoma technique (Kosbor et al., 1983, Immunology Today, 4:72; Cote et al., 1983, Proc. Natl. Acad. Sci., 80:2026-2030) and the EBV-hybridoma technique (Cole et al., 1985, Monoclonal Antibodies and Cancer Therapy, Alan R. Liss, Inc., pp. 77-96). Such antibodies may be of any immunoglobulin class including, but not limited to, IgG, IgM, IgE, IgA, IgD and any subclass thereof The hybridoma producing the monoclonal antibodies of this invention may be cultivated *in vitro* or *in vivo.*

Additionally, recombinant Antibodies, such as chimeric and humanized monoclonal antibodies, comprising both human and non-human portions, which can be made using standard recombinant DNA techniques, are within the scope of the invention. A chimeric antibody is a molecule in which different portions are derived from different species, such as those having a variable region derived from a murine mAb and a human immunoglobulin constant region. (See, *e.g*., Cabilly et al., U.S. Patent No. 4,816,567; and Boss et al., U.S. Patent No. 4,816397, which are incorporated herein by reference in their entirety). Humanized antibodies are antibody molecules from non-human species having one or more complementarily determining regions (CDRs), from the non-human species and a framework region from a human immunoglobulin molecule. (See, *e.g.,* Queen, U.S. Patent No. 5,585,089, which is incorporated herein by reference in its entirety). Such chimeric and humanized monoclonal antibodies can be produced by recombinant DNA techniques known in the art, for example using methods described in PCT Publication No. WO 87/02671; European Patent Application 184,187; European Patent Application 171,496; European Patent Application 173,494; PCT Publication No. WO 86/01533; U.S. Patent No. 4,816,567; European Patent Application 125,023; Better et al. (1988) Science 240:1041-1043; Liu et al. (1987) Proc. Natl. Acad Sci. USA 84:3439-3443; Liu et al, (1987) J. Immunol. 139:3321-3526; Sun et al. (1987) Proc. Natl. Acad. Sci. USA 84:214-218; Nishimura et al. (1987) Canc. Res. 47:999-1005; Wood et al. (1985) Nature 314:446-449; and Shaw et al, (1988) J. Natl. Cancer Inst. 80:1553-1559);Morrison (1985) Science 229:1202-1207; Oi et al. (1986) Bio/Techniques. 4:214; U.S. Patent 5,225,539; Jones et al. (1986) Nature 321:552-525; Verhoeyan et al (1988) Science 239:1534; and Beidler et al, (1988) J. Immunol. 141:4053-4060.

With respect to treatment of a particular species, such as a cow (or dog), completely bovine (or canine) antibodies are particularly desirable for therapeutic treatment. Such antibodies can be produced, for example, using transgenic mice that are incapable of expressing endogenous immunoglobulin heavy and light chains genes, but that can express bovine (or canine) heavy and light chain genes. The transgenic mice are immunized in the normal fashion with a selected antigen, *e.g.,* all or a portion of a polypeptide of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g*., U.S. Patent 5,625,126; U.S. Patent 5.633.425; U.S. Patent 5.569,825; U.S. Patent 5,661,016; and U.S. Patent 5.545.806. In addition, companies such as Abgenix, Inc. (Fremont, CA), can be engaged to provide human antibodies directed against a selected antigen using technology similar to that described above.

Such completely species-specific antibodies that recognize a selected epitope can be generated using a technique preferred to as "guided selection. In this approach a selected non-human monoclonal antibody,*e.g*., *a m*ouse antibody, is used to guide the selection of a completely species-specific, e.g., bovine or canine, antibody recognizing the same epitope. (Jespers et al. (1994) Bio/technology 12:899-903).

Alternatively, techniques described for the production of single chain antibodies (U.S. Patent 4,946,778; Bird, 1988. Science 242:4.23-426; Huston et al., 1988, Proc. Natl. Acad. Sci. USA 85:5879-5883; and Ward et al., 1989, Nature 334:544-546) can be adapted to produce single chain antibodies against LP1454,LP1118, LP1939, MCEII, CADF-like1, CADF-like2, CADF-like3, Lp0022, Lp1499. Lp4337, Lp328 or L21 proteins or polypeptides. Single chain antibodies are formed by linking the heavy and light chain fragments of the Fv region via an amino acid bridge, resulting in a single chain polypeptide.

Antibody fragments that recognize specific epitopes may be generated by known techniques. For example, such fragments include, but are not limited to: the F(ab')₂ fragments, which can be produced by pepsin digestion of the antibody molecule and the Fab fragments, which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity.

Antibodies to a LP1454, LP1118, LP1939, MCEII, CADF-like1, CADF-like2, CADF-like3, Lp0022, Lp1499, Lp4337, Lp328 or L21 protein and/or polypeptide can, in turn, be utilized to generate anti-idiotype antibodies that "mimic" LP1454. LP1118, LP1939, MCEII, CADF-like1, CADF-like2, CADF-like3, Lp0022, Lp1499, Lp4337, Lp328 or L21, using techniques well known to those skilled in the art, (See, *e.g.,* Greenspan & Bona, 1993, FASEB J 7(5):437-444; and Nissinoff, 1991, J. Immunol. 147(8):2429-2438).

**5.7 Serological Diagnosis Techniques: KELA ELISA, Western Blotting (Immunoblots) and PCR**

Recombinant LP1454, LP 1118, LP1939, MCEII. CADF-like1, CADF-like2, CADF-like3, Lp0022, Lp1499, Lp4337, Lp328 or L21 proteins used as diagnostic agents are also provided. One diagnostic technique in which the foregoing OMPs can be used is kinetic enzyme-linked immunosorbent assay (KELA ELISA) (Appel MJ, et al. Experimental Lyme disease in dogs produces arthritis and persistent infection, J Infect Dis. 1993 Mar;167(3):651-64; Chang YF, et al., Recombinant OspA protects dogs against infection and disease caused by Borrelia burgdorferi. Infect Immun. 1995 Sep; 63(9):3543-9). KELA measures the levels of serum antibodies to *Leptospira* that are present in a serum sample. In this diagnostic technique, diluted serum (1:100 dilution) is added to duplicate wells in microtiter plates that contain antigens of the OMP of interest.

Antigens can be expressed and purified using methods known in the art, for example, the antigens can be expressed in *E*. coli, as described herein in Section 6.

The bound antibodies are then detected by using secondary antibodies, e.g., goat anti- canine, bovine, equine or porcine antibodies of heavy and light chain specificity conjugated to horseradish peroxidase (HRP). Color development is seen and measured using the chromogen tetramethylbenzidine with H₂O₂ as a substrate, which is measured kinetically and expressed as the slope of the reaction rate between the enzyme and substrate solution. Each unit of slope is designated as a KELA unit. The cutoff point between positive and negative samples is then confirmed by Western blotting against a French-pressed sample, e.g., *Leptospira* sample.

Western blot analysis can be carried using standard art-known methods (e.g., Appel MJ, et al. Experimental Lyme disease in dogs produces arthritis and persistent infection, J Infect Dis. 1993 Mar; 167(3):651-64; Chang YF, et al., Recombinant OspA protects dogs against infection and disease caused by Borrelia burgdorferi, Infect Immun. 1995 Sep;63(9):3543-9.). Recombinant LP1454, LP1118. LP1939, MCEII, CADF-likel, CADF-like2, CADF-like3, Lp0022, Lp1499, Lp4337, Lp328 or L21 can be used as antigens and are subjected to sodium dodecyl sulfate-polyacrylamide gel electrophoresis (SDS-PAGE). Western blot analysis is performed in a miniblotter. Test sera from experimental animals are used as a first antibody, followed by, for example, a goat anti- canine, bovine, equine, canine or porcine IgG conjugated to HRP as a second antibody. Bands are developed by using substrates, such as 24 µg of 4-chloro-1-naphthol in 8 ml of methyl alcohol, 40 ml of Tris-buffer solution, and 24 µl of 30% H₂O₂.

Another diagnostic technique recombinant LP1454, LP1118, LP1939, MCEII, CADF-likel, CADF-like2, CADF-like3, Lp0022, Lp1499, Lp4337, Lp328 or L21 proteins can be used for is PCR diagnosis. The DNA from biopsy samples of kidney or from post-mortem tissues or blood are extracted by techniques known in the art (Palaniappan RU, et al. Evaluation of lig-based conventional and real time PCR for the detection of pathogenic leptospires. Mol Cell Probes. 2005 Apr;19(2):111-7. Epub 2004 Dec 15; Chang YF, et al. Recombinant OspA protects dogs against infection and disease caused by Borrelia burgdorferi. Infect Immun. 1995 Sep;63(9):3543-9). To prevent contamination of the mixtures and samples, DNA extraction, amplification, and detection of PCR products can be performed at different locations, e.g., in different rooms. Amplification of rLP1454, rLP1118, rLP1939, rMCEII, rCADF-likel, rCADF-like2, rCADF-like3, rLp0022, rLp1499, rLp4337, rLp328 or rL21-specific target sequences can be carried out in a 50-µl reaction mixture. As a positive control, *Leptospira* genomic DNA can be used. As a negative control, distilled water can be used.

The reaction mixture can be put through cycle of DNA amplification, e.g., 40 cycles of amplification using an automated DNA thermal cycler. Each cycle involves beating the reaction mixture to 94°C. from 1 minute, to cause the DNA to denature; cooling of the reaction mixture to 69° C. for 1 minute, to allow the primers to anneal; and then heating the reaction mixture to 72° C. for 2 minutes, to allow primer extension to occur. Gel electrophoresis on a 1.5% agarose gel is done in order to get visualization of the PCR amplification products.

**5.8 Pharmaceutical Compositions and Vaccines**

Pharmaceutical compositions and vaccines are also provided. Such compositions can comprise a therapeutically effective amount of a pharmaceutical composition or vaccine of the invention, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, tale, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the Therapeutic, preferably in purified form, together with a suitably amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. The composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In one embodiment, a pharmaceutical composition can comprise a therapeutically effective amount of an antibody that immunospecifically binds to a *Leptospira* LP1454, LP1118, LP1939, MCEII, CADF-likel, CADF-like2, CADF-like3, Lp0022, Lp1499, Lp4337, Lp328 or L21protein; and a pharmaceutically acceptable carrier.

In another embodiment, a pharmaceutical composition can comprise a therapeutically effective amount of a fragment or derivative of an antibody that immunospecifically binds to a *Leptospira* LP1454, LP1118, LP1939, MCEII, CADF-like1, CADF-like2, CADF-like3, Lp0022. Lp1499, Lp4337, Lp328 or L21 protein; the fragment or derivative containing the binding domain of the antibody; and a pharmaceutically acceptable carrier.

In another embodiment, a pharmaceutical composition can comprise a therapeutically effective amount of a vaccine of the invention; and a pharmaceutically acceptable carrier.

Vaccine formulations for pharmacological administration are also provided. Vaccine formulations can be, for example, DNA vaccine formulations, recombinant vaccine formulations, or T cell epitope vaccine formulations.

Use of isolated and purified antibodies to *Leptospira* LP1454, LP1118, LP1939, MCEII, CADF-like1, CADF-like2, CADF-like3, Lp0022, Lp1499, Lp4337, Lp328 or L21 OMPS to improve vaccines for mammals, including but not limited to cow, pig, dog, sheep and horse, is also provided.

Vaccines and methods for making the vaccines that protect a mammal against a *Leptospira*-related disorder are provided. In particular, a vaccine is provided that elicits active immunity against a leptospirosis infection that contains at least one epitope of a *Leptospira* OMP, wherein the *Leptospira* OMP is selected from the group consisting of LP1454, LP1118, LP1939,MCEII, CADF-likel, CADF-like2, CADF-like3, Lp0022, Lp1499, Lp4337, Lp328 and L21.

A DNA vaccine is provided that elicits active immunity against a leptospirosis infection comprising a DNA encoding at least one epitope of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21.

A vaccine for providing passive immunity to a leptospirosis infection is also provided. The vaccine can comprise antibodies that are directed against at least one epitope of a *Leptospira* OMP*,* wherein the *Leptospira* OMP is selected from the group consisting of LP1454, LP1118, LP1939, MCEII, CADF-likel, CADF-like2, CADF-like3, Lp0022, Lp1499, Lp4337, Lp328 and L21. The antibodies can be selected from the group consisting of polyclonal antibodies and monoclonal antibodies against at least one epitope of a *Leptospira* OMP, wherein the *Leptospira* OMP is selected from the group consisting of LP1454, LP1118, LP1939, MCEII, CADF-likel, CADF-like2, CADF-like3, Lp0022, Lp1499, Lp4337, Lp328 and L21. In a preferred embodiment of the vaccine, the vaccine is provided in a pharmaceutically accepted carrier.

Further, a vaccine for active immunization of a mammal against a leptospirosis infection is provided. The vaccine can comprise an antigen containing at least one epitope of a *Leptospira* OMP, wherein the *Leptospira* OMP is selected from the group consisting of LP1454, LP1118, LP1939, MCEII, CADF-likel, CADF-like2, CADF-like3, Lp0022, Lp1499, Lp4337, Lp328 and L21. In one embodiment, the antigen is a recombinant polypeptide produced in a plasmid in a microorganism other than *Leptospira,* preferably, in an *E. coli.* In a preferred embodiment, the vaccine is provided in a pharmaceutically accepted carrier. In another embodiment, the *Leptospira* OMP antigen can be provided as a fusion polypeptide wherein an amino end and/or a carboxyl end of the antigen is fused to all or a portion of a polypeptide that facilitates the isolation of the antigen from the microorganism in which the antigen is produced. In a preferred embodiment, the polypeptide is selected from the group consisting of glutathione S-transferase, protein A, maltose binding protein, and polyhistidine.

A vaccine for protecting a mammal from leptospirosis infection is also provided. The vaccine can comprise a DNA that encodes at least one epitope of a *Leptospira* OMP, wherein the *Leptospira* OMP is selected from the group consisting of LP1454, LP1118, LP1939, MCEII. CADF-like1, CADF-like2, CADF-like3, Lp0022, Lp1499, Lp4337, Lp328 and L21. In a preferred embodiment, the DNA is operably linked to a promoter to enable transcription of the DNA in a mammalian cell. Preferably, the vaccine is provided in a pharmaceutically accepted carrier.

Methods are provided for using *Leptospira* LP1454, LP1118, LP1939, MCEII, CADF-likel, CADF-like2, CADF-like3, Lp0022, Lp1499, Lp4337, Lp328 or L21 OMPs as vaccines (or to improve vaccines) or as serological diagnostic antigens.

Methods are provided for producing DNA vaccines, recombinant vaccines, and T cell epitope vaccines based on LP1454, LP1118, LP1939, MCEII, CADF-likel, CADF-like2, CADF-like3, Lp0022, Lp1499, Lp4337, Lp328 or L21 OMPs.

A method for vaccinating mammal against a leptospirosis infection is also provided. In one embodiment, the method can comprise: (a) providing a recombinant antigen of the *Leptospira* OMP, wherein the OMP is selected from the group consisting of LP1454, LP1118, LP1939, MCEII, CADF-likel, CADF-like2, CADF-like3, Lp0022, Lp1499, Lp4337, Lp328 and L21 and wherein the OMP is produced from a microorganism culture wherein the microorganism contains a DNA that encodes an antigen of the *Leptospira* OMP; and (b) vaccinating the mammal. Preferably, the vaccine is in a pharmaceutically accepted carrier.

In a preferred embodiment of the method, the recombinant antigen is a fusion polypeptide that is fused at the amino terminus and/or carboxyl terminus to a polypeptide that facilitates the isolation of the recombinant antigen. In particular, the polypeptide can be all or a portion of the polypeptide selected from the group consisting of glutathione S-transferase, protein. A, maltose binding protein, and polyhistidine. Further, the method can comprise producing the antigen from a DNA that is in a plasmid in a microorganism wherein the DNA is operably linked to a promoter which enables transcription of the DNA to produce the recombinant antigen for the vaccine.

A method for vaccinating a mammal against a leptospirosis infection is also provided wherein the method comprises: (a) providing in a carrier solution a DNA in a plasmid that encodes at least one epitope of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21; and (b) vaccinating the mammal with the DNA in the carrier solution. Preferably, the DNA is in a carrier solution that is pharmaceutically accepted for DNA vaccines. In a preferred embodiment, the DNA is operably linked to a promoter to enable transcription of the DNA in a mammalian cell.

A method is further provided for providing passive immunity to a leptospirosis infection in a mammal comprising: (a) providing antibodies selected from the group consisting of polyclonal antibodies and monoclonal antibodies that are directed against at least one epitope of a *Leptospira* OMP, wherein the *Leptospira* OMP is selected from the group consisting of LP1454, LP1118, LP1939, MCEII, CADF-likel, CADF-like2, CADF-like3, Lp0022, Lp1499, Lp4337, Lp328 and L21; and (b) inoculating the mammal. Preferably, the antibodies are provided in a pharmaceutically accepted carrier.

Further still, a method for producing an antigen is provided wherein the method comprises: (a) providing a microorganism in a culture containing a DNA encoding a fusion polypeptide comprising at least one epitope of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21, and a polypeptide that facilitates isolation of the fusion polypeptide; (b) culturing the microorganism in a culture to produce the fusion polypeptide; and (c) isolating the fusion polypeptide.

In one embodiment, the fusion polypeptide is isolated by affinity chromatography which can be affinity chromatography that comprises an IgG-linked resin when the polypeptide consists of all or a portion of protein A, a Ni² resin when the polypeptide is polyhistidine, amylose resin when the polypeptide is all or part of the maltose binding protein, or glutathione Sepharose 4B resin when the polypeptide is all or part of glutathione S-transferase.

A vaccine for a mammal is also provided that comprises an isolated recombinant protein encoded by a cDNA produced from RNA of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21 and a vaccine carrier. In another embodiment, the vaccine for a mammal comprises a recombinant virus vector containing DNA encoding an epitope of a *Leptospira* OMP, wherein the *Leptospira* OMP is selected from the group consisting of LP1454, LP1118, LP1939. MCEII, CADF-like1, CADF-like2, CADF-like3, Lp0022, Lp1499, Lp4337, Lp328 and L21, and a vaccine carrier. In another embodiment, the present invention comprises a DNA vaccine for a mammal comprising a plasmid, containing DNA encoding an epitope of a *Leptospira* OMP.

A method for protecting a mammal against a leptospirosis infection is also provided that comprises providing a vaccine that when injected into the mammal causes the mammal to produce antibodies and cell mediated immunity against a *Leptospira* OMP antigen wherein the antibodies prevent infection by the *Leptospira* organism. In particular, the vaccine comprises at least one epitope of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21 in a vaccine carrier. A vaccine is further provided comprising a recombinant virus vector that expresses the *Leptospira* OMP antigen. The present invention further still provides a vaccine which comprises a DNA plasmid encoding the *Leptospira* OMP antigen.

The present invention further comprises a monoclonal antibody that selectively binds to at least one epitope of a *Leptospira* OMP, wherein the *Leptospira* OMP is selected from the group consisting of LP1454, LP1118, LP1939, MCEII, CADF-like1, CADF-like2, CADF-like3, Lp0022, Lp1499, Lp4337, Lp328 and L21.

Kits are provided comprising in one or more containers an antibody of the invention that immunospecifically bind to a *Leptospira* LP1454, LP1118, LP1939, MCEII, CADF-like1, CADF-like2, CADF-like3, Lp0022, Lp1499, Lp4337, Lp328 or L21 protein.

Kits are also is provided comprising in one or more containers a vaccine of the invention.

Kits are also is provided comprising in one or more containers a plasmid comprising a Lp1454, LP1118, LP1939, MCEII, CADF-like1, CADF-like2, CADF-like3, Lp0022, Lp1499. Lp4337, Lp328 or L21 gene in an expression vector.

**5.8.1 DNA Vaccine**

A recombinant DNA vaccine is provided. In one embodiment, the DNA vaccine comprises:
a) a recombinant DNA wherein the recombinant DNA is selected from the group consisting of:
   a recombinant DNA that encodes a protein having the amino acid sequence as shown in SEQ ID NO: 2 (LP1454),
   a recombinant DNA that encodes a protein having the amino acid sequence as shown in SEQ ID NO: 4 (LP1118),
   a recombinant DNA that encodes a protein having the amino acid sequence as shown in SIiQ ID NO: 6(LP1939),
   a recombinant DNA that encodes a protein having the amino acid sequence as shown in SEQ ID NO: 8 (MCEII),
   a recombinant DNA that encodes a protein having the amino acid sequence as shown in SEQ ID NO: 53 (CADF-like1),
   a recombinant DNA that encodes a protein having the amino acid sequence as shown in SEQ ID NO: 55 (CADF-like2),
   a recombinant DNA that encodes a protein having the amino acid sequence as shown in SEQ ID NO: 57 (CADF-like3),
   a recombinant DNA that encodes a protein having the amino acid sequence as shown in SEQ ID NO: 59 (Lp0022),
   a recombinant DNA that encodes a protein having the amino acid sequence as shown in SEQ ID NO: 61 (Lp1499),
   a recombinant DNA that encodes a protein having the amino acid sequence as shown in SEQ ID NO: 63 (Lp4337),
   a recombinant DNA that encodes a protein having the amino acid sequence as shown in SEQ ID NO: 65 (Lp328),
   a recombinant DNA that encodes a protein having an amino acid sequence as shown in SEQ ID NO: 67 (L21),
   a recombinant DNA. that encodes an immunogenic epitope or immunologically active fragment of any of the above, and
   a recombinant DNA that encodes a protein fragment of at least 10%, 20%, 30%, 40%, 50%, 60% 70%, 80%, 90% or 95% of the length of the amino acid sequence of any of the above; and
b) a vector capable of expressing the recombinant DNA when the recombinant DNA is inserted into the vector, wherein the recombinant DNA is inserted into the vector such that a recombinant protein is expressed when the vector is provided in an appropriate host.

A DNA vaccine can be constructed by subcloning the gene of interest into a eukaryotic plasmid vector. Candidate vectors can include, but are not limited to, pcDNA3, pCI, VR1012, and VR1020, all of which are vectors well known in the art. This construct can then be used as a vaccine.

pcDNA3 is well known in the art as a cloning vector and is commercially available from e.g., Invitrogen, Carlsbad, CA. pCl is well known in the art as a mammalian expression vector and is commercially available from e.g., Promega Corporation, Madison, WI. Vectors VR1012 and VR1020 are both known in the art and available from Vical, San Diego, CA.

A LP1454, LP1118, LP1939, MCEII, CADF-like1, CADF-like2, CADF-like3, Lp0022, Lp1499, Lp4337, Lp328 or L21 protein can be used to create a DNA vaccine (reviewed in Robinson, 1997). In addition, any immunologically active portion of these proteins is a potential candidate for the vaccine. A plasmid containing one of these genes in an expression vector is constructed. The gene must be inserted in the correct orientation in order for the genes to be expressed under the control of eukaryotic promoters. Possible promoters include, but are not limited to, the cytomegalovirus (CMV) immediate early promoter, the human tissue plasminogen activator (t-PA) gene (see, e.g., Degen et al., 1986, J. Biol. Chem. 1986 May 25;261(15):6972-85), and the promoter/enhancer region of the human elongation factor alpha (EF-1 α) (see, e.g., Kim et al., Use of the human elongation factor 1 alpha promoter as a versatile and efficient expression system, Gene. 1990 Jul 16;91(2):217-23; Uetsuki et al., Isolation and characterization of the human chromosomal gene for polypeptide chain elongation factor-1 alpha. J. Biol. Chem. 1989 Apr 5; 264(10):5791-8). Orientation can be identified by restriction endonuclease digestion and DNA sequencing.

Expression of these gene products can be confirmed by indirect immunofluorescent staining of transiently transfected COS cells. CHO cells, or other suitable cells. The same plasmid without these genes is used as a control. Plasmid DNA is transformed into *E. coli* DH5α. DNA can be purified using art-known methods, e.g., by cesium chloride gradients, and the concentration can be determined by a standard protocol known in the art (Nyika et al., 1998, A DNA vaccine protects mice against the rickettsial agent Cowdria ruminantium, Parasite Immunol. 1998 Mar; 20(3): 111-9).

Once the DNA is purified, the vector containing the insert DNA can be suspended in phosphate buffer saline solution and directly injected into test animals such as a dog or cattle. Inoculation into a muscle can be done using standard methods, with a needle or intravenously. Alternatively, a gene gun can be used to transport DNA-coated gold beads into cells using standard art-known methods (Fynan et al., 1993, DNA vaccines: protective immunizations by parenteral, mucosal, and gene-gun inoculations, Proc. Nat'l Acad. Sci. U.S.A. 1993 Dec 15;90(24):11478-82.).

The inoculated host can express the plasmid DNA in its cells, and can produce a protein that raises an immune response, Each of the newly identified genes can be used to create a vaccine by this technique.

CpG molecules can be used as an adjuvant in the vaccine (Klinman et al., 1997, Contribution of CpG motifs to the immunogenicity of DNA vaccines. J. Immunol. 1997 Apr 15;158(8):3635-9). Adjuvants are materials that help antigens or increase the immune response to an antigen. The motifs consist of an unmethylated CpG dinucleotide flanked by two 5'purines and two 3' pyrimidines. Oligonucleotides containing CpG motifs have been shown to activate the immune system, thereby boosting an antigen-specific immune response. This effect can be utilized by mixing the CpG oligonucleotides with the DNA vaccine, or physically linking the CpG motifs to the plasmid DNA using standard techniques known in the art.

In a specific embodiment, a method for producing a vaccine against a *Leptospira-*related disorder is provided comprising: a) providing a recombinant DNA, wherein the recombinant DNA is selected from the group consisting of:
a recombinant DNA that encodes a protein having the amino acid sequence as shown in SEQ ID NO: 2 (LP1454),
a recombinant DNA that encodes a protein having the amino acid sequence as shown in SEQ ID NO: 4 (LP1118),
a recombinant DNA that encodes a protein having the amino acid sequence as shown in SEQ ID NO; 6 (LP1939),
a recombinant DNA that encodes a protein having the amino acid sequence as shown in SEQ ID NO: 8 (MCEII),
a recombinant DNA that encodes a protein having the amino acid sequence as shown in SEQ ID NO: 53 (CADF-like1),
a recombinant DNA that encodes a protein having the amino acid sequence as shown in SEQ ID NO: 55 (CADF-like2),
a recombinant DNA that encodes a protein having the amino acid sequence as shown in SEQ ID NO: 57 (CADF-like3),
a recombinant DNA that encodes a protein having the amino acid sequence as shown in SEQ ID NO: 59 (Lp0022),
a recombinant DNA that encodes a protein having the amino acid sequence as shown in SEQ ID NO: 61 (Lp1499),
a recombinant DNA that encodes a protein having the amino acid sequence as shown in SEQ ID NO: 63 (Lp4337),
a recombinant DNA that encodes a. protein having the amino acid sequence as shown in SEQ ID NO: 65 (Lp328),
a recombinant DNA that encodes a protein having an amino acid sequence as shown in SEQ ID NO: 67 (L21),
a Recombinant DNA, that encodes an immunogenic epitope or immunologically active fragment of any of the above, and
a recombinant DNA that encodes a protein fragment of at least 10%, 20%, 30%, 40%, 50%, 60% 70%, 80%, 90% or 95% of the length of the amino acid sequence of any of the above;

b) providing a vector capable of expressing the recombinant. DNA when the recombinant DNA is inserted into the vector; and
c) inserting the recombinant DNA into the vector, wherein the recombinant DNA is inserted into the vector such that a recombinant protein is expressed when the vector is provided in an appropriate host.

In another embodiment, a method for producing a vaccine against a *Leptospira-*related disorder is provided comprising:
a) providing a Recombinant DNA, wherein the recombinant DNA is selected from the group consisting of:
   a recombinant DNA that encodes a protein having the amino acid sequence as shown in SEQ ID NO: 2 (LP1454),
   a Recombinant DNA that encodes a protein having the amino acid sequence as shown in SEQ ID NO: 4 (LP1118),
   a recombinant DNA that encodes a protein having the amino acid sequence as shown in SEQ ID NO: 6 (LP1939),
   a recombinant DNA that encodes a protein having the amino acid sequence as shown in SEQ ID NO: 8 (MCEII),
   a recombinant DNA that encodes a protein having the amino acid sequence as shown in SEQ ID NO: 53 (CADF-like1),
   a recombinant DNA that encodes a protein having the amino acid sequence as shown in SEQ ID NO: 55 (CADF-like2),
   a recombinant DNA that encodes a protein having the amino acid sequence as shown in SEQ ID NO: 57 (CADF-like3),
   a recombinant DNA that encodes a protein having the amino acid sequence as shown in SEQ ID NO: 59 (Lp0022),
   a recombinant DNA that encodes a protein having the amino acid sequence as shown in SEQ ID NO: 61 (Lp1499),
   a recombinant DNA that encodes a protein having the amino acid sequence as shown in SEQ ID NO: 63 (Lp4337),
   a recombinant DNA that encodes a protein having the amino acid sequence as shown in SEQ ID NO: 65 (Lp328),
   a recombinant DNA that encodes a protein having an amino acid sequence as shown in SEQ ID NO: 67 (L21),
   a recombinant DNA that encodes an immunogenic epitope or immunologically active fragment of any of the above, and
   a recombinant DNA that encodes a protein fragment of at least 10%, 20%, 30%, 40%, 50%, 60% 70%, 80%, 90% or 95% of the length of the amino acid sequence of any of the above;
b) constructing an expression vector comprising a plasmid, wherein the plasmid comprises the recombinant DNA; and
c) inoculating a host with the expression vector, wherein the recombinant DNA is expressed in the host to produce a recombinant protein, and wherein the expressed protein raises an immune response in the host.

**5.8.2 Recombinant Vaccine**

A recombinant vaccine is provided. In one embodiment, the vaccine comprises: a recombinant protein, wherein the recombinant protein is selected from the group consisting of:
a protein having the amino acid sequence as shown in SEQ ID NO: 2 (LP1454),
a protein having the amino acid sequence as shown in SEQ ID NO: 4 (LP1118),
a protein having the amino acid sequence as shown in SEQ ID NO: 6 (LP1939),
a protein having the amino acid sequence as shown in SEQ ID NO: 8 (MCEII),
a protein having the amino acid sequence as shown in SEQ ID NO: 53 (CADF-like1),
a protein having the amino acid sequence as shown in SEQ ID NO: 55 (CADF-like2),
a protein having the amino acid sequence as shown in SEQ ID NO: 57 (CADF-like3),
a protein having the amino acid sequence as shown in SEQ ID NO: 59 (Lp0022),
a protein having the amino acid sequence as shown in SEQ ID NO: 61 (Lp1499),
a protein having the amino acid sequence as shown in SEQ ID NO: 63 (Lp4337),
a protein having the amino acid sequence as shown in SEQ ID NO: 65 (Lp328),
a protein having an amino acid sequence as shown in SEQ ID NO: 67 (L21),
an immunogenic epitope or immunologically active fragment of any of the above, and
a protein fragment of at least 10%, 20%, 30%, 40%, 50%, 60% 70%, 80%, 90% or 95% of the length of the amino acid sequence of any of the above.

To develop a recombinant vaccine, an OMP gene, such as LP1454, LP1118, LP1939, MCEII, CADF-like 1, CADF-like2, CADF-like3, Lp0022, Lp1499, Lp4337, Lp328 or L21, is individually subcloned into overexpression vectors, and then purified for vaccine development using methods known in the art. The OMP of interest can be expressed in a plasmid with a strong promoter such as the tae, T5, or T7 promoter. Alternatively, immunologically active fragments of the OMP can be used in the development of a vaccine. Each of these genes can be subcloned into a plasmid and transformed into an *E. coli* strain as described above.

The recombinant protein can be overexpressed, using art-known techniques, in a vector with a strong promoter. Vectors that can be suitable for use in this technique include pREST (Invitrogen Inc., Calif.), pKK233-3 (Pharmacia, Calif.), and the pET system (Promega, Wis.), although any vector with a strong promoter can be used. After overexpression, the proteins can be purified and mixed with adjuvant. Potential adjuvants include, but are not limited to, aluminum hydroxide, Quil A or MONTANIDE®. Quil A is well known in the art as a highly refined form of saponin, a plant-derived biochemical that stimulates both cell-mediated and humoral immune responses. MONTANIDE®" is U.S. registered trademark for a specific formulation for an emulsifier produced by the Seppic company (Paris, France). MONTANIDE® is well known in the art for its use as an adjuvant.

The purified protein can be used as immunogen to vaccinate dogs by techniques known in the art. (see, e.g., Palaniappan RU, et al. Immunoprotection of recombinant leptospiral immunoglobulin-like protein A against Leptospira interrogans serovar Pomona infection. Infect Immun. 2006 Mar;74(3):1745-50, incorporated herein by reference).

Briefly, the individual protein is expressed and purified from *E*. *coli.* Then, test animals, such as dogs or cattle, are injected intramuscularly or subcutaneously with the purified recombinant vaccine and adjuvant. This injection can elicit an immune response.

In one embodiment, a method for producing a vaccine against a *Leptospira-related* disorder comprises:
a) providing a recombinant DNA, wherein the recombinant DNA is selected from the group consisting of:
   a recombinant DNA that encodes a protein having the amino acid sequence as shown in SEQ ID NO: 2 (LP1454),
   a recombinant DNA that encodes a protein having the amino acid sequence as shown in SEQ ID NO: 4(LP1118),
   a recombinant DNA that encodes a protein having the amino acid sequence as shown in SEQ ID NO: 6 (LP1939),
   a recombinant DNA that encodes a protein having the amino acid sequence as shown in SEQ ID NO: 8 (MCEII),
   a recombinant DNA that encodes a protein having the amino acid sequence as shown in SEQ ID NO: 53 (CADF-likel),
   a recombinant DNA that encodes a protein having the amino acid sequence as shown in SEQ ID NO: 55 (CADF-like2),
   a recombinant DNA that encodes a protein having the amino acid sequence as shown in SEQ ID NO: 57 (CADF-like3),
   a recombinant DNA that encodes a protein having the amino acid sequence as shown in SEQ ID NO: 59 (Lp0022),
   a recombinant DNA that encodes a protein having the amino acid sequence as shown in SEQ ID NO: 61 (Lp1499),
   a recombinant DNA that encodes a protein having the amino acid sequence as shown in SEQ ID NO: 63 (Lp4337),
   a recombinant DNA that encodes a protein having the amino acid sequence as shown in SEQ ID NO: 65 (Lp328),
   a recombinant DNA that encodes a protein having an amino acid sequence as shown in SEQ ID NO: 67 (L21),
   a recombinant DNA that encodes an immunogenic epitope or immunologically active fragment of any of the above, and
   a recombinant DNA that encodes a protein fragment of at least 10%, 20%, 30%, 40%, 50%, 60% 70%, 80%, 90% or 95% of the length of the amino acid sequence of any of the above;
b) providing a vector capable of expressing the recombinant DNA when the recombinant DNA is inserted into the vector;
c) inserting the recombinant DNA into the vector;
d) providing a bacterial strain;
e) transforming the vector into the bacterial strain such that a recombinant protein is expressed when the vector is transformed into the bacterial strain; and
f) harvesting the recombinant protein from the bacterial strain.

In another embodiment, a method for producing a recombinant vaccine is provided comprising:
subcloning a LP1454, LP1118, LP1939, MCEII, CADF-like1, CADF-like2, CADF-like3, Lp0022, Lp1499, Lp4337, Lp328 or L21 gene or a DNA sequence encoding an immunologically active portion of a LP1454. LP1118, LP1939, MCEII, CADF-like1, CADF-like2, CADF-like3, Lp0022, Lp1499, Lp4337, Lp328 or L21 protein into an overexpression vector,
expressing a LP1454, LP11 18, LP1939, MCEII CADF-like1, CADF-like2, CADF-like3, Lp0022, Lp1499, Lp4337, Lp328 or L21 protein, peptide or immunologically active fragment; and
purifying the expressed LP1454, LP1118, LP1939, MCEII, CADF-like1, CADF-like2, CADF-like3, Lp0022, Lp1499, Lp4337, Lp328 or L21 protein, peptide or immunologically active fragment.

**5.8.3 Epitope Mapping and T-Cell Epitope Vaccine**

Direct cell cytotoxicity mediated by CD8⁺ T lymphocytes (CTL) is the major mechanism of defense against intracellular pathogens. These effector lymphocytes eliminate infected cells by recognizing short peptides associated with MHC class I molecules on the cell surface. Exogenous antigens enter the endosomal pathway and are presented to CD4⁺ T cells in association with class II molecules whereas endogenously synthesized antigens are presented to CD8⁺ T cells in association with MHC class I molecules.

*Leptospira* is an intracellular pathogen that resides in monocytes and macrophages. A *Leptospira-*specific CTL response can be generated that can eliminate the organism from monocytes or macrophages of infected animals.

To increase the protective response of a protein vaccine, a test animal can be immunized with selective epitopes of the protein. The rationale behind this is that an epitope vaccine contains the most relevant immunogenic peptide components without the irrelevant portions. Therefore, a search can be performed for the most highly antigenic portions of the newly identified proteins.

In one embodiment, a T-cell epitope vaccine is provided. The T-cell epitope vaccine can comprise a recombinant protein, wherein the recombinant protein comprises a T-cell epitope, and wherein the T-cell epitope comprises an amino acid peptide fragment of a protein selected from the group consisting of:
a protein having the amino acid sequence as shown in SEQ ID NO: 2 (LP1454),
a protein having the amino acid sequence as shown in SEQ ID NO: 4 (LP1118),
a protein having the amino acid sequence as shown in SEQ ID NO: 6 (LP1939),
a protein having the amino acid sequence as shown in SEQ ID NO: 8 (MCEII),
a protein having the amino acid sequence as shown in SEQ ID NO: 53 (CADF-likel),
a protein having the amino acid sequence as shown in SEQ ID NO: 55 (CADF-like2),
a protein having the amino acid sequence as shown in SEQ ID NO: 57 (CADF-like3),
a protein having the amino acid sequence as shown in SEQ ID NO: 59 (Lp0022),
a protein having the amino acid sequence as shown in SEQ ID NO: 61 (Lp1499),
a protein having the amino acid sequence as shown in SEQ ID NO: 63 (Lp4337),
a protein, having the amino acid sequence as shown in SEQ ID NO: 65 (Lp328),
a protein having an amino acid sequence as shown in SEQ ID NO: 67 (L21),
an immunogenic epitope or immunologically active fragment of any of the above, and
a protein fragment of at least 10%, 20%, 30%, 40%, 50%, 60% 70%, 80%, 90% or 95% of the length of the amino acid sequence of any of the above.

To identify T-cell epitopes from a newly discovered OMP or putative OMP, an initial electronic search for homologous sequences to known T-cell epitopes is performed using standard art-known searching methods such as BLAST searching in the NCBI databases.

In once embodiment, a T-cell epitope consisting of or comprising a fragment of a *Leptospira* OMP, wherein the *Leptospira* OMP is selected from the group consisting of LP1434, LP1118, LP1939, MCEII, CADF-like1, CADF-like2. CADF-like3, Lp0022, Lp1499, Lp4337, Lp328 and L21 is provided. In a specific embodiment, the T-cell epitope consists of at least 10 (continuous) amino acids of the Leptospira OMP is provided, In other embodiments, the fragment consists of at least 20 or 50 amino acids of the *Leptospira* OMP protein. In specific embodiments, such fragments are not larger than 35, 100 or 200 amino acids. Derivatives or analogs of *Leptospira* OMP T-cell epitopes include but are not limited to those molecules comprising regions that are substantially homologous to *Leptospira* OMP T-cell epitopes or fragments thereof (e.g., in various embodiments at least 60% or 70% or 80% or 90% or 95% identity over an amino acid sequence of identical size or when compared to an aligned sequence in which the alignment is done by a computer homology program known in the art) or whose encoding nucleic acid is capable of hybridizing to a coding *Leptospira* OMP epitope sequence, under stringent, moderately stringent, or nonstringent conditions.

In addition, extensive T-cell, epitope mapping can be carried out. Each LP1454, LP1118, LP1939, MCEII, CADF-like1, CADF-like2, CADF-like3, Lp0022. Lp1499, Lp4337, Lp328 or L21 OMP can be tested for immunogenic peptide fragments. Mapping of T-cell epitopes is well known in the art (see, e.g., Launois et al., 1994, T-cell-epitope mapping of the major secreted mycobacterial antigen Ag85A in tuberculosis and leprosy, Infect Immun. 1994 Sep; 62(9):3679-87; Lee and Horwitz, 1999, T-cell epitope mapping of the three most abundant extracellular proteins of Mycobacterium tuberculosis in outbred guinea pigs, Infect Immun. 1999 May;67(5);2665-70).

Briefly, short, overlapping peptide sequences (9-20 amino acids) can be synthesized over the entire length of the protein in question. These short peptide fragments can be tested using healthy cows, which can be immunized with the protein of interest. Peripheral blood mononuclear cells from the cows can be tested for T-cell stimulatory and IFN-γ inducing properties. Those fragments that elicit the strongest response may be preferred candidates for a T-cell epitope vaccine.

Once fragments are identified that can make a preferred epitope, a recombinant adenylate cyclase of *Bordetella bronchiseptica* is constructed carrying a *Leptospira* CD8⁺ T cell epitope. The adenylate cyclase toxin (CyuA) of *Bordetella bronchiseptica* elicits an immune response. In addition, CyaA is well suited for intracytoplasmic targeting. Its catalytic domain (AC), corresponding to the N-tenninal 400 amino acid residues of the 1,706-residue-long protein, can be delivered to many eukaryotic cells, including cells of the immune system. Also, toxin internalization is independent of receptor-mediated endocytosis, suggesting that the catalytic domain can be delivered directly to the cytosol of target cells through the cytoplasmic membrane.

The *Pseudomonas aeruginosa* exotoxin A (PE) is another toxin that can be used in this procedure to deliver peptides or proteins into cells (Donnelly et al., 1993, Targeted delivery of peptide epitopes to class I major histocompatibility molecules by a modified Pseudomonas exotoxin, Proc Natl Acad Sci USA. 1993 Apr 15;90(8):3530-4).

Foreign peptides (16 residues) have been inserted into various sites of the AC domain of CyaA without altering its stability or catalytic and calmodulin-binding properties. Thus, protein engineering allows the design and delivery of antigens that specifically stimulate CTLs.

The adenylate cyclase (AC) toxin (cya) gene of *B*. *bronchiseptica* has been cloned. A synthetic double-stranded oligonucleotide encoding a 9 to 20 amino acid class I T cell epitope of either LP1454, LP1118, LP1939 or MCEII, can be designed using standard methods according to *B. bronchiseptica* codon usage. The complementary oligonucleotides can be inserted in the hypervariable region of the cloned AC-coding sequence of the cya. This technique is known in the art in other systems (see, e.g., Sebo et al., 1995, Cell-invasive activity of epitope-tagged adenylate cyclase of Bordetella pertussis allows in vitro presentation of a foreign epitope to CD8+ cytotoxic T cells, Infect Immun. 1995 Oct;63(10):3851-7; Guermonprez et al., 1999, Direct delivery of the Bordetellape pertussis adenylate cyclase toxin to the MHC class I antigen presentation pathway. J. Immunol. 1999 Feb 15;162(4):1910-6).

Recombinant plasmids carrying the chimeric cya gene can be sequenced using standard sequencing methods to determine the copy number and orientation of the inserted epitope. A plasmid with a complete copy of the insert that specifies the T-cell epitope (CD8⁺) in the correct orientation can be chosen from the sequenced plasmids. The ability of the new chimeric protein to enter eukaryotic cells can be used to ensure intracellular targeting of the epitopes (Fayolle et al., 1996, Therapy of murine tumors with recombinant Bordetella pertussis adenylate cyclase carrying a cytotoxic T cell epitope, J. Immunol. 1999 Apr 1;162(7):4157-62).

A vaccine can be created in several ways. For example, a recombinant chimeric protein can be purified and used to inoculate test animals, e.g., dogs or cows. Alternatively, an attenuated *B*. *bronchiseptica* strain that carries a T-cell epitope gene or a LP1454, LP1118, LP1939, MCEII, CADF-like1, CADF-like2, CADF-like3, Lp0022, Lp1499. Lp4337, Lp328 or L21 gene by in-frame insertion into adenylate cyclase can be created by allelic-exchange as known in the art (Cotter PA and Miller JF. BvgAS-mediated signal transduction: analysis of phase-locked regulatory mutants of Bordetella bronchiseptica in a rabbit model. Infect Immun. 1994 Aug; 62(8);3381-90).

In a specific embodiment, a method for producing a T-cell epitope vaccine is provided that comprises:
a) providing a recombinant protein that comprises a T-cell epitope, wherein the T-cell epitope comprises an amino acid peptide fragment of a protein selected from the group consisting of:
   a protein having the amino acid sequence as shown in SEQ ID NO: 2 (LP1434),
   a protein having the amino acid sequence as shown in SEQ ID NO: 4 (LP1118),
   a protein having the amino acid sequence as shown in SEQ ID NO: 6 (LP1939),
   a protein having the amino acid sequence as shown in SEQ ID NO : 8 (MCEII),
   a protein having the amino acid sequence as shown in SEQ ID NO: 53 (CADF-like1),
   a protein having the amino acid sequence as shown in SEQ ID NO: 55 (CADF-like2),
   a protein having the amino acid sequence as shown in SEQ ID NO: 57 (CADF-like3),
   a protein having the amino acid sequence as shown in SEQ ID NO: 59 (Lp0022),
   a protein having the amino acid sequence as shown in SEQ ID NO: 61 (Lp1499),
   a protein having the amino acid sequence as shown in SEQ ID NO: 63 (Lp4337),
   a protein having the amino acid sequence as shown in SEQ ID NO: 65 (Lp328),
   a protein having an amino acid sequence as shown in SEQ ID NO: 67 (L21),
   an immunogenic epitope or immunologically active fragment of any of the above, and
   a protein fragment of at least 10%, 20%, 30%, 40%, 50%, 60% 70%, 80%, 90% or 95% of the length of the amino acid sequence of any of the above;
b) identifying the T-cell epitope from the protein;
c) inserting DNA encoding the T-cell epitope into a construct capable of expressing the T-cell epitope as a protein; and
d) harvesting the protein.

Finally, protection against leptospirosis infection in test animals, e.g., dogs, cows, pigs, or horses, that are vaccinated with the adenylate cyclase- LP1454, -LP1118, -LP1939, - MCEII, -CADF-like1, -CADF-like2, -CADF-like3, -Lp0022, -Lp1499, -Lp4337,- Lp328 or - L21 chimeric protein can be determined. Using standard methods, wild type and recombinant ACs and CyAs can be diluted to working concentrations in PBS and the chimeric protein can be injected into test animals, e.g., dogs or cows, either intramuscularly or subcutaneously. Alternatively, the T-cell epitope can be inserted into the adenylate cyclase gene of an attenuated *B. bronchiseptica* strain in frame, and the test animals subsequently vaccinated or inoculated with live bacteria.

Recombinant antigens are promising candidates for human and animal vaccination against various pathogens. However, a serious drawback is the poor immunogenicity of recombinant antigens as compared to native antigens, A major challenge in the development of a new recombinant vaccine is, therefore, to have a new adjuvant system that increases the immunogenicity of antigens. Cytokines are powerful immunoregulatory molecules. Cytokines that can be used as adjuvants include, but are not limited to,IL-12 (interleukin- 12), GM-CSF (granulocyte-macrophage colony stimulating factor), IL-1β (interleukin-1β) and γ-IFN (gamma interferon).

These cytokines can have negative side effects including pyrogenic and/or proinflammatory symptoms in the vaccinated host. Therefore, to avoid the side effects of a whole cytokine protein, an alternate approach can be to use synthetic peptide fragments with the desired immunostimulatory properties. The nonapeptide sequence VQGEESNDK (SEQ ID NO: 15) of IL-1β protein is endowed with powerful immuno-enhancing properties, and is discussed here to illustrate the use of a cytokine to increase immunogenicity.

This nonapeptide can be inserted into the OMP, e.g., a LP1454, LP1118, LP1939 or MCEII protein, and its immunogenicity can be compared to that of the native protein. Reportedly, the insertion of this sequence into a poorly immunogenic recombinant antigen increases the chance of a strong protective immune response after vaccination. This peptide cam enhance the *in vivo* immune response against both T-dependent and T-independent antigens. The bovine IL-1 β, sequence may mimic many immunomodulatory activities of the entire molecule of IL-1 β while apparently lacking many of its undesirable proinflammatory properties. This strategy can be employed to increase the immunogenicity of the OMP antigens.

The efficacy of the recombinant proteins as vaccines can be tested in cattle, pigs, horses or dogs. The purified protein can be injected, e.g., intraperitoneally, into the animal of interest.

For example, when testing the efficacy of the recombinant protein as vaccines in dogs, specific pathogen free (SPF) dogs can be used. The SPF dogs can be divided into five groups: one group can be given recombinant adenylate cyclase *of Bordetella bronchiseptica* carrying CD8⁺ T cell epitopes derived from LP1454, LP1118, LP1939, MCEII, CADF-like1, CADF-like2, CADF-like3 , Lp0022, Lp1499, Lp4337, Lp328 or L21, one group is given recombinant adenylate cyclase of *Bordetella bronchiseptica* as a control, one group can be given the LP1454, LP1118, LP1939, MCEII, CADF-like1, CADF-like2, CADF-like3, Lp0022, Lp1499, Lp4337, Lp328 or L21 protein plus a bovine IL-1β 163-171 insert, one group can be given a T cell epitope derived from LP1454, LP1118, LP1939, MCEII, CADF-likel, CADF-like2, CADF-like3, Lp0022, Lp1499, Lp4337, Lp328 or L21 alone, and the last group can be given PBS as a negative control.

Animals can be vaccinated using standard methods known in the art (see, e.g., Chang, Y.-F. et al. 1995. Recombinant OspA Protects Dogs against Infection and Disease Caused by Borrelia burgdorferi, Infection and Immunity 63 (9): 3543-3549).

For example, dogs can be vaccinated twice (e.g., 30-40 µg for each vaccination). Dogs can be challenged 21 days after the last vaccination with a virulent strain of *Leptospira* spp. At day five postchallenge, approximately 1 ml blood from each dog can be collected in an EDTA tube. Whether the vaccinated groups eliminate the organisms as compared to that of the control group can be tested by standard culture and PCR methods.

Amplification of the gene product can be performed using standard methods known in the art. For example, two primers derived from the genes cloned can be used to amplify the gene product from the tissues or blood samples from these dogs. The internal primer can also be designed for use as an oligonucleotide probe to hybridize the PCR gene product.

**5.9 Methods for prevention and for treatment of a *Leptospira*-related disorder**

A method is provided for preventing a *Leptospira*-related disorder in an animal or human subject in need thereof comprising administering an amount of antibody of the invention sufficient to confer immunity to the *Leptospira-*related disorder to the subject.

A method is also provided for preventing a *Leptospira*-related disorder in an animal or human subject in need thereof, comprising administering an amount of a vaccine of the invention sufficient to confer immunity to the *Leptospira*-related disorder to the subject.

A method is also provided for treating *Leptospira*-related disorder in an animal or human subject in need thereof, comprising administering an amount of an antibody of the invention sufficient to inhibit or decrease the activity of a *Leptospira* pathogen.

In one embodiment, the method comprises administering an amount of a vaccine of the invention sufficient to inhibit or decrease the activity of the *Leptospira* pathogen.

In another embodiment, the amount of the vaccine of the invention administered is sufficient to confer immunity to *Leptospira* infection or a *Leptospira-*relateddisorder to the subject.

In another embodiment, the method for treating the *Leptospira*-related disorder may not involve administration of antibodies or a vaccine of the invention to a subject. For example, antibodies of the invention can be used to kill infectious organisms *in vitro* where eventual intended use is to combat infection in animals or humans.

**5.10 Methods for assaying, diagnosis and monitoring therapy**

Methods are provided for assaying for the presence or activity of a LP1454 LP1116, LP1939, MCEII, CADF-like1, CADF-like2, CADF-like3, Lp0022, LP1499 Lp4337, Lp328 or L21 protein using antibodies to *Leptospira* LP1424, LP1118, LP1939, MCEII, CADF-like1, CADF-like2, CADF-like3, Lp0022, Lp1499, Lp4337, Lp328 or L21 OMPS or fragments thereof.

Methods are provided for diagnosing a *Leptospira*-related disorder in a subject comprising:

providing one or more antibodies to a *Leptospira* LP1454, LP1118, LP1939, MCEII, CADF-like1, CADF-like2, CADF-like3, Lp0022, Lp1499, Lp4337, Lp328 or L21 OMP or fragment thereof;

collecting a cell or tissue sample, e.g., a blood sample, from the subject,

contacting the sample with the antibodies; and

assaying for the presence or activity of a LP1454, LP1118, LP1939, MCEII. CADF-like1, CADF-like2, CADF-like3, Lp0022, Lp1499, Lp4337, Lp328 or L21 protein in the sample using antibodies to the OMP or fragment thereof;

wherein presence or activity of a LP1454, LP1118, LP1939, MCEII, CADF-like1, CADF-like2, CADF-like3, Lp0022, Lp1499, Lp4337, Lp328 or L21 protein is indicative of the presence of the *Leptospira*-related disorder in the subject.

Methods are provided for diagnosing or screening for the presence of a *Leptospira*-related disorder in a subject comprising measuring the level of a LP1454, LP1118, LP1939, MCEII, CADF-like1, CADF-like2, CADF-like3, Lp0022, Lp1499, Lp433 7, Lp328 or L21 protein or protein functional activity in a sample derived from the subject, in which a decrease in the level of LP1454, LP1118. LP1939, MCEII, CADF-like1, CADF-like2, CADF-like3, Lp0022, Lp1499, Lp4337, Lp328 or L21 protein or protein functional activity in the sample, relative to the level of LP1454, LP1118, LP1939, MCEII, CADF-like1, CADF-like2, CADF-like3, Lp0022, Lp1499, Lp4337, Lp328 or L21 protein or protein functional activity found in an analogous sample not having the disorder, indicates the presence of the disease or disorder or a predisposition for developing the disorder.

Methods are also provided for monitoring therapy using antibodies to *Leptospira* LP1454, LP1118, LP1939, MCEII, CADF-like1, CADF-like2, CADF-like3, Lp0022, LpM99, Lp4337, Lp328 or L21 OMPs or fragments thereof.

Methods for using recombinant LP1454, TP1118, LP1939, MCEII, CADF-like1, CADF-like2, CADF-like3, Lp0022, Lp1499, Lp4337, Lp328 or L21 proteins as diagnostic agents are also provided. In one embodiment, a LP1454, LP 1118, LP 1939, MCEII, CADF-like1, CADF-like2, CADF-like3, Lp0022, Lp1499, Lp4337, Lp328 or L21 protein is used in a kinetic enzyme-linked immunosorbent assay (KELA ELISA).

The following examples are offered by way of illustration and not by way of limitation.

### 6. EXAMPLES

**6.1 Example 1: Identification of immunogenic proteins from genome-derived putative outer membrane *of Leptospira***

**6.1.1 Introduction**

Study of *Leptospira* outer membrane proteins (OMPs) can help in the understanding *Leptospira* mode of infection, since *Leptospira* utilize membrane proteins extensively during infection. Since leptospires survive outside as well as inside the host, some OMPs are differentially regulated, for examples lipoprotein LipL36 is downregulated whereas Lig proteins are upregulated during infection (Matsunaga, J., M. A. Barocchi, J. Croda, T. A. Young, Y. Sanchez, I. Siqueira, C. A. Bolin, M. G. Reis, L. W. Riley, D. A. Haake, and A. I. Ko. 2003. Pathogenic Leptospira species express surface-exposed proteins belonging to the bacterial immunoglobulin superfamily. Mol. Microbiol. 49;929-945 Palaniappan, R. U., Y. F. Chang, S. S. Jusuf. S. Artiushin, J. F. Timoney, S. P. McDonough, S. C. Barr. T. J. Divers, K. W. Simpson, P. L. McDonough, and H. O. Mohammed. 2002. Cloning and molecular characterization of an immunogenic LigA protein of Leptospira interrogans. Infect. Immun. 70:5924-5930).

Therefore, the identification of outer membrane proteins that are expressed only during infection can help to develop diagnostic reagents for leptospirosis. 15 putative outer membrane proteins *of Leptospira* that did not show any significant homology with other organisms in the NCBI database were randomly selected from the available genomic sequences of Leptospira and expressed as GST fusion proteins in *Escherichia coli*. Purified recombinant putative outer membrane proteins, along with recombinant conserved region of LigA and B, variable region of LigB and LipL32, were evaluated by immunoblot analysis with bovine sera from naturally infected, experimentally infected and healthy animals. Except for recombinant ("r") protein, rLA1947, all the recombinant outer membrane proteins (OMPs) showed reactivity with experimentally infected bovine sera. However, variable reactivity of OMPs was found with naturally infected sera. Nevertheless, rLP1939, and rMCEII showed consistently strong reactivity to all the naturally infected animals irrespective of MAT titer indicating that these immunogenic proteins *of Leptospira* can serve as effective diagnostic tools or vaccine for leptospirosis.

We investigated the expression and immunogenicity of genome-derived putative outer membrane proteins for the development of effective diagnostic reagents and/or recombinant proteins/DNA vaccine for leptospirosis. We selected 15 outer membrane proteins that did not show any significant homology to other proteins in the NCBI database and expressed them as GST fusion proteins in. *E. coli.* Antigenic analysis of the purified recombinant OMPs, along with conserved region of LigA and B and variable region of LigB and LipL32, were evaluated by immunoblot with sera from experimentally and naturally infected cattle. The data indicate that rLP1939 and rMceII show consistently stronger reactivity with injected serum samples indicating that these proteins can serve as effective diagnostic antigens for leptospirosis.

***6.1.2* Materials and Methods.**

**Bacterial strains and growth.** *L. interrogans serovar* Pomona (NVSL 1427-35-093002) was used in this study. Leptospires were grown on EMJH medium at 30°C. Growth was monitored by dark-field microscopy.

**Antisera.** Experimentally infected bovine sera to serovars Cannicola, Copenhageni, Grippotyphosa and Hardjobovis were previously described (Surujballi, O. P. R. M. Marenger, M. D. Eaglesome, and E. A. Sugden. 1997. Development and initial evaluation of an indirect enzyme-linked immunosorbent assay for the detection of Leptospira interrogans serovar hardjo antibodies in bovine sera. Can. J. Vet. Res. 61:260-266). Infected animal were identified by exhibition of clinical symptoms and MAT. Naturally infected bovine sera were obtained from the New York State Animal Health Diagnostic Center at Cornell University (Ithaca, NY) from animals that tested positive for leptospiral infection by MAT. Negative control bovine sera were obtained from healthy animals using standard methods known in the art. All the sera were collected from the jugular vein and stored at - 20°C until use.

**Microscopic Agglutination Test (MAT).** MAT was carried out as previously described (Cole, J. R., Jr., H. C. Ellinghausen, and H. L. Rubin. 1979. Laboratory diagnosis of leptospirosis of domestic animals. Proc. Annu. Meet. U S Anim. Health Assoc: 189-195) with the whole cell antigens of the following serovars; Pomona, Grippotyphosa, Icterohaemorrhagiae, Hardjo, Canicola, Autumnalis and Bratislava.
**Cloning and expression of leptospiral OMPs.** Primers for the putative outer membrane protein genes were designed without the signal sequences using standard methods known in the art and are listed in Table 1.

Table 1. Primers used to amplify and clone *L. interrogans* serovar specific sequences

| Gene no.^{a} | GenBank Accession Number | SEQ ID NO: | Primer sequences^{c} | Calculated mol Weight (kDa) | Base pair (bp) |
|---|---|---|---|---|---|
| LP1118 | AAN48316 | 16 | CGCGGATCCACGTTAGGCAACT | 34.8 | 873 |
| | | 17 | CCGCTCGAGTTAGTTTTGTTT | | |
| LP1228 | AAN48427 | 18 | CGCGGATCCAATCCGATAACGA AT | 23 | 507 |
| | | 19 | CCGCTCGAGTTAATGATATTCGT TTT | | |
| LP1404 | AAN48603 | 20 | CGCGGATCCAGAGTTCCTTTTTT T | 43 | ~1100 |
| | | 21 | CCGCTCGAGTTAAGGAACGTTT | | |
| MCEI LP2025 | AAN49254 | 22 | TTTGGATCCGCGATTACCGTA | 27 | 753 |
| | | 23 | CTTCTCGAGTTAAAAGCACTT | | |
| LP2268 | AAN49267 | 24 | CGCGGATCCCCCGACTTAAATG T | 50 | 1056 |
| | | 25 | CCGCTCGAGTCAAAGTATCGAA T | | |
| LigBVT | AF534640 | 26 | CGCGGATCCGTCAATAACAACA TTGC | 66 | 1500 |
| | | 27 | CCGCTCGAGTTGGTTTCCTTTTA CGTT | | |
| LP1332 | AAN48531 | 28 | CGCGGATCCATGTTGTTACAC | 36 | 984 |
| | | 29 | CCGCTCGAGCTACTGAGAAATC TTT | | |
| LP1965 | AAN49164 | 30 | CGCGGATCCTCTCCCGAATGG | 28 | ~710 |
| | | 31 | CCGCTCGAGTTATCTCGTATCAA A | | |
| LP1192 | AAN48391 | 32 | CGCGGATCCATAGTACGTTTAA AA | 59.5 | ~1500 |
| | | 33 | CCGCTCGAGTTAAAAACTGTGG GA | | |
| LP1947 | AAN49146 | 34 | CGCGGATCCCAATCAAAATCGG C | 45 | 1140 |
| | | 35 | CCGCTCGAGTTAATCCTCTAAAT CT | | |
| LigCon | AF534640 | 36 | TCCCCCGGGGCTGGCAAAAGA | 66 | 1797 |
| | | 37 | CCCTCGAGAATATCCGTATTAG A | | |
| LP2637 LIPL32 | AAN49836 | 38 | AGGGGAATTCTATGAAAAACTT TCGAT | 32 | 900 |
| | | 39 | CCCTCGAGCTTAGTCGCGTCAG | | |
| LP1495 | AAN48694 | 40 | CGCGGATCCGATCAGATCAACT T | 37.5 | 921 |
| | | 41 | CCGCTCGAGTTAATTTTGTGTTT TT | | |
| LP1939 | AAN49138 | 42 | CGCGGATCCTGCAAACAAGAT | 17 | 411 |
| | | 43 | CGCCTCGAGTTATTGAGAAGCG TAT | | |
| MCEII LP0607 | AAN47806 | 44 | CGCGGATCCGAAAAAGCCGGAA | 25 | 718 |
| | | 45 | CCGCTCGAGTTATAACTTTCCGA AAT | | |
| LP2471 | AAN49670 | 46 | CGCGGATCCTTTCCAGAAATTTC T | 37.5 | 909 |
| | | 47 | CCGCTCGAGTTAATAATATCTTT GT | | |
| LP1931 | AAN49130 | 48 | CGCGGATCCATTGCACAAATC | 42 | ~1100 |
| | | 49 | CCGCTCGAGTCAAAGAGCAAAC TT | | |
| LP1454 | AAN48653 | 50 | CGCGGATCCGAAGAAAGATCCT | 42 | 981 |
| | | 51 | CCGCTCGAGTCATCTTTTATCTT T | | |

| | | | | | |
|---|---|---|---|---|---|
| ^{a}Gene numbers are from GenBank and http://www.chgc.org.cn/. ^{b}For, forward; Rev, reverse. ^{c}Listed 5' to 3' ^{d}N indicates no confirmed location in the genome of *Leptospira* | | | | | |

Genomic DNA of *L. interrogans* serovar Pomona was prepared using a DNAeasy kit (Qiagen, Valencia, CA) and subjected to PCR as described by the manufacturer using Accuprime Taq polymerase (Invitrogen, CA). PCR products were run in a 1% agarose gel and visualized by ethidium bromide staining. PCR products were eluted from the gel using a gel elution kit (Qiagen, Valencia, CA) and cloned into TOPO TA vector as described by the manufacturer (Invitrogen, CA). Except for LP1947, which was subcloned in pRSETA (InVitroGen, CA), all inserts were sub-cloned into the expression vector pGEX-KG (Stratagene) and transformed into *E*. *coli* (BL21 DE3 or CQ21), DNA sequencing was performed with an ABI model 377 automated nucleic acid sequencer at the Bioresource Center, Cornell University. Homology searches were performed using the NCBI database and BLAST (Altschul, S. F., W. Gish, W. Miller, E. W. Myers, and D. J. Lipman. 1990. Basic local alignment search tool. J. Mol. Biol. 215:403-410).

**Expression and purification of recombinant proteins.** Exponentially growing cultures (OD₆₀₀=0.6) of *E*. *coli* (BL21 DE3) or CQ21 harboring recombinant plasmids were induced to synthesize the fusion proteins with 1mM isopropyl-β-D-thiogalactopyranoside (IPTG; Sigma, St. Louis, Mo.). For each recombinant protein, a pilot experiment was performed with 3mL culture volume and the molecular weight of the expressed recombinant protein was checked by SDS-PAGE analysis. Mass cultivation was performed in 500mL of LB broth with ampicillin (50 µg/mL). Bacteria were harvested by centrifugation at 5,000 RPM for 30 min and the cell pellets were washed and suspended in PBS followed by passing through a French pressure cell (American Instruments). The lysates were centrifuged at 8,000 xg for 30 min and the inclusion bodies were washed and purified (Palaniappan, R. U., Y. F. Chang, F. Hassan, S. P. McDonough, M. Pough, S. C. Barr, K. W. Simpson, H. O. Mohammed, S. Shin, P. McDonough, R. L. Zuerner, J. Qu, and B. Roe. 2004. Expression of leptospiral immunoglobulin-like protein by Leptospira interrogans and evaluation of its diagnostic potential in a kinetic ELISA. J. Med. Microbiol. 53:975-984, Palaniappan, R. U., Y. F. Chang, S. S. Jusuf, S. Artiushin, J. F. Timoney, S. P. McDonough, S. C. Barr, T. J. Divers, K. W. Simpson, P. L. McDonough, and H. O. Mohammed. 2002. Cloning and molecular characterization of an immunogenic LigA protein of Leptospira interrogans. Infect. Immun. 70:5924-5930) with model prep cell (BioRad. CA).

The purified protein was dialyzed at 4°C with PBS by changing PBS for at least 4 times. The purified protein was concentrated and lyophilized (Palaniappan, R. U., Y. F. Chang, F. Hassan, S. P. McDonough, M. Pough, S. C. Barr, K. W. Simpson, H. O. Mohammed, S. Shin, P. McDonough, R. L. Zuerner, J. Qu, and B. Roe. 2004. Expression of leptospiral immunoglobulin-like protein by Leptospira interrogans and evaluation of its diagnostic potential in a kinetic ELISA. J. Med. Microbiol. 53:975-984.). The lyophilized protein was suspended with sterile PBS and quantified by the Bradford assay (Bio-Rad).

**SDS-PAGE and western blotting**. SDS-PAGE was performed as previously described (Chang, Y. F., D. P. Ma, J. Shi, and M. M. Chengappa. 1993. Molecular characterization of a leukotoxin gene from a Pasteurella haemolytica-like organism, encoding a new member of the RTX toxin family. Infect. Immun. 61:2089-2095). Equal amounts of recombinant proteins (1.5-2 µg) were loaded into an SDS-PAGE gel and the gel was stained with Coomassie brilliant blue R-250 followed by destaining. For immunoblotting, recombinant proteins were separated by running SDS-PAGE followed by transfer onto a nitrocellulose membrane (Schleicher & Schuell, Keene, N.H.) in a Trans Blot Cell (Bio-Rad Laboratories, Hercules, CA.), After transfer, the membrane was blocked with TBS plus 1% bovine serum albumin (BSA) for 1 h. Antisera were diluted as shown in FIGS. 3A-3B in TBS-BSA and were incubated with the blot for 1 h. After three washings in TBS plus 0.1% Tween 20, the blot was incubated for 1 h with 1:2,000 goat anti-bovine IgG conjugated with alkaline phosphatase (KPL). The blot was washed three times as described above and developed with NBT/BCIP as described by Chang et al. (1995. Recombinant OspA protects dogs against infection and disease caused by Borrelia burgdorferi. Infect. Immun. 63:3543-3549).

**6.1.3 Results**

Identification, expression and purification of outer membrane proteins. The genomic sequences of *L. interrogans* serovar Icterohaemorrghiae encode for 131 putative outer membrane proteins are known in the art (see, for example, the listings at website address: www.chgc,org.cn), 15 outer membrane proteins, including MceI and II, that did not show high homology to proteins of other pathogens within the NCBI database were randomly seleted from the available genomic sequences of *Leptospira.* These 15 OMPs show 98-100% homology with the genomes from *L. interrogans* serovar Pomona and *L. interrogans* serovar Copenhageni.

FIG. 1 shows the homology of hypothesized mammalian cell invasion proteins of *Leptospira,* The top sequence is MceII, a hypothetical MceI-like protein of *L*. *interrogans* serovar Pomona. This protein was renamed "MceII " (Lp0607, SEQ ID NOs: 7-8) to distinguish it from the MceI of *L*. *interrogans* serovar Lai (middle sequence). The middle sequence is Mce I of *L*. *interrogans* serovar Lai (GenBank Accession number AAN49254; SEQ ID NO: 12). The bottom sequence is amino acids 31-293 of Rv1968, an Mce of *Mycobacterium tuberculosis* CDC1551 (GenBank accession number AE000516, SEQ ID NOS: 13-14). The top sequence, MceII, has approximately 30% homology with the middle sequence, MceI of *L*. *interrogans* serovar Lai, and approximately 25-30% homology with the bottom sequence, Rv1968 Mce of *Mycobacterium tuberculosis.*

All these genes encoding OMPs and in addition, LigBVT (variable regions of LigB), LigCon (conserved region of LigA and B) and LipL32, were successfully expressed in *E*. *coli* as glutathione-S-transferase (GST) fusion proteins. These recombinant proteins migrated approximately to the expected molecular weight (FIG. 2A). The results were further confirmed by immunoblotting using polyclonal antibodies to GST (FIG. 2B). FIGS. 3A-3B list the number of base pairs and calculated molecular size for each OMP protein of *Leptospira*.

**Analysis of immunogenicity of fusion proteins with bovine sera from natural infection, experimental infection and heathy cows.** Bovine sera from experimentally infected, naturally infected and healthy cows were evaluated for reactivity with whole cell proteins of serovar Pomona. The experimentally infected and naturally infected sera showed reactivity to whole cell antigens, whereas the sera from healthy cows did not. These sera were also tested for reactivity with GST protein and none of them showed reactivity to GST (data not shown).

To determine the immunogenicity of recombinant proteins specifically to infection with *L*. *borgpetersenii* serovar Hardjo, *L. interrogans* serovar Grippotyphosa *L. interrogans* serovar Copenhagenii and *L. interrogans* serovar Canicola, the immunoblot with purified recombinant outer membrane proteins was probed with sera from experimentally infected cattle. Except for recombinant protein LA 1947, all the recombinant OMPs showed reactivity to the experimental infection. (FIG. 2C). However, LigCon, LipL32, LA 1939 and MceII showed stronger reactivity than other recombinant proteins (FIGS. 3A-3B).

To examine the immunogenicity of recombinant OMPs to natural infection, sera from naturally infected cows with different MAT titers were also evaluated. The recombinant proteins reacted variably to naturally infected sera (For example, see FIG, 2D). However, LigCon, LipL32, LA1939 and MceII showed consistent reactivity to all the naturally infected sera (FIGS. 3A-3B). Control sera from healthy cattle exhibited no antibodies to the recombinant fusion proteins (data not shown).

Sera from cattle experimentally infected with *L*. *borgpetersenii* serovar Hardjo during time course infection studies (Day0. Day28, Day50) were also evaluated for reactivity to the recombinant OMPs. As discussed above, LA 1939 and MceII showed stronger reactivity than other recombinant proteins (FIGS. 3A-3B). These preliminary data indicate that the proteins encoded by LP1939 and MceII may serve as potential diagnostic antigens or vaccines for leptospiral infection, apart from LigA/B.

**6.1.4 Discussion**

Leptospirosis has emerged as a globally important infectious disease. It occurs in urban as well as in rural regions of industrialized and developing countries (Levett, P. N. 2001. Leptospirosis. Clin. Microbiol. Rev. 14:296-326). Mortality and infection rates remains significant and are related to delays in diagnosis, lack of infrastructure and adequate clinical suspicion, and also other poorly understood reasons that may include inherent pathogenicity of some leptospiral strains or genetically determined host immunopathological responses. Diagnostic tools are greatly needed that accurately identify infected animals, and thus, play a vital role in preventing the disease. A highly specific test that could distinguish between infected and noninfected animals within herds can have a greater and immediate impact on the dairy industry than any other current management approach, including vaccination (Masuzawa, T., T. Matsumoto, R. Nakamura, R. Suzuki, T. Shimizu, and Y. Yanagihara. 1990. Protective activity of glycolipid antigen against infection by Leptospira interrogans serovar canicola. J. Gen. Microbiol. 136:327-330).

Because of a low index of clinical manifestations, various kinds of diagnostic methods are currently used, such as culture, PCR assays, microscopic agglutination tests (MAT) and other serological assays (Matsuo, K., E. Isogai, and Y. Araki. 2000. Control of immunologically crossreactive leptospiral infection by administration of lipopolysaccharides from a nonpathogenic strain of Leptospira biflexa. Microbiol. Immunol. 44:887-890). The drawbacks of culture and isolation of leptospires from clinical specimens are that they are insensitive and slow. A real-time quantitative PCR assay using TaqMan chemistry to detect leptospires in clinical and environmental samples has been reported (Palaniappan et al. 2005. Evaluation of lig-based conventional and real time PCR for the detection of pathogenic leptospires. Mol. Cell Probes 19: 111-117). This PCR assay is sensitive and can differentiate between pathogenic and non-pathogenic species. However, it needs advanced instruments and is difficult to apply widely.

Serology is currently the most frequently used diagnostic approach for leptospirosis. The microscopic agglutination test (MAT) is the standard test for serological diagnosis of leptospires because of its high sensitivity and specificity (Murray, R. D. 1990. A field investigation of causes of abortion in dairy cattle. Vet. Rec. 127:543-547). The MAT detects agglutinating antibodies in serum, but requires significant expertise from its users, and interlaboratory variation in results is high. Whole cell antigen-based diagnostic tests for leptospirosis contain undefined antigens that can exhibit cross-reactivity with other bacteria. Therefore, recombinant protein-based, and especially OMP-based, diagnostic tests may serve as an ideal tool for diagnosis of leptospirosis.

To develop more-reliable antigen-based tests such as ELISAs, identification of leptospiral antigens that are expressed or upregulated only during infection was performed in the present study. With the available genomic sequences for *Leptospira,* 15 OMPs of *Leptospira* were chosen and were cloned and expressed in *E*. *coli.* These randomly selected OMPs do not show high homology with other proteins from other pathogens in NCBI database. Analyses of antigenicity of OMPs indicate that all the recombinant proteins except LA 1947 showed immunoreactivity to experimentally infected cattle. Sera from cattle experimentally infected during a time course infection study reacted strongly to LA1939 and MceII. Further evaluation of recombinant OMPs with naturally infected cattle also confirmed the immunogenicity of recombinant OMPs of LA 1939 and MceII. Therefore, LA 1939 and MceII can serve as potential diagnostic antigens.

An *E*. *coli* expression system with GST fusion proteins for OMPs of *Leptospira* was also developed in this study. Stable expression of intact antigen was optimal only with GST fusion protein. The GST affinity tag used in this study has distinct advantages (Deutz, A., K. Fuchs, N. Nowotny, H. Auer, W. Schuller, D. Stunzner, H. Aspock, U. Kerbl, and J. Kofer. 2003. Sero-epidemiological studies of zoonotic infections in hunters--comparative analysis with veterinarians, farmers, and abattoir workers. Wien Klin Wochenschr 115 Suppl 3:61-67). Generally, expression of recombinant proteins as a fusion protein with GST can overcome problems such as toxicity in *E. coli,* low expression levels, inclusion body formation, and insolubility. The disadvantage of GST is the size of the affinity tag, which complicates any downstream immunoassays. The lack of reactivity of sera from experimentally and naturally infected cattle, as well as healthy cows, to rGST confirmed the absence of antibodies to GST proteins in these samples. However, the development of immunoassays without GST can be done to evaluate the utilization of these recombinant antigens in the field samples.

Currently available diagnostic test cannot discriminate vaccination and infection. Apparently, the administration of whole cell antigen vaccines would definitely increase the titer of antibodies to OMPs and other proteins. Therefore, targeting the proteins that are upregulated during infection can be used to develop immunoassays to discriminate infection **and** vaccination. The recombinant OMPs of LP 1939 and MceII can serve, therefore, as an ideal diagnostic tool to identify infection, and further evaluation of these antigens with vaccinated and infected animals can be performed.

In a conclusion. 15 putative OMPs of *Leptospira* were randomly selected, cloned and expressed in *E*. *coli.* The recombinant proteins were evaluated for potential use in diagnosis of leptospirosis. Out of 15 recombinant proteins, LP1939 and MceII showed consistently stronger reactivity to sera from experimentally and naturally infected animals.

**6.2 Example 2: Immunogenicity of recombinant leptospiral outer membrane proteins and their uses as vaccine candidates**

**6.2.1 Introduction**

*Leptospiral* outer membrane proteins (OMPs) can be components of effective vaccines for leptospirosis. Genomic sequencing of *Leptospira* has allowed us to target putative OMPs for the development of recombinant vaccines. We focused on 12 OMPs that had no homology with other organisms listed in the NCBI database except MceI and MceII of *Leptospira*. All putative OMPs were cloned, expressed and purified as glutathione-S-transferase (GST) fusion proteins. Primary screening for immunoprotective potential was performed in hamsters challenged with an LD50 inoculum of low passage serovar Pomona. The fusion proteins rLP1454, rLP1118 118 and rMCEII were protective when administered to hamsters, as compared to rGST, which was administered as the negative control. All these recombinant proteins were evaluated again for protective efficacy based on lethality, histopathological lesions and antibody responses. rLP1454, rLP1118 and rMCEII showed protection as far as lethality is concerned. Histopathological analyses of rLP1454, rLP1118 and rMCEII immunized animals only showed minor renal lesions, in contrast to rGST-immunized animals (control group), which showed serious lesions. To improve protective efficacy, three recombinant protective antigens were evaluated in combination with rGST and rLigA as negative and positive control respectively. The results indicate that rLP1454, rLP1118, and rMCEII showed protection individually and synergistically against serovar Pomona infection, which can be used to develop a multicomponent vaccine for leptospirosis.

Leptospirosis is a world-wide zoonotic disease caused by gram-negative spirochetes belonging to the genus *Leptospira*. People who contract leptospirosis, either directly through contact with infected animals or indirectly from a contaminated environment, often develop kidney and liver failure (Bain et al. 1973. Renal failure and transient paraproteinemia due to Leptospira pomona. Case reports and literature review. Arch. Intern. Med. 131:740-745; Divers, et al. 1992. Renal dysfunction associated with infection of Leptospira interrogans in a horse. J. Am. Vet. Med. Assoc. 201:1391-1392; Garcia Garcia, et al. 1979. Icterohemorrhagic leptospirosis with acute renal failure (author's transl). Med. Clin. (Barc) 73:362-366; Kager, et al. 2001. Fever and chills due to leptospirosis after travel to Thailand. Ned. Tijdschr. Geneeskd. 145:184-186; Menzies, et al. 1989. Leptospira icterohaemorrhagiae infection presenting as acute renal failure. Scott Med. J. 34:410; Petros, et al. 2000. Serum procalcitonin and proinflammatory cytokines in a patient with acute severe leptospirosis. Scand. J. Infect Dis. 32: 104-105; San Segundo, et al. 1982. Leptospirosis caused by L. grippotyphosa accompanied by acute renal failure (author's transl). Med. Clin. (Bare) 78:28-31; Schubert, et al. Acute renal failure in leptospirosis (with a report on the differential diagnosis of Leptospira sejroe and Leptospira icterohaemorrhagiae). Munch. Med. Wochenschr. 113:80-86; Winearls, et al. 1984. Acute renal failure due to leptospirosis: clinical features and outcome in six cases. Q. J. Med. 53:487-495). Infection in horses, cattle, dogs and pigs can cause abortion, still birth, renal failure, and uveitis (Akkermans, J. P. 1966. Incidence of abortion and sterility in swine in the Netherlands due to infection with Leptospira hyos. Bull. Off. Int. Epizoot. 66:849-866; Andreani, E., F. Tolari, and R, Farina. 1983. Experimental infection in sheep with Leptospira interrogans serotype hardjo, Br. Vet. J. 139:165-170; Bernard, W. V. 1993. Leptospirosis. Vet. Clin. North Am. Equine Pract. 9:435-444; Bolin, C. A., J. A. Cassells, H. T. Hill, J. C. Frantz, and J. N. Nielsen. 1991. Reproductive failure associated with Leptospira interrogans serovar Bratislava infection of swine. J. Vet. Diagn. Invest. 3:152-154; Broll. S., A. S. Waldvogel, M. Rosskopf, L. Corboz, and A. Pospischil. 1993. The infectious causes of abortion and stillbirth in swine in Switzerland. Zentralbl. Veterinarmed. B 40:641-653; Donahue, J. M., B. J. Smith, J. K. Donahoe, C. L. Rigsby, R. R. Tramontin, K. B.Poonacha, and M. A. Wilson. 1992. Prevalence and serovars of leptospira involved in equine abortions in central Kentucky during the 1990 foaling season. J. Vet. Diagn. Invest. 4:279-284; Donahue, J. M., B. J. Smith, K. B. Poonacha, J. K. Donahoe, and C. L. Rigsby. 1995. Prevalence and serovars of leptospira involved in equine abortions in central Kentucky during the 1991-1993 foaling seasons. J. Vet. Diagn. Invest. 7:87-91; Donahue, J. M., B. J. Smith, K. J. Redmon, and J. K. Donahue. 1991. Diagnosis and prevalence of leptospira infection in aborted and stillborn horses. J. Vet. Diagn. Invest. 3:148-151; Donahue, J. M., and N. M. Williams. 2000. Emergent causes of placentitis and abortion. Vet. Clin. North Am. Equine Pract. 16:443-456, viii; Elder, J. K., P. M. Pepper, M. W. Hill, and W. H. Ward. 1985. The significance of leptospiral titres associated with bovine abortion. Aust. Vet. J. 62:258-262; Ellis, T. M., G. M, Robertson, L. Hustas, and M. Kirby. 1983. Detection of leptospires in tissue using an immunoperoxidase staining procedure. Aust. Vet. J. 60:364-367; Ellis, W. A., D. G. Bryson, and J. B. McFerran. 1976. Abortion associated with mixed Leptospira/equid herpesvirus 1 infection. Vet. Rec. 98:218-219; Rocha, T. 1990. Isolation of Leptospira interrogans serovar mozdok from aborted swine fetuses in Portugal. Vet. Rec. 126:602) and can even lead to multi-organ failure.

Currently available whole cell leptospiral vaccines generally stimulate only short-term immunity and do not provide cross protection against the 250 known serovars of pathogenic *Leptospira*. Furthermore *Leptospira* infection can trigger autoimmune disease in horses and people (Parma, A. E., S. I. Cerone, and S. A. Sansinanea. 1992. Biochemical analysis by SDS-PAGE and western blotting of the antigenic relationship between Leptospira and equine ocular tissues. Vet. Immunol. Immunopathol. 33:179-185; Parma, A. E., S. I. Cerone, S. A. Sansinanea, and M. Ghezzi. 1992. C3 fixed in vivo to cornea from horses inoculated with Leptospira interrogans. Vet. Immunol. Immunopathol. 34:181-187; Parma, A. E., A. S. Fernandez, C. G. Santisteban, R. A. Bowden, and S. I. Cerone. 1987. Tears and aqueous humor from horses inoculated with Leptospira contain antibodies which bind to cornea. Vet. Immunol. Immunopathol. 14:181-185; Parma, A. E., M. E. Sanz, P. M. Lucchesi, J. Mazzonelli, and M. A. Petruccelli. 1997. Detection of an antigenic protein of Leptospira interrogans which shares epitopes with the equine cornea and lens. Vet. J. 153:75-79; Rathinam, S. R., S. Rathnam, S. Selvaraj, D. Dean, R. A. Nozik, and P. Namperumalsamy. 1997. Uveitis associated with an epidemic outbreak of leptospirosis. Am. J. Ophthalmol.124:71-79. Thus, the safety and efficacy of leptospiral vaccines may be improved by administering immunoprotective subunits rather than administering whole cell antigens.

The conventional approach to vaccine development depends on the identification of protective antigens for use as subunit vaccines (Rappuoli, R., and G. Del Giudice. 1999. Identification of vaccine targets. CRC, Boca Raton). Study of leptospiral extracts indicates that outer membrane components contain protective antigens. Importantly, some of these protective OMPs are expressed at only low levels or may not be expressed at all by leptospires grown in artificial media (Palaniappan, R. U., Y. F. Chang, S. S. Jusuf, S. Artiushin, J. F. Timoney, S. P. McDonough, S. C. Barr, T, J. Divers, K. W. Simpson, P. L. McDonough, and H. O. Mohammed. 2002. Cloning and molecular characterization of an immunogenic LigA protein of Leptospira interrogans, Infect. Immun. 70:5924-5930). Various leptospiral OMPs such as OmpL1, LipL4I, LipL32, LipL36, Lig and LipL21 have been cloned and characterized (Cullen, P. A., S. J. Cordwell, D. M. Bulach, D. A. Haake, and B. Adler. 2002, Global analysis of outer membrane proteins from Leptospira interrogans serovar Lai. Infect. Immun. 70:2311-2318; Haake, D. A. 2000. Spirochaetal lipoproteins and pathogenesis. Microbiology 146:1491-1504; Haake, D. A., C. Martinich, T. A. Summers, E. S. Shang, J. D. Pruetz, A. M. McCoy, M. K. Mazel, and C. A. Bolin. 1998. Characterization of leptospiral outer membrane lipoprotein LipL36: downregulation associated with late-log-phase growth and mammalian infection. Infect. Immun. 66:1579-1587; Palaniappan, R. U., Y. F. Chang, F. Hassan, S. P. McDonough, M. Pough, S. C. Barr, K. W. Simpson, H. O. Mohammed, S. Shin, P. McDonough,R. L. Zuerner; J. Qu, and B. Roe. 2004. Expression of leptospiral immunoglobulin-like protein by Leptospira interrogans and evaluation of its diagnostic potential in a kinetic ELISA. J. Med. Microbiol. 53:975-984; Palaniappan, R. U., Y. F. Chang, S. S. Jusuf, S. Artiushin, J. F. Timoney, S. P. McDonough, S. C. Barr, T. J. Divers, K. W. Simpson, P. L. McDonough, and H. O. Mohammed. 2002. Cloning and molecular characterization of an immunogenic LigA protein of Leptospira interrogans. Infect. Immun. 70:5924-5930; Palaniappan, R. U., S. P. McDonough, T. J. Divers, C. S. Chen, M. J. Pan, M. Matsumoto, and Y. F. Chang. 2006. Immunoprotection of recombinant leptospiral immunoglobulin-like protein A against Leptospira interrogans serovar Pomona infection. Infect. Immun. 74:1745-1750; Shang, E. S., M. M. Exner, T. A. Summers, C. Martinich, C. I. Champion, R. E. Hancock, and D. A. Haake. 1995. The rare outer membrane protein, OmpL1, of pathogenic Leptospira species is a heat-modifiable porin. Infect. Immun. 63:3174-3181). LipL32 protects animals when used in a DNA vaccine formula, but not as a recombinant protein (Branger, C., B. Chatrenet, A. Gauvrit, F. Avial, A. Aubert, J. M. Bach, and G. Andre-Fontaine. 2005. Protection against Leptospira interrogans sensulato challenge by DNA immunization with the gene encoding hemolysin-associated protein 1. infect. Immun. 73:4062-4069). In contrast, the recombinant protein LigA is able to induce immunoprotection against leptospirosis (Koizumi, N., and H. Watanabe. 2003. Identification of a novel antigen of pathogenic Leptospira spp. that reacted with convalescent mice sera. J. Med. Microbiol. 52:585-589;Palaniappan, R. U., S. P. McDonough, T. J. Divers, C. S. Chen, M. J. Pan. M. Matsumoto, and Y. F. Chang. 2006. Immunoprotection of recombinant leptospiral immunoglobulin-like protein A against Leptospira interrogans serovar Pomona infection. Infect. Immun. 74:1745-1750).

Although results of protection experiments in hamsters and mice with Lig proteins are promising, a combination of key antigens and/or epitopes is likely to be more efficacious against multiple *Leptospira* serovars, especially in outbred animals. Such a multicomponent vaccine circumvents the problems of host genetic restriction and antigenic variability associated with single antigen-based vaccines.

Recently, vaccine development has taken advantage of the genome sequence of pathogenic bacteria (Kelly, D. F., and R. Rappuoli. 2005. Reverse vaccinology and vaccines for serogroup B Neisseria meningitidis. Adv. Exp. Med. Biol. 568:217-223; Rappuoli, R. 2000. Reverse vaccinology. Curr. Opin. Microbiol. 3:445-450; Rappuoli, R. 2001. Reverse vaccinology, a genome-based approach to vaccine development. Vaccine 19:2688-2691; Rappuoli. R., and A. Covacci. 2003. Reverse vaccinology and genomics. Science 302:602).

This approach not only identifies all the antigens described by conventional methods, but also enables the discovery of novel antigens. Since OMPs are the primary bacterial components that interact with host cells, targeting the OMPs for development of recombinant vaccine is advantageous.

In this study, we focused on 13 OMPs that had no homology with other bacterial proteins listed in NCBI database as well as *Leptospira* MceI and II. All these OMPs were cloned, expressed and purified as GST fusion proteins. Screening for protective efficacy of recombinant OMPs was performed in a hamster model. Of these, rMceII, rLP 1118 and rLP1454 serve as immunoprotective antigens against *L. interrogans* serovar Pomona infection. All these recombinant antigens were evaluated separately as well as in combination along with rLigA (positive control) and rGST (negative control) suggesting that rMceII, rLP1118 and rLP1454 could serve as protective antigens individually and synergistically paving the way for the development of a multicomponent vaccine against leptospirosis.

**6.2.2 Materials and Methods**

**Bacterial strains, media and plasmids .** *L*. *interrogans* serovar Pomona was used as previously described (Palaniappan, et al. 2002. Cloning and molecular characterization of an immunogenic LigA protein of Leptospira interrogans. Infect. Immun. 70:5924-5930). Leptospires were maintained on EMJH medium at 30°C (Palaniappan, et al. 2002. Cloning and molecular characterization of an immunogenic LigA protein of Leptospira interrogans. Infect.Immun. 70:5924-5930). To isolate low passage cultures of leptospires, hamsters were experimentally infected with a sublethal dose of *L. interrogans* serovar Pomona (NVSL 1427-35-093002). Infected hamster tissues were harvested aseptically, homogenized with sterile PBS, and the lysates were inoculated into EMJH medium. Growth was monitored using dark field microscopy.

**LD₅₀ value of *L interrogans* in hamster.** Twenty-four 8-9 week-old Golden Syrian hamsters (Harlan Sprague Dawley) were divided into 4 equal groups. Each group was infected intraperitoneally with 10⁹ to 10⁷ low passage (3-4 passages) *L. interrogans* serovar Pomona (NVSL 1427-35-093002). One group of hamsters received only.PBS (control), The animals were monitored daily. Tissues were collected aseptically from animals that died due to infection and subjected to culture and histopathological analysis.

**OMP cloning and expression.** Primers for the putative outer membrane were designed without the signal sequences and are listed in Table I (Section 6.1). Genomic DNA of *L. interrogans* serovar Pomona was prepared using a DNAeasy kit (Qiagen, Valencia, CA) and subjected to PCR as described by the manufacturer using Accuprime Taq polymerase (Invitrogen, CA). PCR products were run in 1% agarose gel and visualized by ethidium bromide staining. PCR products were eluted from the gel using gel elution kit (Qiagen, Valencia, CA) and cloned into TOPO TA Vector as described by the manufacturer (Invitrogen, CA). The insert was then sub-cloned into the expression vector pGEX-KG (Stratagene). LP1454,1118 and 0607(MCEII) were also inserted into pRSETA (inVitroGen, CA) to express recombinant proteins for serologic test. DNA sequencing was analyzed with an ABI model 377 automated nucleic acid sequencer at the Bioresource Center, Cornell University. Homology searches were performed with the NCBI database and BLAST (AlischuL S. F., W. Gish, W. Miller, E. W. Myers, and D. J. Lipman. 1990. Basic local alignment search tool. J. Mol. Biol. 215:403-410).

**Expression and purification of recombinant proteins.** Exponentially growing cultures (OD₆₀₀=0.6) of *E*. *coli* (BL21 DE3) or CQ21 harboring recombinant plasmids were induced with or without 1mM isopropyl-β-D-thiogalactopyranoside (IPTG; Sigma, St, Louis, Mo.). For each recombinant protein, a pilot experiment was performed with 3mL culture volume and the molecular weight of the expressed recombinant proteins was checked by SDS-PAGE analysis. Mass cultivation was performed in 1,000mL of LB broth with ampicillin (50 µg/mL). Bacteria were harvested by centrifugation at 5000 RPM for 30 min. The cell pellets was washed and suspended in PBS and subjected to a French pressure cell (American Instruments). The lysates were centrifuged at 8,000 xg for 30 min and the inclusion bodies were washed and purified as mentioned previously (Palaniappan, et al. 2004. Expression of leptospiral immunoglobulin-like protein by Leptospira interrogans and evaluation of its diagnostic potential in a kinetic ELISA. J. Med. Microbiol. 53:975-984; Palaniappan, et at 2002. Cloning and molecular characterization of an immunogenic LigA protein of Leptospira interrogans. Infect. Immun. 70:5924-5930) with model prep cell (BioRad, CA). The purified protein was dialyzed at 4°C with PBS. The purified protein was concentrated and lyophilized. (Palaniappan, et at. 2004. Expression of leptospiral immunoglobulin-like protein by Leptospira interrogans and evaluation of its diagnostic potential in a kinetic ELISA. J. Med. Microbiol. 53:975-984). The lyophilized protein was suspended with sterile PBS and quantified using Bio-Rad protein assay.

**SDS-PAGE**. SDS-PAGE was performed as previously described (Chang, et al. 1993. Molecular characterization of a leukotoxin gene from a Pasteurella haemolytica-like organism, encoding a new member of the RTX toxin family. Infect.Immun. 61:2089-2095). The equal amount of recombinant proteins (1.5 µg) was subjected to SDS-PAGE and the gel was stained with Coomassie brilliant blue R-250 followed by de- staining (Chang, et al. 1993. Molecular characterization of a leukotoxin gene from a Pasteurella haemolytica-like organism, encoding a new member of the RTX toxin family. Infect. Immun. 61:2089-2095).

**Immunization and challenge experiments.** Preliminary screening of recombinant putative outer membrane proteins was performed in a group of 4 week-old Golden Syrian hamsters (Harlan Sprague Dawley; 3 hamsters per group). Hamsters were immunized twice by subcutaneous injection with recombinant proteins (50 pg) at an interval of three weeks (Day 0 and Day 21). Hamsters receiving rGST were considered a control group. Prior to immunization, the recombinant proteins or rGST were mixed with an equal volume of aluminum hydroxide (Alhydrogel, Accurate Chemical & Scientific Corp. Westbury, NY). Blood for serum separation was collected on days 0 (pre-vaccination), 21 and 42 directly from the saphenous vein.

Immunized hamsters were challenged intraperitoneally with the estimated LD₅₀ value of 10⁸ leptospires on D42. The animals were monitored twice daily, sacrificed on day 71 and blood was collected by cardiac puncture. The tissues from infected animals where collected aseptically for histopathological analysis and culture.

Recombinant protective antigens (rLP1454, rLP1118 and rMCEII) were analyzed again as discussed above in a group of hamster (8 animals per group). Animals immunized with rGST and rLigA were considered as negative and positive controls, respectively. The protective efficacy of the three recombinant antigens in combination (50 µg of each antigen) was assessed in a group of hamsters (8 hamsters per group) with rGST and rLigA considered as negative and positive controls, respectively.

**Culture.** Hamster tissues such as liver, kidney, spleen, lungs, urinary bladder and blood were collected aseptically from each animal, homogenized in 0.5 ml EMJH medium, transferred into 20 ml EMJH medium and maintained at 30°C for 4 weeks (Palaniappan, et al. 2002. Cloning and molecular characterization of an immunogenic LigA protein of Leptospira interrogans. Infect. Immun. 70:5924-5930; et al. 2006. Immunoprotection of recombinant leptospiral immunoglobulin-like protein A against Leptospira interrogans serovar Pomona infection. Infect. Immun. 74:1745-1750). Growth was monitored using dark field microscopy.

**Histopathology.** Hamster tissues were fixed by immersion in 10% neutral buffered formalin, embedded in paraffin, sectioned at 5 µm, stained with hematoxylin and eosin (H&E) using standard histological techniques, and examined by light microscopy. The severity of *Leptospire* induced tubulointerstitial nephritis was graded by a veterinary pathologist (SPMcD) who was blind to the identity of the treatment groups and who graded on the following scale: 0 = normal, 1 = mild, 2 = moderate, and 3 = severe.

Mild renal lesions had interstitial infiltrates of small numbers of lymphocytes occasionally mixed with a few heterophils and macrophages. Occasional tubules were mildly dilated and distended with hyaline material that contained rare sloughed epithelial cells. The lining epithelium was also moderately attenuated. Rarely, desquamated epithelial cells and heterophils formed mixed casts with hyaline material within the lumina of proximal convoluted. Moderate renal lesions had prominent perivascular accumulations of lymphocytes mixed with lesser number of plasma cells, macrophages and occasional heterophils. Tubular changes were more common than in mildly affected kidneys and hyaline casts were noted occasionally. Severely affected kidneys were irregularly shrunken with widespread interstitial fibrosis accompanied by extensive degeneration and loss of proximal convoluted tubules. Hyaline casts were numerous but glomerular changes were generally mild. Bowman's capsules of glomeruli in regions of severe tubular atrophy and fibrosis were dilated and the uriniferous spaces were filled with a faintly eosinophilic lacy material. Some glomeruli had shrunken glomerular tufts while others had patchy increases in the amount of mesangial matrix. Mild hypertrophy of the parietal epithelium lining Bowman's capsules was also present is a subset of affected glomeruli.

**Kinetic Enzyme-Linked Immunosorbent Assay (KELA) with recombinant antigens**

Sera from hamsters given recombinant antigens and rGST were evaluated for the presence of specific IgG using a KELA assay with recombinant antigens (Palaniappan, et al. 2004. Expression of leptospiral immunoglobulin-like protein by Leptospira interrogans and evaluation of its diagnostic potential in a kinetic ELISA. J. Med. Microbiol. 53:975-984). A checkerboard titration was followed to optimize the concentrations of reagents and the KELA assay was performed as previously described (Koizuimi, N., and H. Watanabe. 2003. Identification of a novel antigen of pathogenic Leptospira. spp. that reacted with convalescent mice sera. J. Med. Microbiol. 52.-585-589; Naiman, et al. 2002. Evaluation of type 1 immune response in naive and vaccinated animals following challenge with Leptospira borgpetersenii serovar Hardjo: involvement of WC1(+) gammadelta and CD4 T cells. Infect. Immun. 70:6147-6157; et al. 2005. Evaluation of lig-based conventional and real time PCR for the detection of pathogenic leptospires. Mol. Cell. Probes 19:111-117).

Briefly, the optimum concentration of recombinant Histag-fusion proteins (rMceII, rLP1454, and rLP1118) were diluted in 0.1M bicarbonate buffer, coated onto a 96 well microtiter plate (Nunc, Denmark), rocked for 1 hour, and then incubated overnight at 4°C. The plates were washed three times with 0.1M PBS containing (0.05% Tween 20 (PBST). Next, 100 µl of hamster serum (primary antibody) diluted 1:100 in PBST was added to each well and incubated for 1 hour at 37°C in a humid chamber. The plates were washed three times as described above with PBST and incubated with 100 µl of a 1: 1000 dilution of goat anti-hamster IgG conjugated to horseradish peroxidase (Cappel, Durham. NC) for 30 min at room temperature. The plates were washed again three times with PBST and 100 µl of TMB (Kirkegaard, MD) was added to each well. Each plate was read three times at 650 nm OD with an interval of 1-minute (Biotek EL-312, Winoski, VT).

The results were calculated by the KELA computer program (publicly available for use at the Animal Health Diagnostic Center, College of Veterinary Medicine Cornell University, Ithaca NY) and expressed as the slope of the reaction between the enzyme and the substrate to the amount of antibody bound (Chang et al. 1999. Vaccination against lyme disease with recombinant Borrelia burgdorferi outer-surface protein A (rOspA) in horses. Vaccine 18:540-548; Chang, et al. 1995. Recombinant OspA protects dogs against infection and disease caused by Borrelia burgdorferi. Infect Immun 63:3543-3549).

6.2.3 **Results**

**Expression and purification of recombinant putative outer membrane proteins as GST fusion proteins:** The genomic sequences of *Leptospira* predicted several putative OMPs. Of these, 13 putative outer membrane proteins and also MCEI and II were targeted for vaccine development. All these OMPs were cloned and expressed as fusion proteins. Purified recombinant GST fusion proteins appeared as a single band by SDS-PACE analysis (FIG. 4).

**LD50 value for *L. interrogans* serovar Pomona in a hamster model:** Hamsters (8 animals per group) that received 10⁹ suffered 100% mortality. However, 5 of 8 hamsters survived after receiving a dose of 10⁸ leptospires. All the hamsters that received a dose of 10⁷ leptospires survived, indicating the LD₅₀ for *L interrogans* serovar Pomona infection was approximately 10⁸.

**Antibody responses to recombinant antigens:** To examine whether hamsters given recombinant antigens developed IgG antibody response against the antigens, hamsters immunized with 50 µg of recombinant proteins (rMCEII, rLP1454 and rLP1118) individually and in combination were evaluated for serum IgG antibody level. Sera from the animals collected on days 0, 21, 42 and 71 were analyzed by KELA using rMceII, r1454, rLP1118, and rLigA. Hamsters showed lgG antibodies to rMceII, r1454, rLP1118 and rLigA, indicating the immune response against recombinant protein (FIG. 5). FIG. 5 shows that serum from immunized animals was collected on D0 (pre-immune) D21 (serum samples after first immunization), D42 (serum samples after second immunization) and D72 (29 days after challenge). FIG. 5 represents hamsters immunized individually with recombinant proteins (rLP1454, rLP0607, and rLP111b) or immunized with combination of recombinant proteins (rLP1454C, rLP0607C, and rLP111bC).

**Recombinant antigens rMceII, rLP1118 and r1454 confer protective immunity in a hamster model:** Preliminary screening was evaluated in hamsters (3 animals per group) given either recombinant OMP or rGST as a negative control. All the animals given rGST succumbed (Table 2).

**Table 2. Preliminary screening of protective efficacy of recombinant proteins of OMPs in a hamster model**

| No: | Group | No. of surviving animals/totals no: of animals |
|---|---|---|
| 1 | rGST (control) | 0/3 (0) |
| 2 | rLA2471 | 0/3 (0) |
| 3 | rLA1434 | 2/3 (66) |
| 4 | rLA1495 | 0/3 (0) |
| 5 | rLA1939 | 0/3 (0) |
| 6 | rLA1965 | 0/3 (0) |
| 7 | rLA1332 | 0/3 (0) |
| 8 | rMCE1 | 1/2 (50) |
| 9 | rLA1404 | 1/2 (50) |
| 10 | rLA1228 | 1/3(33) |
| 11 | RLA1118 | 2/3 (66) |
| 12 | rLA1947 | 1/3 (33) |
| 13 | rMCEII | 2/3 (66) |

However, 2/3 of the hamsters that received rMceII, rLP1118 and r1454 survived after challenge, indicating that these recombinant proteins may serve as protective antigens.

To confirm the protective efficacy of rMceII, rLP1118 and r1454, these recombinant antigens were analyzed again in hamsters (8 hamsters per group) with rLigAand rGST as positive and negative controls, respectively. Compared to the rGST immunized animals, where only 3/7 hamsters survived, 5/7, 6/8, 7/7 and 8/8 hamsters given rLP1454, rLP1118, rMCEII and rLigA, respectively, survived (Table 3).

**Table 3. Confirmation of protective effect of rLPT1454, rLP1118 and rMCEII**

| No: | Group | No. of surviving animals/total no: of animals |
|---|---|---|
| 1. | rLA1454 | 5/7 (71%) |
| 2 | rLA1118 | 6/8 (75%) |
| 3 | rMCEII | 7/7 (100%) |
| 4 | rGST (control)] | 3/7 (43%) |

Thus, rMceII, rLP1454 and LP1118 provided protection from the lethal effects of *L. interrogans* serovar Pomona infection.

To evaluate whether these recombinant proteins can enhance protection synergistically, hamsters were given a combination of antigens (rLP1454, rLP1118 and rMCEII) and evaluated against an LD50 inoculum of *L. interrogans* serovar Pomona. Almost all hamsters that were given the recombinant OMP combination survived (7/8) whereas only 4/8 hamsters survived in the control group (rGST immunized animals). These results confirm the synergistic immunoprotective effect of these recombinant antigens (Table 4).

**Table 4. Synergistic protective effect of rLP1454, rLP1118 and rMCEII**

| No: | Group | No. of surviving animals/total no: of animals |
|---|---|---|
| 1 | rLP1454-LP1118-MCEII (combination) | 7/8 (87.5) |
| 2 | rLigA | 8/8 (100) |
| 3 | rGST (control) | 4/8 (50) |

**Histopathological analysis.** All but two hamsters that received rGST developed severe tubulointerstitial nephritis (Table 6) while those given rLig were completely protected from severe disease.

**Table 5. Effect of recombinant outer membrane protein vaccination on the severity of tubulointerstitial nephritis in hamsters challenged with L. interrogans serovar Pomona**

| Recombinant Antigen | Proportion with Severe (grade 3) Tubulointerstitial Neptiritis* |
|---|---|
| rGST | 85% |
| rLigA | 0% |
| r1454 | 77% p=0.6188 |
| r1118 | 36% p=0.0098 |
| rMCEII | 71% p=0.4102 |
| (r1454+ r1118+rMCEII) | 25% p=0.0261 |

*A Chi-Square test for heterogeneity or independence was used to determine the difference in histopathologic lesion of kidney to the control group (GST). Statistical significance was claimed if p≤0,05.

The recombinant OMPs LP1454 and MCE II had only modest protective effects but rLP1118 markedly decreased the incidence of severe tubulointerstitial nephritis. Interestingly, when rLP1454, rLP1118 and rMCE II were given in combination, the protective effects appear to be synergistic, with only 25% of hamsters developing severe renal lesions. A Chi-Square test for heterogeneity or independence was used to determine the difference in histopathologic lesion of kidney to the control group (GST). Statistical significance was claimed if p≤0.05.

***Leptospira* culture from tissues.** Kidney, liver, urinary bladder, lungs and spleen from all hamsters including immunized and control hamsters were evaluated for the presence of leptospires by culture. Except for the control groups that cultured positive in kidney, urinary bladder and/or spleen in each animal, all other vaccinated groups were cultured positive only in two or three animals per group in kidney and/or urinary bladder only.

**6.2.4 Discussion**

In this study, we focused on putative OMPs of the *Leptospira* genome to develop a protein-based vaccine for the prevention and/or treatment of leptospirosis.

We have demonstrated the feasibility of developing recombinant vaccines based on OMPs. Considering the increase in serovars of *Leptospira* and also host restriction of some of the serovars (for example, serovar Hardjo in cattle), it is advantageous to screen more protective antigens to develop a multi-component vaccine. Conventional approaches to vaccine development are time consuming and indentify only abundant antigens that may or may not provide immunity.

Reverse vaccinology (i.e. genomic-based approaches to vaccine development) can overcome these problems and enable the identification of novel vaccine candidates. All the OMPs targeted in the present study for vaccine development showed 98-100% homology in amino acid sequences among *L*. *interrogans* serovar Icterohaemmorhagiae, *L*. *interrogans* serovar Copenhageni and *L. interrogans serovar* Pomona. Interestingly, 13 putative OMPs did not show homology to other organisms in the NCBI database, except MCEI and II showed 25-30% similarities to MCE of *M. tuberculosis* (data not shown). The antibody response to MceII immunized animals showed lesser level compare to other recombinant proteins. Evaluation of imimunogenicity of recombinant proteins with bovine sera from experimental and natural infection indicates that MCEII is highly immunogenic compare to other recombinant proteins of *Leptospira* (our unpublished data). Immunization of hamsters with denatured rMceII may cause lower level of antibody responses (FIG. 5). Although MCE did not provide immunoprotection in intracellular mycobacteria, we found the protective efficacy of rMCE II in a hamster model. MCE is involved in invasion of the *Mycobacterium* (Chitale et al. 2001. Recombinant Mycobacterium tuberculosis protein associated with mammalian cell entry. Cell. Microbiol. 3.247-254).

We have evaluated the protective potential of recombinant proteins using a hamster intraperitoneal challenge model. Hamster is a preferred model of leptospirosis disease and also allows us to study the protective efficacy against heterologous challenge. The study described here, in which we screened to assess the immunoprotection potential against *Leptospira* in a hamster model, suggests that rLP1454, rLP1118 and MCEII are protective antigens based on lethality. We have previously shown protection conferred by recombinant LigA protein (Palaniappan et al. 2006. Immunoprotection of recombinant leptospiral immunoglobulin-like protein A against Leptospira interrogans serovar Pomona infection. Infect. Immun. 74:1745-1750). To improve the protective efficacy of the recombinant antigens identified in this study, synergistic application of rLP1454, rLP118 and rMCEII was attempted with rLigA and rCST as positive and negative control, respectively. The results imply a synergistic effect with better protection was obtained with all three antigens than alone.

In general terms, the predominant immunological elector response for an extracellular bacterium such as *Leptospira* is antibody-mediated whereby the bacteria are either killed directly via complement activation or opsonized for phagocytosis. Other mechanisms besides complement-mediated bactericidal activity may be responsible for the protection demonstrated in the present study and the contribution of other aspects of the immune system to the defense against leptospiral infection can also be assessed. A monovalent Leptospiral vaccine has been reported to induce type 1 protective immune response (Naiman, et al 2002. Evaluation of type 1 immune response in naive and vaccinated animals following challenge with Leptospira borgpetersenii serovar Hardjo: involvement of WC1(+) gammadelta and CD4 T cells. Infect. Immun. 70:6147-6157).

Recently, passive immunization of polyclonal antibodies of rLigA failed to provide protection against infection (Palaniappan, et al. 2005. Evaluation of lig-based conventional and real time PCR for the detection of pathogenic leptospires. Mol. Cell. Probes 19:111-117). It therefore appears that both humoral and cell mediated immunity can play a role in immunoprotection. The generation of the protective antigens identified in this study has the potential to enhance the efficacy of a recombinant based vaccine and can also lead to the development of multi-component vaccine to generate cross-protection against a wide range of increasing serovars of *Leptospira.*

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

An references cited herein are incorporated herein by reference in their entirety and for all purposes to the same extent as if each individual publication, patent or patent application was specifically and individually indicated to be incorporated by reference in its entirety for all purposes.

The citation of any publication is for its disclosure prior to the filing date and should not be construed as an admission that the present invention is not entitled to antedate such publication by virtue of prior invention.

## Claims

1. A vector expressing a recombinant DNA, wherein the recombinant DNA is selected from the group consisting of:
(i) a recombinant DNA that encodes a protein having the amino acid sequence as shown in SEQ ID NO: 8 (MCEII),
(ii) a recombinant DNA that encodes an immunogenic epitope or immunologically active fragment of the protein of (i) above, and
(iii) a recombinant DNA that encodes a protein fragment of at least 10%, 20%, 30%, 40%, 50%, 60%, 70%, 80%, 90% or 95% of the length of the amino acid sequence of the protein of (i) or the immunogenic epitope or the immunologically active fragment of (ii) above,
and wherein the recombinant DNA is inserted into the vector such that a recombinant protein is expressed when the vector is provided in an appropriate host.

2. A recombinant DNA vaccine comprising:
a) a recombinant DNA wherein the recombinant DNA is selected from the group consisting of:
(i) a recombinant DNA that encodes a protein having the amino acid sequence as shown in SEQ ID NO: 8 (MCEII),
(ii) a recombinant DNA that encodes an immunogenic epitope or immunologically active fragment of the protein of (i) above, and
(iii) a recombinant DNA that encodes a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above;
and
b) a vector capable of expressing the recombinant DNA when the recombinant DNA is inserted into the vector, wherein the recombinant DNA is inserted into the vector such that a recombinant protein is expressed when the vector is provided in an appropriate host.

3. A method for producing a vaccine against a *Leptospira*-related disorder comprising:
a) providing a recombinant DNA, wherein the recombinant DNA is selected from the group consisting of:
(i) a recombinant DNA that encodes a protein having the amino acid sequence as shown in SEQ ID NO: 8 (MCEII),
(ii) a recombinant DNA that encodes an immunogenic epitope or immunologically active fragment of the protein of (i) above, and
(iii) a recombinant DNA that encodes a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above;
b) providing a vector capable of expressing the recombinant DNA when the recombinant DNA is inserted into the vector; and
c) inserting the recombinant DNA into the vector, wherein the recombinant DNA is inserted into the vector such that a recombinant protein is expressed when the vector is provided in an appropriate host.

4. A method for producing a vaccine against a *Leptospira-*related disorder comprising:
a) providing a recombinant DNA, wherein the recombinant DNA is selected from the group consisting of:
(i) a recombinant DNA that encodes a protein having the amino acid sequence as shown in SEQ ID NO: 8 (MCEII),
(ii) a recombinant DNA that encodes an immunogenic epitope or immunologically active fragment of the protein of (i) above, and
(iii) a recombinant DNA that encodes a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above;
b) constructing an expression vector comprising a plasmid, wherein the plasmid comprises the recombinant DNA; and
c) inoculating a host with the expression vector, wherein the recombinant DNA is expressed in the host to produce a recombinant protein, and wherein the expressed protein raises an immune response in the host.

5. A vaccine comprising a recombinant protein, wherein the recombinant protein is selected from the group consisting of:
(i) a protein having the amino acid sequence as shown in SEQ ID NO: 8 (MCEII),
(ii) an immunogenic epitope or immunologically active fragment of the protein of (i) above, and
(iii) a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above.

6. A method for producing a vaccine against a *Leptospira*-related disorder comprising:
a) providing a recombinant DNA, wherein the recombinant DNA is selected from the group consisting of:
(i) a recombinant DNA that encodes a protein having the amino acid sequence as shown in SEQ ID NO: 8 (MCEII),
(ii) a recombinant DNA that encodes an immunogenic epitope or immunologically active fragment of the protein of (i) above, and
(iii) a recombinant DNA that encodes a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above;
b) providing a vector capable of expressing the recombinant DNA when the recombinant DNA is inserted into the vector;
c) inserting the recombinant DNA into the vector;
d) providing a bacterial strain;
e) transforming the vector into the bacterial strain such that a recombinant protein is expressed when the vector is transformed into the bacterial strain; and
f) harvesting the recombinant protein from the bacterial strain.

7. A T-cell epitope vaccine comprising a recombinant protein, wherein the recombinant protein comprises a T-cell epitope, and wherein the T-cell epitope comprises an amino acid peptide fragment of a protein selected from the group consisting of:
(i) a protein having the amino acid sequence as shown in SEQ ID NO: 8 (MCEII),
(ii) an immunogenic epitope or immunologically active fragment of the protein of (i) above, and
(iii) a protein fragment of at least 10%, 20%, 30%, 40%, 50%, 60%, 70%, 80%, 90% or 95% of the length of the amino acid sequence of the protein of (i) or the immunogenic epitope or the immunologically active fragment of (ii) above.

8. The vaccine of claim 7, **characterized in that** the amino acid peptide fragment comprises nine to twenty amino acids.

9. A method for producing a T-cell epitope vaccine comprising:
a) providing a recombinant protein that comprises a T-cell epitope, wherein the T-cell epitope comprises an amino acid peptide fragment of a protein selected from the group consisting of:
(i) a protein having the amino acid sequence as shown in SEQ ID NO: 8 (MCEII),
(ii) an immunogenic epitope or immunologically active fragment of the protein of (i) above, and
a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above;
b) identifying the T-cell epitope from the protein;
c) inserting DNA encoding the T-cell epitope into a construct capable of expressing the T-cell epitope as a protein; and
d) harvesting the protein.

10. The method of claim 9, **characterized in that** the amino acid peptide fragment comprises nine to twenty amino acids.

11. A vaccine according to claim 2, 5 or 7 for use in preventing a *Leptospira*-related disorder in an animal or human subject in need thereof.

12. A pharmaceutical composition comprising:
a) a therapeutically effective amount of the vaccine of claim 2, 5 or 7; and
b) a pharmaceutically acceptable carrier.

13. An isolated and purified antibody directed against a *Leptospira* outer membrane protein, wherein the *Leptospira* outer membrane protein is selected from the group consisting of:
(i) a protein having the amino acid sequence as shown in SEQ ID NO: 8 (MCEII),
(ii) an immunogenic epitope or immunologically active fragment of the protein of (i) above, and
(iii) a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above.

14. The antibody of claim 13, **characterized in that** the antibody is directed against at least one T-cell epitope of the *Leptospira* outer membrane protein.

15. A method for producing an antibody directed against a *Leptospira* outer membrane protein the method comprising:
a) providing a host animal; and
b) immunizing the host animal by injection with the *Leptospira* outer membrane protein, wherein the *Leptospira* outer membrane protein is selected from the group consisting of:
(i) a protein having the amino acid sequence as shown in SEQ ID NO: 8 (MCEII),
(ii) an immunogenic epitope or immunologically active fragment of the protein of (i) above, and
(iii) a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above,
and wherein the antibody specifically recognizes at least one T-cell epitope of the *Leptospira* outer membrane protein.

16. An antibody directed against a Leptospira outer membrane protein for use in a method of diagnosing a *Leptospira*-related disorder in a subject, wherein the *Leptospira* outer membrane protein is selected from the group consisting of:
(i) a protein having the amino acid sequence as shown in SEQ ID NO: 8 (MCEII),
(ii) an immunogenic epitope or immunologically active fragment of the protein of (i) above, and
(iii) a protein fragment of at least 10%, 20%, 30%, 40%, 50%, 60%, 70%, 80%, 90% or 95% of the length of the amino acid sequence of the protein of (i) or the immunogenic epitope or immunologically active fragment of (ii) above.


SEQUENCE LISTING

<110>  Chang, Yung-Fu

<120>  Immunogenic proteins from genome-derived outer membrane of
       Leptospira and compositions and methods based thereon

<130>  1258_3959PCT

<150>  60/976,088
<151>  2007-09-28

<150>  60/974,818
<151>  2007-09-24

<160>  67

<170>  PatentIn version 3.5

<210>  1
<211>  1077
<212>  DNA
<213>  Leptospira interrogans serovar Pomona

<220>
<221>  CDS
<222>  (1)..(1077)

<300>
<308>  GenBank / AAN48653
<309>  2006-02-01
<313>  (1)..(1077)

<400>  1
gtg ggg aaa aac aat tcg ttc aga ccg aaa aat tct ttc tta aag ttc      48
Met Gly Lys Asn Asn Ser Phe Arg Pro Lys Asn Ser Phe Leu Lys Phe
1               5                   10                  15

caa gtt gta ttc ggt ttt ttt ctt ttg agt ttt att tct tta aat tca      96
Gln Val Val Phe Gly Phe Phe Leu Leu Ser Phe Ile Ser Leu Asn Ser
            20                  25                  30

gaa gaa aga tcc tca att tta gat cct acg ttt ttt tta att cat aag      144
Glu Glu Arg Ser Ser Ile Leu Asp Pro Thr Phe Phe Leu Ile His Lys
        35                  40                  45

aat ctg aat cct tct tta gga ggt tta ttc gaa acc aga aaa aaa ttt      192
Asn Leu Asn Pro Ser Leu Gly Gly Leu Phe Glu Thr Arg Lys Lys Phe
    50                  55                  60

act tct tat gga gcc tgg ttt gaa ctt cca gtt tgg aaa cga gaa cgt      240
Thr Ser Tyr Gly Ala Trp Phe Glu Leu Pro Val Trp Lys Arg Glu Arg
65                  70                  75                  80

aaa cat ttt tgg aaa acc cat ttt ctt tat gag aat cac aag act ttt      288
Lys His Phe Trp Lys Thr His Phe Leu Tyr Glu Asn His Lys Thr Phe
                85                  90                  95

tca gtc gtt cat caa act tac ata gga ctt gaa tat tct ttt att tca      336
Ser Val Val His Gln Thr Tyr Ile Gly Leu Glu Tyr Ser Phe Ile Ser
            100                 105                 110

gaa aag aag gat ctg cac cct tcc gtt ttt gta ggt tgg gaa aga gga      384
Glu Lys Lys Asp Leu His Pro Ser Val Phe Val Gly Trp Glu Arg Gly

115                 120                 125

gaa aag gat tta gga att ttc gga atc cac ctt aca att ccg gac aaa        432
Glu Lys Asp Leu Gly Ile Phe Gly Ile His Leu Thr Ile Pro Asp Lys
    130                 135                 140

caa acg atc caa gtt ttt gga aaa act gga aaa gat ttt aaa agt ggt        480
Gln Thr Ile Gln Val Phe Gly Lys Thr Gly Lys Asp Phe Lys Ser Gly
145                 150                 155                 160

tct atc ttt ctt cat tct aac ttt gat act gga ctt caa tta ttt tta        528
Ser Ile Phe Leu His Ser Asn Phe Asp Thr Gly Leu Gln Leu Phe Leu
                165                 170                 175

gga ttt tca agg aca tgg gac aaa tcc tat acc gaa gat caa ttc act        576
Gly Phe Ser Arg Thr Trp Asp Lys Ser Tyr Thr Glu Asp Gln Phe Thr
            180                 185                 190

ctt gga atc agg gtt tct tgg gaa aaa att tac tct tct ttt ttc tgg        624
Leu Gly Ile Arg Val Ser Trp Glu Lys Ile Tyr Ser Ser Phe Phe Trp
        195                 200                 205

aat caa acg cag gaa gaa gaa tca ttt cta acc ggg aaa ttt gga atc        672
Asn Gln Thr Gln Glu Glu Glu Ser Phe Leu Thr Gly Lys Phe Gly Ile
    210                 215                 220

agt aat ttc caa caa gat ctc aaa tct aaa aca ctt ttt gaa tcc aca        720
Ser Asn Phe Gln Gln Asp Leu Lys Ser Lys Thr Leu Phe Glu Ser Thr
225                 230                 235                 240

gac gaa ttt cag tcg aat tca ctt ttt aaa tct aaa aat tta agt tct        768
Asp Glu Phe Gln Ser Asn Ser Leu Phe Lys Ser Lys Asn Leu Ser Ser
                245                 250                 255

aaa aac aca aaa tca aaa tcg ttc gaa act tct ctt tta gaa aaa gat        816
Lys Asn Thr Lys Ser Lys Ser Phe Glu Thr Ser Leu Leu Glu Lys Asp
            260                 265                 270

aaa aca aat tat tcc tac ata agt tca ttt att cgt agt tat acg aat        864
Lys Thr Asn Tyr Ser Tyr Ile Ser Ser Phe Ile Arg Ser Tyr Thr Asn
        275                 280                 285

att tcg att tcc gtt cag gaa tta ttg tcc gcc ggt ttt act ctt tcc        912
Ile Ser Ile Ser Val Gln Glu Leu Leu Ser Ala Gly Phe Thr Leu Ser
    290                 295                 300

tcc gct ttg gaa att tct aag gcg agc tat aac tct aaa gaa gaa ttt        960
Ser Ala Leu Glu Ile Ser Lys Ala Ser Tyr Asn Ser Lys Glu Glu Phe
305                 310                 315                 320

cta aag tta ttt cat tct ctt tca gta aaa gaa caa acc aaa att ttc       1008
Leu Lys Leu Phe His Ser Leu Ser Val Lys Glu Gln Thr Lys Ile Phe
                325                 330                 335

gtt ctt ttg aaa aag aaa aat ccg aag cac att ctt aaa aat tca att       1056
Val Leu Leu Lys Lys Lys Asn Pro Lys His Ile Leu Lys Asn Ser Ile
            340                 345                 350

ctt ccc aaa gat aaa aga tga                                           1077
Leu Pro Lys Asp Lys Arg
        355

<210>  2
<211>  358

<212>  PRT
<213>  Leptospira interrogans serovar Pomona

<400>  2

Met Gly Lys Asn Asn Ser Phe Arg Pro Lys Asn Ser Phe Leu Lys Phe
1               5                   10                  15

Gln Val Val Phe Gly Phe Phe Leu Leu Ser Phe Ile Ser Leu Asn Ser
            20                  25                  30

Glu Glu Arg Ser Ser Ile Leu Asp Pro Thr Phe Phe Leu Ile His Lys
        35                  40                  45

Asn Leu Asn Pro Ser Leu Gly Gly Leu Phe Glu Thr Arg Lys Lys Phe
    50                  55                  60

Thr Ser Tyr Gly Ala Trp Phe Glu Leu Pro Val Trp Lys Arg Glu Arg
65                  70                  75                  80

Lys His Phe Trp Lys Thr His Phe Leu Tyr Glu Asn His Lys Thr Phe
                85                  90                  95

Ser Val Val His Gln Thr Tyr Ile Gly Leu Glu Tyr Ser Phe Ile Ser
            100                 105                 110

Glu Lys Lys Asp Leu His Pro Ser Val Phe Val Gly Trp Glu Arg Gly
        115                 120                 125

Glu Lys Asp Leu Gly Ile Phe Gly Ile His Leu Thr Ile Pro Asp Lys
    130                 135                 140

Gln Thr Ile Gln Val Phe Gly Lys Thr Gly Lys Asp Phe Lys Ser Gly
145                 150                 155                 160

Ser Ile Phe Leu His Ser Asn Phe Asp Thr Gly Leu Gln Leu Phe Leu
                165                 170                 175

Gly Phe Ser Arg Thr Trp Asp Lys Ser Tyr Thr Glu Asp Gln Phe Thr
            180                 185                 190

Leu Gly Ile Arg Val Ser Trp Glu Lys Ile Tyr Ser Ser Phe Phe Trp
        195                 200                 205

Asn Gln Thr Gln Glu Glu Glu Ser Phe Leu Thr Gly Lys Phe Gly Ile
    210                 215                 220

Ser Asn Phe Gln Gln Asp Leu Lys Ser Lys Thr Leu Phe Glu Ser Thr
225                 230                 235                 240

Asp Glu Phe Gln Ser Asn Ser Leu Phe Lys Ser Lys Asn Leu Ser Ser
                245                 250                 255

Lys Asn Thr Lys Ser Lys Ser Phe Glu Thr Ser Leu Leu Glu Lys Asp
            260                 265                 270

Lys Thr Asn Tyr Ser Tyr Ile Ser Ser Phe Ile Arg Ser Tyr Thr Asn
        275                 280                 285

Ile Ser Ile Ser Val Gln Glu Leu Leu Ser Ala Gly Phe Thr Leu Ser
    290                 295                 300

Ser Ala Leu Glu Ile Ser Lys Ala Ser Tyr Asn Ser Lys Glu Glu Phe
305                 310                 315                 320

Leu Lys Leu Phe His Ser Leu Ser Val Lys Glu Gln Thr Lys Ile Phe
                325                 330                 335

Val Leu Leu Lys Lys Lys Asn Pro Lys His Ile Leu Lys Asn Ser Ile
            340                 345                 350

Leu Pro Lys Asp Lys Arg
        355

<210>  3
<211>  951
<212>  DNA
<213>  Leptospira interrogans serovar Pomona

<220>
<221>  CDS
<222>  (1)..(951)

<300>
<308>  GenBank / AAN48316
<309>  2006-02-01
<313>  (1)..(951)

<400>  3
gtg aaa cat cca aaa atg aac ctc ttg gga gcg ctt tta ctt agc tcc        48
Met Lys His Pro Lys Met Asn Leu Leu Gly Ala Leu Leu Leu Ser Ser
1               5                   10                  15

tta ttc tta aat cct ttg aat tcc gac cct acg tta ggc aac tgt gaa        96
Leu Phe Leu Asn Pro Leu Asn Ser Asp Pro Thr Leu Gly Asn Cys Glu
            20                  25                  30

gta ttt cca aca aat aat att tgg aat act ccc gta gat aca ctt ccc       144
Val Phe Pro Thr Asn Asn Ile Trp Asn Thr Pro Val Asp Thr Leu Pro
        35                  40                  45

ctg cat cct ttt tca gaa tct tat gtt cga agt att gga gca caa aaa       192
Leu His Pro Phe Ser Glu Ser Tyr Val Arg Ser Ile Gly Ala Gln Lys
    50                  55                  60

aaa tta aaa gcg gat ttc ggc tct ggg ctt tgg gag gga atg cca att       240

Lys Leu Lys Ala Asp Phe Gly Ser Gly Leu Trp Glu Gly Met Pro Ile
65                  70                  75                  80

gga att cca ttt ata cta acg tct ggt gcc aat cct gtc cca gta tct      288
Gly Ile Pro Phe Ile Leu Thr Ser Gly Ala Asn Pro Val Pro Val Ser
                85                  90                  95

ttt gaa tat act gat gaa agt gaa cct gga ccg tat cca att cca cat      336
Phe Glu Tyr Thr Asp Glu Ser Glu Pro Gly Pro Tyr Pro Ile Pro His
            100                 105                 110

aac gca cct ata gaa ggt gga gaa aca agc gac gga gac agg cac gtt      384
Asn Ala Pro Ile Glu Gly Gly Glu Thr Ser Asp Gly Asp Arg His Val
        115                 120                 125

cta gtt gta gag caa aaa aca tgc aag tta tat gaa ttg tat tcc gca      432
Leu Val Val Glu Gln Lys Thr Cys Lys Leu Tyr Glu Leu Tyr Ser Ala
    130                 135                 140

agg aaa aaa gga aaa tct tgg act gct gtg tct ggt gcg gtc ttt gat      480
Arg Lys Lys Gly Lys Ser Trp Thr Ala Val Ser Gly Ala Val Phe Asp
145                 150                 155                 160

ttg aag tcc aat caa ctt cgt ccg gct aac tgg act tct gcg gac gca      528
Leu Lys Ser Asn Gln Leu Arg Pro Ala Asn Trp Thr Ser Ala Asp Ala
                165                 170                 175

gcc gga cta cca att tta cct ggt tta gtg aga tac gaa gaa ata gct      576
Ala Gly Leu Pro Ile Leu Pro Gly Leu Val Arg Tyr Glu Glu Ile Ala
            180                 185                 190

tct gga gaa atc aaa cac gca att cgt ttc acc gct aaa aaa aca caa      624
Ser Gly Glu Ile Lys His Ala Ile Arg Phe Thr Ala Lys Lys Thr Gln
        195                 200                 205

aaa gcg tat cta tgg cct gct cgt cat tac gct tct aaa atc aca gac      672
Lys Ala Tyr Leu Trp Pro Ala Arg His Tyr Ala Ser Lys Ile Thr Asp
    210                 215                 220

aaa aac gtc cca ccg atg gga act cgt ttc cgt tta aaa gca agt ttt      720
Lys Asn Val Pro Pro Met Gly Thr Arg Phe Arg Leu Lys Ala Ser Phe
225                 230                 235                 240

aac ata gac gga ttt agt aaa gaa aac caa gta att cta cgt gca tta      768
Asn Ile Asp Gly Phe Ser Lys Glu Asn Gln Val Ile Leu Arg Ala Leu
                245                 250                 255

aaa aaa tat gga atg ata ctt gcc gac aac gga tca gat tgg ttt tta      816
Lys Lys Tyr Gly Met Ile Leu Ala Asp Asn Gly Ser Asp Trp Phe Leu
            260                 265                 270

tcc gga gct cca aac gaa aaa tgg aac aat gat caa ctt cat aaa cta      864
Ser Gly Ala Pro Asn Glu Lys Trp Asn Asn Asp Gln Leu His Lys Leu
        275                 280                 285

ggt aag gtt tta gga gat cag ttc gaa gca gta gat tca gaa agt tta      912
Gly Lys Val Leu Gly Asp Gln Phe Glu Ala Val Asp Ser Glu Ser Leu
    290                 295                 300

atg att tcg aca gat tcc gga gaa gcc aaa caa aac taa                  951
Met Ile Ser Thr Asp Ser Gly Glu Ala Lys Gln Asn
305                 310                 315

<210>  4

<211>  316
<212>  PRT
<213>  Leptospira interrogans serovar Pomona

<400>  4

Met Lys His Pro Lys Met Asn Leu Leu Gly Ala Leu Leu Leu Ser Ser
1               5                   10                  15

Leu Phe Leu Asn Pro Leu Asn Ser Asp Pro Thr Leu Gly Asn Cys Glu
            20                  25                  30

Val Phe Pro Thr Asn Asn Ile Trp Asn Thr Pro Val Asp Thr Leu Pro
        35                  40                  45

Leu His Pro Phe Ser Glu Ser Tyr Val Arg Ser Ile Gly Ala Gln Lys
    50                  55                  60

Lys Leu Lys Ala Asp Phe Gly Ser Gly Leu Trp Glu Gly Met Pro Ile
65                  70                  75                  80

Gly Ile Pro Phe Ile Leu Thr Ser Gly Ala Asn Pro Val Pro Val Ser
                85                  90                  95

Phe Glu Tyr Thr Asp Glu Ser Glu Pro Gly Pro Tyr Pro Ile Pro His
            100                 105                 110

Asn Ala Pro Ile Glu Gly Gly Glu Thr Ser Asp Gly Asp Arg His Val
        115                 120                 125

Leu Val Val Glu Gln Lys Thr Cys Lys Leu Tyr Glu Leu Tyr Ser Ala
    130                 135                 140

Arg Lys Lys Gly Lys Ser Trp Thr Ala Val Ser Gly Ala Val Phe Asp
145                 150                 155                 160

Leu Lys Ser Asn Gln Leu Arg Pro Ala Asn Trp Thr Ser Ala Asp Ala
                165                 170                 175

Ala Gly Leu Pro Ile Leu Pro Gly Leu Val Arg Tyr Glu Glu Ile Ala
            180                 185                 190

Ser Gly Glu Ile Lys His Ala Ile Arg Phe Thr Ala Lys Lys Thr Gln
        195                 200                 205

Lys Ala Tyr Leu Trp Pro Ala Arg His Tyr Ala Ser Lys Ile Thr Asp
    210                 215                 220

Lys Asn Val Pro Pro Met Gly Thr Arg Phe Arg Leu Lys Ala Ser Phe
225                 230                 235                 240

Asn Ile Asp Gly Phe Ser Lys Glu Asn Gln Val Ile Leu Arg Ala Leu
                245                 250                 255

Lys Lys Tyr Gly Met Ile Leu Ala Asp Asn Gly Ser Asp Trp Phe Leu
            260                 265                 270

Ser Gly Ala Pro Asn Glu Lys Trp Asn Asn Asp Gln Leu His Lys Leu
        275                 280                 285

Gly Lys Val Leu Gly Asp Gln Phe Glu Ala Val Asp Ser Glu Ser Leu
    290                 295                 300

Met Ile Ser Thr Asp Ser Gly Glu Ala Lys Gln Asn
305                 310                 315

<210>  5
<211>  480
<212>  DNA
<213>  Leptospira interrogans serovar Pomona

<220>
<221>  CDS
<222>  (1)..(480)

<300>
<308>  GenBank /  AAN49138
<309>  2006-02-01
<313>  (1)..(480)

<400>  5
atg aaa aaa cat tct atc agt aaa atc att ata act gct tgt tgc att      48
Met Lys Lys His Ser Ile Ser Lys Ile Ile Ile Thr Ala Cys Cys Ile
1               5                   10                  15

ttt ctt tta aca tac gga tgc aaa caa gat cca gta gat tac aat aat      96
Phe Leu Leu Thr Tyr Gly Cys Lys Gln Asp Pro Val Asp Tyr Asn Asn
            20                  25                  30

aaa atc atg gaa atc atg aac gct tct aca aac gat tta gat gcg tta      144
Lys Ile Met Glu Ile Met Asn Ala Ser Thr Asn Asp Leu Asp Ala Leu
        35                  40                  45

aac gca gcc atg gaa aag gaa gac ctt aca aac gca gaa aat gtt aga      192
Asn Ala Ala Met Glu Lys Glu Asp Leu Thr Asn Ala Glu Asn Val Arg
    50                  55                  60

aaa gct tgg gaa aca aag cta gtt tct tca ctc gat aag ctt aaa gga      240
Lys Ala Trp Glu Thr Lys Leu Val Ser Ser Leu Asp Lys Leu Lys Gly
65                  70                  75                  80

atc agt gat ttt aaa gga gat tcc agt ttt aaa aat gca agc gtc caa      288
Ile Ser Asp Phe Lys Gly Asp Ser Ser Phe Lys Asn Ala Ser Val Gln
                85                  90                  95

gct ctc gaa act tat ttg aac ata gta agt aaa gac tac aaa cgt ttg      336
Ala Leu Glu Thr Tyr Leu Asn Ile Val Ser Lys Asp Tyr Lys Arg Leu
            100                 105                 110

atc gaa tta cga gga tta ggt gac aaa gca gac tca aat gaa atc aac 384
Ile Glu Leu Arg Gly Leu Gly Asp Lys Ala Asp Ser Asn Glu Ile Asn
        115                 120                 125

caa gtt ctc aat cgt att aat cag gat ttt gaa aaa gct gca aat act 432
Gln Val Leu Asn Arg Ile Asn Gln Asp Phe Glu Lys Ala Ala Asn Thr
    130                 135                 140

ctc aat gct gct tct gat aaa ttt gcg aaa gaa tac gct tct caa taa 480
Leu Asn Ala Ala Ser Asp Lys Phe Ala Lys Glu Tyr Ala Ser Gln
145                 150                 155

<210> 6
<211> 159
<212> PRT
<213> Leptospira interrogans serovar Pomona

<400> 6

Met Lys Lys His Ser Ile Ser Lys Ile Ile Ile Thr Ala Cys Cys Ile
1               5                   10                  15

Phe Leu Leu Thr Tyr Gly Cys Lys Gln Asp Pro Val Asp Tyr Asn Asn
            20                  25                  30

Lys Ile Met Glu Ile Met Asn Ala Ser Thr Asn Asp Leu Asp Ala Leu
        35                  40                  45

Asn Ala Ala Met Glu Lys Glu Asp Leu Thr Asn Ala Glu Asn Val Arg
    50                  55                  60

Lys Ala Trp Glu Thr Lys Leu Val Ser Ser Leu Asp Lys Leu Lys Gly
65                  70                  75                  80

Ile Ser Asp Phe Lys Gly Asp Ser Ser Phe Lys Asn Ala Ser Val Gln
                85                  90                  95

Ala Leu Glu Thr Tyr Leu Asn Ile Val Ser Lys Asp Tyr Lys Arg Leu
            100                 105                 110

Ile Glu Leu Arg Gly Leu Gly Asp Lys Ala Asp Ser Asn Glu Ile Asn
        115                 120                 125

Gln Val Leu Asn Arg Ile Asn Gln Asp Phe Glu Lys Ala Ala Asn Thr
    130                 135                 140

Leu Asn Ala Ala Ser Asp Lys Phe Ala Lys Glu Tyr Ala Ser Gln
145                 150                 155

<210> 7
<211> 801
<212> DNA
<213> Leptospira interrogans serovar Pomona

<220>
<221>  CDS
<222>  (1)..(801)

<400>  7
atg aat ctt tcc aaa cac aca gcc gtt ctc aca ggg att gtt ttc ttc      48
Met Asn Leu Ser Lys His Thr Ala Val Leu Thr Gly Ile Val Phe Phe
1               5                   10                  15

tta gca ttt tcc tta agt atg tat gtc tcc gtt att gaa aaa gcc gga      96
Leu Ala Phe Ser Leu Ser Met Tyr Val Ser Val Ile Glu Lys Ala Gly
            20                  25                  30

acc aaa gac gaa tat cca tat acc atg aaa att tat tat ccc cgt ttg      144
Thr Lys Asp Glu Tyr Pro Tyr Thr Met Lys Ile Tyr Tyr Pro Arg Leu
        35                  40                  45

gaa gga att cat cca ggt gca ccc gtt cga att tta gga gta gaa aaa      192
Glu Gly Ile His Pro Gly Ala Pro Val Arg Ile Leu Gly Val Glu Lys
    50                  55                  60

gga att gta cgt agt tta gac gtg gtt ccg att gac gaa gta gaa gat      240
Gly Ile Val Arg Ser Leu Asp Val Val Pro Ile Asp Glu Val Glu Asp
65                  70                  75                  80

caa aga ttc ctc aat aag gat caa acg aag gca atc gaa att att gtt      288
Gln Arg Phe Leu Asn Lys Asp Gln Thr Lys Ala Ile Glu Ile Ile Val
                85                  90                  95

aga ctg aaa gag cca atc aca ctc tgg gac aat tat aaa att aca ttc      336
Arg Leu Lys Glu Pro Ile Thr Leu Trp Asp Asn Tyr Lys Ile Thr Phe
            100                 105                 110

caa acc aac acg att ctc tct gga aga acc atc gat ata gac cct gga      384
Gln Thr Asn Thr Ile Leu Ser Gly Arg Thr Ile Asp Ile Asp Pro Gly
        115                 120                 125

tct ttc gat aaa gaa gag acg tcc ttt ttt caa cct act tac tta gaa      432
Ser Phe Asp Lys Glu Glu Thr Ser Phe Phe Gln Pro Thr Tyr Leu Glu
    130                 135                 140

gaa gaa caa aaa tct cca gac ttc tta cct tct gca gat tac ttt gaa      480
Glu Glu Gln Lys Ser Pro Asp Phe Leu Pro Ser Ala Asp Tyr Phe Glu
145                 150                 155                 160

gat ttt ttc gcg gct tcc acg gga gtc atc cga gaa aat cgt gaa gac      528
Asp Phe Phe Ala Ala Ser Thr Gly Val Ile Arg Glu Asn Arg Glu Asp
                165                 170                 175

atc cga act tct ttt aat aat ttt tat gaa att tca gaa aaa ttg aaa      576
Ile Arg Thr Ser Phe Asn Asn Phe Tyr Glu Ile Ser Glu Lys Leu Lys
            180                 185                 190

tca aat cga gga aca att cct cag atc att aac tct ccg gaa acg tac      624
Ser Asn Arg Gly Thr Ile Pro Gln Ile Ile Asn Ser Pro Glu Thr Tyr
        195                 200                 205

gat aac gtg ata gaa tta ctt aca gat gct aga att ttc ggt aac gat      672
Asp Asn Val Ile Glu Leu Leu Thr Asp Ala Arg Ile Phe Gly Asn Asp
    210                 215                 220

gct cgt cgt tat ctg gaa ggg aac cgt aag ttg gaa cgt tct gct ccg      720
Ala Arg Arg Tyr Leu Glu Gly Asn Arg Lys Leu Glu Arg Ser Ala Pro

225                 230                 235                 240

att cca ctc acg att aat atg tat cgt aga act act ttg atc gga aac       768
Ile Pro Leu Thr Ile Asn Met Tyr Arg Arg Thr Thr Leu Ile Gly Asn
                245                 250                 255

gtg agt aat cgg tat tat ttc gga aag tta taa                           801
Val Ser Asn Arg Tyr Tyr Phe Gly Lys Leu
            260                 265


<210>  8
<211>  266
<212>  PRT
<213>  Leptospira interrogans serovar Pomona

<400>  8

Met Asn Leu Ser Lys His Thr Ala Val Leu Thr Gly Ile Val Phe Phe
1               5                   10                  15

Leu Ala Phe Ser Leu Ser Met Tyr Val Ser Val Ile Glu Lys Ala Gly
            20                  25                  30

Thr Lys Asp Glu Tyr Pro Tyr Thr Met Lys Ile Tyr Tyr Pro Arg Leu
        35                  40                  45

Glu Gly Ile His Pro Gly Ala Pro Val Arg Ile Leu Gly Val Glu Lys
    50                  55                  60

Gly Ile Val Arg Ser Leu Asp Val Val Pro Ile Asp Glu Val Glu Asp
65                  70                  75                  80

Gln Arg Phe Leu Asn Lys Asp Gln Thr Lys Ala Ile Glu Ile Ile Val
                85                  90                  95

Arg Leu Lys Glu Pro Ile Thr Leu Trp Asp Asn Tyr Lys Ile Thr Phe
            100                 105                 110

Gln Thr Asn Thr Ile Leu Ser Gly Arg Thr Ile Asp Ile Asp Pro Gly
        115                 120                 125

Ser Phe Asp Lys Glu Glu Thr Ser Phe Phe Gln Pro Thr Tyr Leu Glu
    130                 135                 140

Glu Glu Gln Lys Ser Pro Asp Phe Leu Pro Ser Ala Asp Tyr Phe Glu
145                 150                 155                 160

Asp Phe Phe Ala Ala Ser Thr Gly Val Ile Arg Glu Asn Arg Glu Asp
                165                 170                 175

Ile Arg Thr Ser Phe Asn Asn Phe Tyr Glu Ile Ser Glu Lys Leu Lys
            180                 185                 190

Ser Asn Arg Gly Thr Ile Pro Gln Ile Ile Asn Ser Pro Glu Thr Tyr
        195                 200                 205

Asp Asn Val Ile Glu Leu Leu Thr Asp Ala Arg Ile Phe Gly Asn Asp
    210                 215                 220

Ala Arg Arg Tyr Leu Glu Gly Asn Arg Lys Leu Glu Arg Ser Ala Pro
225                 230                 235                 240

Ile Pro Leu Thr Ile Asn Met Tyr Arg Arg Thr Thr Leu Ile Gly Asn
                245                 250                 255

Val Ser Asn Arg Tyr Tyr Phe Gly Lys Leu
            260                 265

<210>  9
<211>  1263
<212>  DNA
<213>  Leptospira interrogans serovar Pomona

<220>
<221>  CDS
<222>  (1)..(1263)

<400>  9
atg ctc aac aca aaa ata aag aaa aaa tta ttt ctc tca gtt att ttt      48
Met Leu Asn Thr Lys Ile Lys Lys Lys Leu Phe Leu Ser Val Ile Phe
1               5                   10                  15

ttt cta ttt cta cca tcg gtc gcc tgt gcg gaa aaa agt ttt cgt aaa      96
Phe Leu Phe Leu Pro Ser Val Ala Cys Ala Glu Lys Ser Phe Arg Lys
            20                  25                  30

cat atc gcg gac tca aaa ctc att cct tcc gaa atc gaa ttt tat tct      144
His Ile Ala Asp Ser Lys Leu Ile Pro Ser Glu Ile Glu Phe Tyr Ser
        35                  40                  45

aac att ctt ccc gga ctt tcc gga aaa aat gtc att cta att aca aac      192
Asn Ile Leu Pro Gly Leu Ser Gly Lys Asn Val Ile Leu Ile Thr Asn
    50                  55                  60

cca tct gga atc gga aga agc ccc gaa agg att tta cga gaa ttt aaa      240
Pro Ser Gly Ile Gly Arg Ser Pro Glu Arg Ile Leu Arg Glu Phe Lys
65                  70                  75                  80

aaa cac gac gta aaa atc aaa cat ctc atc gga ttg gaa cac gga ttt      288
Lys His Asp Val Lys Ile Lys His Leu Ile Gly Leu Glu His Gly Phe
                85                  90                  95

tta gga ctc gag gag gac ttc agt aaa tct ccc gtt acg gtg gat gaa      336
Leu Gly Leu Glu Glu Asp Phe Ser Lys Ser Pro Val Thr Val Asp Glu
            100                 105                 110

ttt ttt aat ctc cca atc tat cat atc tat cga gtc aag aac gca gaa      384
Phe Phe Asn Leu Pro Ile Tyr His Ile Tyr Arg Val Lys Asn Ala Glu
        115                 120                 125

ctt cct acg att ttg aaa gga gcc gac gcg att ctt ttt gat gtg caa      432

Leu Pro Thr Ile Leu Lys Gly Ala Asp Ala Ile Leu Phe Asp Val Gln
    130                 135                 140

gat atg ggg atg aga tgt tat act tat cta acc gtt tta aaa aga att      480
Asp Met Gly Met Arg Cys Tyr Thr Tyr Leu Thr Val Leu Lys Arg Ile
145                 150                 155                 160

atg gat gga att cca gat cct aca aat acg aga ctg atc gtt ttg gat      528
Met Asp Gly Ile Pro Asp Pro Thr Asn Thr Arg Leu Ile Val Leu Asp
                165                 170                 175

cac gta aac ccc gct ctt tat tta aaa gga aga gga gaa atg atc gat      576
His Val Asn Pro Ala Leu Tyr Leu Lys Gly Arg Gly Glu Met Ile Asp
            180                 185                 190

aaa cgt ttc tta aat ttt gca gga gaa ttc cct tct ctt ttt ttt gga      624
Lys Arg Phe Leu Asn Phe Ala Gly Glu Phe Pro Ser Leu Phe Phe Gly
        195                 200                 205

ggt ttg acc ttg gga gaa tca gcg gtt tat tat aac tct gaa tat tta      672
Gly Leu Thr Leu Gly Glu Ser Ala Val Tyr Tyr Asn Ser Glu Tyr Leu
    210                 215                 220

gat aaa aag gtt cgt tta gaa gtg gta tct cct aaa aac gca aaa cga      720
Asp Lys Lys Val Arg Leu Glu Val Val Ser Pro Lys Asn Ala Lys Arg
225                 230                 235                 240

tct ttt gat tgg gat aga gaa gga att cct tgg act aca cct tct cct      768
Ser Phe Asp Trp Asp Arg Glu Gly Ile Pro Trp Thr Thr Pro Ser Pro
                245                 250                 255

aat tta cca act ata gat tct gcg atc aat tat ctg ggg ctt gtt ttg      816
Asn Leu Pro Thr Ile Asp Ser Ala Ile Asn Tyr Leu Gly Leu Val Leu
            260                 265                 270

tta gaa gga gtg aac gtt tct gtg gga agg ggt aca acc gca cct ttt      864
Leu Glu Gly Val Asn Val Ser Val Gly Arg Gly Thr Thr Ala Pro Phe
        275                 280                 285

gta tat ttc gga gcg cct tgg atg aca gag ccc gaa aag tta gcg gaa      912
Val Tyr Phe Gly Ala Pro Trp Met Thr Glu Pro Glu Lys Leu Ala Glu
    290                 295                 300

gaa tta aat caa aat tca ggt gga gaa tat tat tat cag act gtg ttt      960
Glu Leu Asn Gln Asn Ser Gly Gly Glu Tyr Tyr Tyr Gln Thr Val Phe
305                 310                 315                 320

ttt aaa cct gta ttt ggt cct tac aag aat gag att tgt aga gga ttg     1008
Phe Lys Pro Val Phe Gly Pro Tyr Lys Asn Glu Ile Cys Arg Gly Leu
                325                 330                 335

cgc cta acg gta gta aat cga aag tat gat cct tta aaa atg gca ttc     1056
Arg Leu Thr Val Val Asn Arg Lys Tyr Asp Pro Leu Lys Met Ala Phe
            340                 345                 350

cag ttg atc tcc gct tta aaa tcg aat tat aaa gaa ttt aaa tgg aga     1104
Gln Leu Ile Ser Ala Leu Lys Ser Asn Tyr Lys Glu Phe Lys Trp Arg
        355                 360                 365

tcg tat ccg gat gga acc tac aat ata gat ttc cta tgg gga acg gaa     1152
Ser Tyr Pro Asp Gly Thr Tyr Asn Ile Asp Phe Leu Trp Gly Thr Glu
    370                 375                 380

tcg ttt cga aaa acg atc gac gcc gga aaa aaa tac gat caa tat gcg     1200
Ser Phe Arg Lys Thr Ile Asp Ala Gly Lys Lys Tyr Asp Gln Tyr Ala

385                 390                 395                 400

gaa tac tta agt tcc att gag aaa gaa tat aat gaa aaa att aag aaa      1248
Glu Tyr Leu Ser Ser Ile Glu Lys Glu Tyr Asn Glu Lys Ile Lys Lys
                405                 410                 415

tat tat ctc tac tga                                                  1263
Tyr Tyr Leu Tyr
            420

<210>  10
<211>  420
<212>  PRT
<213>  Leptospira interrogans serovar Pomona

<400>  10

Met Leu Asn Thr Lys Ile Lys Lys Lys Leu Phe Leu Ser Val Ile Phe
1               5                   10                  15

Phe Leu Phe Leu Pro Ser Val Ala Cys Ala Glu Lys Ser Phe Arg Lys
            20                  25                  30

His Ile Ala Asp Ser Lys Leu Ile Pro Ser Glu Ile Glu Phe Tyr Ser
        35                  40                  45

Asn Ile Leu Pro Gly Leu Ser Gly Lys Asn Val Ile Leu Ile Thr Asn
    50                  55                  60

Pro Ser Gly Ile Gly Arg Ser Pro Glu Arg Ile Leu Arg Glu Phe Lys
65                  70                  75                  80

Lys His Asp Val Lys Ile Lys His Leu Ile Gly Leu Glu His Gly Phe
                85                  90                  95

Leu Gly Leu Glu Glu Asp Phe Ser Lys Ser Pro Val Thr Val Asp Glu
            100                 105                 110

Phe Phe Asn Leu Pro Ile Tyr His Ile Tyr Arg Val Lys Asn Ala Glu
        115                 120                 125

Leu Pro Thr Ile Leu Lys Gly Ala Asp Ala Ile Leu Phe Asp Val Gln
    130                 135                 140

Asp Met Gly Met Arg Cys Tyr Thr Tyr Leu Thr Val Leu Lys Arg Ile
145                 150                 155                 160

Met Asp Gly Ile Pro Asp Pro Thr Asn Thr Arg Leu Ile Val Leu Asp
                165                 170                 175

His Val Asn Pro Ala Leu Tyr Leu Lys Gly Arg Gly Glu Met Ile Asp
            180                 185                 190

Lys Arg Phe Leu Asn Phe Ala Gly Glu Phe Pro Ser Leu Phe Phe Gly
        195                 200                 205

Gly Leu Thr Leu Gly Glu Ser Ala Val Tyr Tyr Asn Ser Glu Tyr Leu
    210                 215                 220

Asp Lys Lys Val Arg Leu Glu Val Val Ser Pro Lys Asn Ala Lys Arg
225                 230                 235                 240

Ser Phe Asp Trp Asp Arg Glu Gly Ile Pro Trp Thr Thr Pro Ser Pro
                245                 250                 255

Asn Leu Pro Thr Ile Asp Ser Ala Ile Asn Tyr Leu Gly Leu Val Leu
            260                 265                 270

Leu Glu Gly Val Asn Val Ser Val Gly Arg Gly Thr Thr Ala Pro Phe
        275                 280                 285

Val Tyr Phe Gly Ala Pro Trp Met Thr Glu Pro Glu Lys Leu Ala Glu
    290                 295                 300

Glu Leu Asn Gln Asn Ser Gly Gly Glu Tyr Tyr Tyr Gln Thr Val Phe
305                 310                 315                 320

Phe Lys Pro Val Phe Gly Pro Tyr Lys Asn Glu Ile Cys Arg Gly Leu
                325                 330                 335

Arg Leu Thr Val Val Asn Arg Lys Tyr Asp Pro Leu Lys Met Ala Phe
            340                 345                 350

Gln Leu Ile Ser Ala Leu Lys Ser Asn Tyr Lys Glu Phe Lys Trp Arg
        355                 360                 365

Ser Tyr Pro Asp Gly Thr Tyr Asn Ile Asp Phe Leu Trp Gly Thr Glu
    370                 375                 380

Ser Phe Arg Lys Thr Ile Asp Ala Gly Lys Lys Tyr Asp Gln Tyr Ala
385                 390                 395                 400

Glu Tyr Leu Ser Ser Ile Glu Lys Glu Tyr Asn Glu Lys Ile Lys Lys
                405                 410                 415

Tyr Tyr Leu Tyr
            420

<210>  11
<211>  266
<212>  PRT
<213>  Leptospira interrogans serovar Lai

<300>
<308>  GenBank / AAN47806
<309>  2006-02-01
<313>  (1)..(266)

<400>  11

Met Asn Leu Ser Lys His Thr Ala Val Leu Thr Gly Ile Val Phe Phe
1               5                   10                  15

Leu Ala Phe Ser Leu Ser Met Tyr Val Ser Val Ile Glu Lys Ala Gly
            20                  25                  30

Thr Lys Asp Glu Tyr Pro Tyr Thr Met Lys Ile Tyr Tyr Pro Arg Leu
        35                  40                  45

Glu Gly Ile His Pro Gly Ala Pro Val Arg Ile Leu Gly Val Glu Lys
    50                  55                  60

Gly Ile Val Arg Ser Leu Asp Val Val Pro Ile Asp Glu Val Glu Asp
65                  70                  75                  80

Gln Arg Phe Leu Asn Lys Asp Gln Thr Lys Ala Ile Glu Ile Ile Val
                85                  90                  95

Arg Leu Lys Glu Pro Ile Thr Leu Trp Asp Asn Tyr Lys Ile Thr Phe
            100                 105                 110

Gln Thr Asn Thr Ile Leu Ser Gly Arg Thr Ile Asp Ile Asp Pro Gly
        115                 120                 125

Ser Phe Asp Lys Glu Glu Thr Ser Phe Phe Gln Pro Thr Tyr Leu Glu
    130                 135                 140

Glu Glu Gln Lys Ser Pro Asp Phe Leu Pro Ser Ala Asp Tyr Phe Glu
145                 150                 155                 160

Asp Phe Phe Ala Ala Ser Thr Gly Val Ile Arg Glu Asn Arg Glu Asp
                165                 170                 175

Ile Arg Thr Ser Phe Asn Asn Phe Tyr Glu Ile Ser Glu Lys Leu Lys
            180                 185                 190

Ser Asn Arg Gly Thr Ile Pro Gln Ile Ile Asn Ser Pro Glu Thr Tyr
        195                 200                 205

Asp Asn Val Ile Glu Leu Leu Thr Asp Ala Arg Ile Phe Gly Asn Asp
    210                 215                 220

Ala Arg Arg Tyr Leu Glu Gly Asn Arg Lys Leu Glu Arg Ser Ala Pro

225                 230                 235                 240

Ile Pro Leu Thr Ile Asn Met Tyr Arg Arg Thr Thr Leu Ile Gly Asn
                245                 250                 255

Val Ser Asn Arg Tyr Tyr Phe Gly Lys Leu
            260                 265

<210>  12
<211>  251
<212>  PRT
<213>  Leptospira interrogans serovar Lai

<300>
<308>  GenBank / AAN49254
<309>  2006-02-01
<313>  (1)..(251)

<400>  12

Met Asn Met Asn Ser Leu Arg Tyr Leu Leu Val Gly Ile Ile Phe Thr
1               5                   10                  15

Ala Ala Ile Thr Val Val Gly Tyr Phe Thr Ile Ile Thr Glu Gly Gly
            20                  25                  30

Pro Ile Lys Lys Lys Gly Glu Phe Met Lys Val Thr Phe Arg Asn Ala
        35                  40                  45

Glu Gly Ile Lys Val Gly Asn Lys Val Thr Val Gln Gly Val Pro Phe
    50                  55                  60

Gly Tyr Val Ser Ala Ile Arg Leu Ile Gln Ile Asp Glu Asn Gly Thr
65                  70                  75                  80

Glu Val Gln Ser Gly Glu Met Gly Ile Gly Thr Arg Val Glu Ile Thr
                85                  90                  95

Met Leu Leu Arg Glu Lys Ile Ser Leu Tyr Asp Asn Tyr Asp Ile Ile
            100                 105                 110

Ile Lys Asn Glu Ser Leu Leu Thr Gly Arg Val Ile Ala Ile Asp Pro
        115                 120                 125

Gly Thr Ala Asp Leu Glu Pro Lys Gln Leu Lys Thr Arg Thr Thr Pro
    130                 135                 140

Ile Thr Met Ile Asp Tyr Lys Thr Thr Gly Ser Leu Lys Gly Arg Val
145                 150                 155                 160

Leu Gln Asp Pro Leu Val Ser Leu Ser Glu Leu Ile Ser Glu Asn Arg
                165                 170                 175

Gly Asp Ile Arg Lys Thr Phe Ser Asn Ile Ala Asp Ile Thr Thr Lys
            180                 185                 190

Ile Asn Thr Gly Asp Gly Ser Leu Gly Arg Leu Ile Asn Asn Asp Asp
        195                 200                 205

Val His Lys Asn Val Asn Thr Val Leu Thr Asp Ala Gln Ile Val Leu
    210                 215                 220

Arg Glu Leu Arg Glu Gly Leu Glu Asp Thr Arg Glu Gln Thr Pro Val
225                 230                 235                 240

Thr Ser Phe Ile Arg Ala Ala Leu Ser Ala Phe
                245                 250

<210>  13
<211>  1323
<212>  DNA
<213>  Mycobacterium tuberculosis CDC1551

<220>
<221>  CDS
<222>  (1)..(1323)

<300>
<308>  GenBank / AE000516
<309>  2004-08-04
<313>  (91)..(879)

<400>  13
gtg cga cgt cgt gct caa ggt caa cgg caa ggg cgg cca gcc ggt gta      48
Met Arg Arg Arg Ala Gln Gly Gln Arg Gln Gly Arg Pro Ala Gly Val
1               5                   10                  15

cat caa gct ggc cgg tca gga cag cgg gcg gtg cgc gcc gaa atg aaa      96
His Gln Ala Gly Arg Ser Gly Gln Arg Ala Val Arg Ala Glu Met Lys
            20                  25                  30

tcc ttc gcc gaa cgc aac cgt ctg gcc atc ggc aca gtc ggc atc gtc      144
Ser Phe Ala Glu Arg Asn Arg Leu Ala Ile Gly Thr Val Gly Ile Val
        35                  40                  45

gtc gtc gcc gcc gtt gcg ctg gcc gcg ctg caa tac cag cgg ctg ccg      192
Val Val Ala Ala Val Ala Leu Ala Ala Leu Gln Tyr Gln Arg Leu Pro
    50                  55                  60

ttt ttc aac cag ggc acc agg gtc tcc gcc tat ttc gcc gac gcc ggc      240
Phe Phe Asn Gln Gly Thr Arg Val Ser Ala Tyr Phe Ala Asp Ala Gly
65                  70                  75                  80

ggg ctg cgc acc ggc aac acc gtc gag gtc tcc ggc tat ccg gtg gga      288
Gly Leu Arg Thr Gly Asn Thr Val Glu Val Ser Gly Tyr Pro Val Gly
                85                  90                  95

aag gtg tcc agc atc tcg ctc gac gga ccg ggc gtg ctg gtg gag ttc      336
Lys Val Ser Ser Ile Ser Leu Asp Gly Pro Gly Val Leu Val Glu Phe
            100                 105                 110

aag gtc gac acc gac gtc cga ctc gga aac cgc acc gaa gtg gca atc 384
Lys Val Asp Thr Asp Val Arg Leu Gly Asn Arg Thr Glu Val Ala Ile
        115                 120                 125

aaa acc aag ggc ttg ttg ggc agc aag ttc ctc gac gtc acc ccc cgc 432
Lys Thr Lys Gly Leu Leu Gly Ser Lys Phe Leu Asp Val Thr Pro Arg
    130                 135                 140

ggg gac ggc cga ctc gat tct ccg atc ccg atc gag cgg acc acg tcg 480
Gly Asp Gly Arg Leu Asp Ser Pro Ile Pro Ile Glu Arg Thr Thr Ser
145                 150                 155                 160

ccc tac caa ctg ccc gac gcc ctt ggc gat ttg gcc gcc acg atc agc 528
Pro Tyr Gln Leu Pro Asp Ala Leu Gly Asp Leu Ala Ala Thr Ile Ser
                165                 170                 175

ggg ttg cac acc gag cgg ctg tcc gaa tcg ctg gcc acc ctg gcg cag 576
Gly Leu His Thr Glu Arg Leu Ser Glu Ser Leu Ala Thr Leu Ala Gln
            180                 185                 190

acc ttt gcc gat acg ccg gcg cac ttc cgc aac gcc ata cac ggg gtg 624
Thr Phe Ala Asp Thr Pro Ala His Phe Arg Asn Ala Ile His Gly Val
        195                 200                 205

gcc cgg ctc gcc caa acc ctc gat gag cgc gac aac caa ctg cgc agc 672
Ala Arg Leu Ala Gln Thr Leu Asp Glu Arg Asp Asn Gln Leu Arg Ser
    210                 215                 220

ctg ctg gcc aac gcg gcc aaa gcc acc ggg gtg ctg gcc aac cgc acc 720
Leu Leu Ala Asn Ala Ala Lys Ala Thr Gly Val Leu Ala Asn Arg Thr
225                 230                 235                 240

gac cag atc gtc ggc ctg gtg cgc gac acg aat gtg gtc ttg gcg cag 768
Asp Gln Ile Val Gly Leu Val Arg Asp Thr Asn Val Val Leu Ala Gln
                245                 250                 255

ctg cgc acc caa agc gcc gcc ctg gac cgg atc tgg gcg aac atc tcg 816
Leu Arg Thr Gln Ser Ala Ala Leu Asp Arg Ile Trp Ala Asn Ile Ser
            260                 265                 270

gcg gtg gcc gaa caa ctg cgg ggc ttc atc gct gag aac cgc cag cag 864
Ala Val Ala Glu Gln Leu Arg Gly Phe Ile Ala Glu Asn Arg Gln Gln
        275                 280                 285

ctg cgc ccg gcg ctg gac aag ctc aac ggg gtg ctg gct atc gtc gaa 912
Leu Arg Pro Ala Leu Asp Lys Leu Asn Gly Val Leu Ala Ile Val Glu
    290                 295                 300

aac cgc aaa gag cgt gtg cgg cag gcc atc ccg ctg atc aac acc tat 960
Asn Arg Lys Glu Arg Val Arg Gln Ala Ile Pro Leu Ile Asn Thr Tyr
305                 310                 315                 320

gtc atg tcg ctg ggt gag tcg ctg tcg tcg ggc ccg ttc ttc aag gca 1008
Val Met Ser Leu Gly Glu Ser Leu Ser Ser Gly Pro Phe Phe Lys Ala
                325                 330                 335

tac gtg gtg aac ctg ctg ccg ggt cag ttc gtg caa ccg ttc atc agc 1056
Tyr Val Val Asn Leu Leu Pro Gly Gln Phe Val Gln Pro Phe Ile Ser
            340                 345                 350

gcc gcg ttc tcc gac ctg ggg ctc gac ccg gcc acg ttg ctg ccg tcg 1104
Ala Ala Phe Ser Asp Leu Gly Leu Asp Pro Ala Thr Leu Leu Pro Ser
        355                 360                 365

cag ctg acc gac cca ccg acc ggt caa ccc gga acc ccg ccg ttg ccg          1152
Gln Leu Thr Asp Pro Pro Thr Gly Gln Pro Gly Thr Pro Pro Leu Pro
    370                 375                 380

atg ccc tac ccg cgc acg ggc cag ggc ggt gag ccg cgg ctg acg ctg          1200
Met Pro Tyr Pro Arg Thr Gly Gln Gly Gly Glu Pro Arg Leu Thr Leu
385                 390                 395                 400

ccc gac gcg atc acc ggc aat ccc ggc gat ccg cgc tat ccg tac cgg          1248
Pro Asp Ala Ile Thr Gly Asn Pro Gly Asp Pro Arg Tyr Pro Tyr Arg
                405                 410                 415

ccg gag ccg ccc gcg ccg ccg ccc ggc ggg ccg ccg ccc ggc ccg ccc          1296
Pro Glu Pro Pro Ala Pro Pro Pro Gly Gly Pro Pro Pro Gly Pro Pro
            420                 425                 430

gcg cag cag ccg gga gac caa ccg tga                                      1323
Ala Gln Gln Pro Gly Asp Gln Pro
        435                 440


<210>  14
<211>  440
<212>  PRT
<213>  Mycobacterium tuberculosis CDC1551

<400>  14

Met Arg Arg Arg Ala Gln Gly Gln Arg Gln Gly Arg Pro Ala Gly Val
1               5                   10                  15


His Gln Ala Gly Arg Ser Gly Gln Arg Ala Val Arg Ala Glu Met Lys
            20                  25                  30


Ser Phe Ala Glu Arg Asn Arg Leu Ala Ile Gly Thr Val Gly Ile Val
        35                  40                  45


Val Val Ala Ala Val Ala Leu Ala Ala Leu Gln Tyr Gln Arg Leu Pro
    50                  55                  60


Phe Phe Asn Gln Gly Thr Arg Val Ser Ala Tyr Phe Ala Asp Ala Gly
65                  70                  75                  80


Gly Leu Arg Thr Gly Asn Thr Val Glu Val Ser Gly Tyr Pro Val Gly
                85                  90                  95


Lys Val Ser Ser Ile Ser Leu Asp Gly Pro Gly Val Leu Val Glu Phe
            100                 105                 110


Lys Val Asp Thr Asp Val Arg Leu Gly Asn Arg Thr Glu Val Ala Ile
        115                 120                 125


Lys Thr Lys Gly Leu Leu Gly Ser Lys Phe Leu Asp Val Thr Pro Arg
    130                 135                 140


Gly Asp Gly Arg Leu Asp Ser Pro Ile Pro Ile Glu Arg Thr Thr Ser

145                 150                 155                 160

Pro Tyr Gln Leu Pro Asp Ala Leu Gly Asp Leu Ala Ala Thr Ile Ser
                165                 170                 175

Gly Leu His Thr Glu Arg Leu Ser Glu Ser Leu Ala Thr Leu Ala Gln
            180                 185                 190

Thr Phe Ala Asp Thr Pro Ala His Phe Arg Asn Ala Ile His Gly Val
        195                 200                 205

Ala Arg Leu Ala Gln Thr Leu Asp Glu Arg Asp Asn Gln Leu Arg Ser
    210                 215                 220

Leu Leu Ala Asn Ala Ala Lys Ala Thr Gly Val Leu Ala Asn Arg Thr
225                 230                 235                 240

Asp Gln Ile Val Gly Leu Val Arg Asp Thr Asn Val Val Leu Ala Gln
                245                 250                 255

Leu Arg Thr Gln Ser Ala Ala Leu Asp Arg Ile Trp Ala Asn Ile Ser
            260                 265                 270

Ala Val Ala Glu Gln Leu Arg Gly Phe Ile Ala Glu Asn Arg Gln Gln
        275                 280                 285

Leu Arg Pro Ala Leu Asp Lys Leu Asn Gly Val Leu Ala Ile Val Glu
    290                 295                 300

Asn Arg Lys Glu Arg Val Arg Gln Ala Ile Pro Leu Ile Asn Thr Tyr
305                 310                 315                 320

Val Met Ser Leu Gly Glu Ser Leu Ser Ser Gly Pro Phe Phe Lys Ala
                325                 330                 335

Tyr Val Val Asn Leu Leu Pro Gly Gln Phe Val Gln Pro Phe Ile Ser
            340                 345                 350

Ala Ala Phe Ser Asp Leu Gly Leu Asp Pro Ala Thr Leu Leu Pro Ser
        355                 360                 365

Gln Leu Thr Asp Pro Pro Thr Gly Gln Pro Gly Thr Pro Pro Leu Pro
    370                 375                 380

Met Pro Tyr Pro Arg Thr Gly Gln Gly Gly Glu Pro Arg Leu Thr Leu
385                 390                 395                 400

Pro Asp Ala Ile Thr Gly Asn Pro Gly Asp Pro Arg Tyr Pro Tyr Arg
                405                 410                 415

Pro Glu Pro Pro Ala Pro Pro Pro Gly Gly Pro Pro Pro Gly Pro Pro
            420                 425                 430

Ala Gln Gln Pro Gly Asp Gln Pro
        435                 440

<210>  15
<211>  9
<212>  PRT
<213>  Artificial Sequence

<220>
<223>  IL-1beta nonapeptide

<400>  15

Val Gln Gly Glu Glu Ser Asn Asp Lys
1               5

<210>  16
<211>  22
<212>  DNA
<213>  Artificial Sequence

<220>
<223>  Primer

<400>  16
cgcggatcca cgttaggcaa ct                                              22

<210>  17
<211>  21
<212>  DNA
<213>  Artificial Sequence

<220>
<223>  Primer

<400>  17
ccgctcgagt tagttttgtt t                                               21

<210>  18
<211>  24
<212>  DNA
<213>  Artificial Sequence

<220>
<223>  Primer

<400>  18
cgcggatcca atccgataac gaat                                            24

<210>  19
<211>  26
<212>  DNA
<213>  Artificial Sequence

<220>
<223>  Primer

<400>  19
ccgctcgagt taatgatatt cgtttt                                            26


<210>  20
<211>  24
<212>  DNA
<213>  Artificial Sequence

<220>
<223>  Primer

<400>  20
cgcggatcca gagttccttt tttt                                              24


<210>  21
<211>  22
<212>  DNA
<213>  Artificial Sequence

<220>
<223>  Primer

<400>  21
ccgctcgagt taaggaacgt tt                                                22


<210>  22
<211>  21
<212>  DNA
<213>  Artificial Sequence

<220>
<223>  Primer

<400>  22
tttggatccg cgattaccgt a                                                 21


<210>  23
<211>  21
<212>  DNA
<213>  Artificial Sequence

<220>
<223>  Primer

<400>  23
cttctcgagt taaaagcact t                                                 21


<210>  24
<211>  23
<212>  DNA
<213>  Artificial Sequence

<220>
<223>  Primer

<400>  24
cgcggatccc ccgacttaaa tgt                                               23

<210>  25
<211>  23
<212>  DNA
<213>  Artificial Sequence

<220>
<223>  Primer

<400>  25
ccgctcgagt caaagtatcg aat                                              23


<210>  26
<211>  26
<212>  DNA
<213>  Artificial Sequence

<220>
<223>  Primer

<400>  26
cgcggatccg tcaataacaa cattgc                                           26


<210>  27
<211>  27
<212>  DNA
<213>  Artificial Sequence

<220>
<223>  Primer

<400>  27
ccgctcgagt tggtttcctt ttacgtt                                          27


<210>  28
<211>  21
<212>  DNA
<213>  Artificial Sequence

<220>
<223>  Primer

<400>  28
cgcggatcca tgttgttaca c                                                21


<210>  29
<211>  25
<212>  DNA
<213>  Artificial Sequence

<220>
<223>  Primer

<400>  29
ccgctcgagc tactgagaaa tcttt                                            25


<210>  30
<211>  21
<212>  DNA

<213>  Artificial Sequence

<220>
<223>  Primer

<400>  30
cgcggatcct ctcccgaatg g                                                21


<210>  31
<211>  24
<212>  DNA
<213>  Artificial Sequence

<220>
<223>  Primer

<400>  31
ccgctcgagt tatctcgtat caaa                                             24


<210>  32
<211>  24
<212>  DNA
<213>  Artificial Sequence

<220>
<223>  Primer

<400>  32
cgcggatcca tagtacgttt aaaa                                             24


<210>  33
<211>  24
<212>  DNA
<213>  Artificial Sequence

<220>
<223>  Primer

<400>  33
ccgctcgagt taaaaactgt ggga                                             24


<210>  34
<211>  23
<212>  DNA
<213>  Artificial Sequence

<220>
<223>  Primer

<400>  34
cgcggatccc aatcaaaatc ggc                                              23


<210>  35
<211>  25
<212>  DNA
<213>  Artificial Sequence

<220>
<223>  Primer

<400>  35
ccgctcgagt taatcctcta aatct                                          25


<210>  36
<211>  21
<212>  DNA
<213>  Artificial Sequence

<220>
<223>  Primer

<400>  36
tcccccgggg ctggcaaaag a                                              21


<210>  37
<211>  23
<212>  DNA
<213>  Artificial Sequence

<220>
<223>  Primer

<400>  37
ccctcgagaa tatccgtatt aga                                            23


<210>  38
<211>  27
<212>  DNA
<213>  Artificial Sequence

<220>
<223>  Primer

<400>  38
aggggaattc tatgaaaaac tttcgat                                        27


<210>  39
<211>  22
<212>  DNA
<213>  Artificial Sequence

<220>
<223>  Primer

<400>  39
ccctcgagct tagtcgcgtc ag                                             22


<210>  40
<211>  23
<212>  DNA
<213>  Artificial Sequence

<220>
<223>  Primer

<400>  40
cgcggatccg atcagatcaa ctt                                            23


<210>  41

<211>  25
<212>  DNA
<213>  Artificial Sequence

<220>
<223>  Primer

<400>  41
ccgctcgagt taattttgtg ttttt                                              25


<210>  42
<211>  21
<212>  DNA
<213>  Artificial Sequence

<220>
<223>  Primer

<400>  42
cgcggatcct gcaaacaaga t                                                  21


<210>  43
<211>  25
<212>  DNA
<213>  Artificial Sequence

<220>
<223>  Primer

<400>  43
cgcctcgagt tattgagaag cgtat                                              25


<210>  44
<211>  22
<212>  DNA
<213>  Artificial Sequence

<220>
<223>  Primer

<400>  44
cgcggatccg aaaaagccgg aa                                                 22


<210>  45
<211>  26
<212>  DNA
<213>  Artificial Sequence

<220>
<223>  Primer

<400>  45
ccgctcgagt tataactttc cgaaat                                             26


<210>  46
<211>  24
<212>  DNA
<213>  Artificial Sequence

<220>

<223>  Primer

<400>  46
cgcggatcct ttccagaaat ttct                                              24


<210>  47
<211>  25
<212>  DNA
<213>  Artificial Sequence

<220>
<223>  Primer

<400>  47
ccgctcgagt taataatatc tttgt                                             25


<210>  48
<211>  21
<212>  DNA
<213>  Artificial Sequence

<220>
<223>  Primer

<400>  48
cgcggatcca ttgcacaaat c                                                 21


<210>  49
<211>  24
<212>  DNA
<213>  Artificial Sequence

<220>
<223>  Primer

<400>  49
ccgctcgagt caaagagcaa actt                                              24


<210>  50
<211>  22
<212>  DNA
<213>  Artificial Sequence

<220>
<223>  Primer

<400>  50
cgcggatccg aagaaagatc ct                                                22


<210>  51
<211>  24
<212>  DNA
<213>  Artificial Sequence

<220>
<223>  Primer

<400>  51
ccgctcgagt catcttttat cttt                                              24

<210>  52
<211>  2064
<212>  DNA
<213>  Leptospira interrogans serovar Pomona

<220>
<221>  CDS
<222>  (1)..(2064)

<400>  52
atg cgt cct ttt tct aaa tta att ttt att ctg gcc ttt tgt att ttt      48
Met Arg Pro Phe Ser Lys Leu Ile Phe Ile Leu Ala Phe Cys Ile Phe
1               5                   10                  15

ttg ccc gtt ttc tct caa cct cta ccg gac ctt ccg gaa aaa caa ttt      96
Leu Pro Val Phe Ser Gln Pro Leu Pro Asp Leu Pro Glu Lys Gln Phe
            20                  25                  30

ggt caa cct ctt aat aca caa aac gac gaa tac aat cct ata gta agc      144
Gly Gln Pro Leu Asn Thr Gln Asn Asp Glu Tyr Asn Pro Ile Val Ser
        35                  40                  45

cca gat gga aga tac atc gta ttc cag tcc aac cgt cca ggc gga gaa      192
Pro Asp Gly Arg Tyr Ile Val Phe Gln Ser Asn Arg Pro Gly Gly Glu
    50                  55                  60

gga gga atg gac att tgg att tcc gag aac att cgc ttt tta gat aag      240
Gly Gly Met Asp Ile Trp Ile Ser Glu Asn Ile Arg Phe Leu Asp Lys
65                  70                  75                  80

gag ata cca gca gaa tgg act aaa ccc gta aat atg aat caa aat att      288
Glu Ile Pro Ala Glu Trp Thr Lys Pro Val Asn Met Asn Gln Asn Ile
                85                  90                  95

tgg gaa gaa tta aaa cgt cca cca gct gct gga gtt cgt aaa cca aat      336
Trp Glu Glu Leu Lys Arg Pro Pro Ala Ala Gly Val Arg Lys Pro Asn
            100                 105                 110

cta ttc aac tct aac gcg ttt gaa ggt gga att tca att ctt ttc gat      384
Leu Phe Asn Ser Asn Ala Phe Glu Gly Gly Ile Ser Ile Leu Phe Asp
        115                 120                 125

tct aac aac gcg cct tcg gaa att tat ttt act tct aca att aac ctt      432
Ser Asn Asn Ala Pro Ser Glu Ile Tyr Phe Thr Ser Thr Ile Asn Leu
    130                 135                 140

gct gtt ggg cgt tcc ggt ttt gaa ggt ttg aat att tat aga aca att      480
Ala Val Gly Arg Ser Gly Phe Glu Gly Leu Asn Ile Tyr Arg Thr Ile
145                 150                 155                 160

aaa gat aaa aaa aca gga aga tgg aca gac cca gaa cat ctc agt gaa      528
Lys Asp Lys Lys Thr Gly Arg Trp Thr Asp Pro Glu His Leu Ser Glu
                165                 170                 175

att aat tcc aac ttc aat gat aag atg ccc gca att tct ccc gat gga      576
Ile Asn Ser Asn Phe Asn Asp Lys Met Pro Ala Ile Ser Pro Asp Gly
            180                 185                 190

aat ttt ttg atc ttc tct tcg gac cgt ccg ggg ggt tac ggt gat ttc      624
Asn Phe Leu Ile Phe Ser Ser Asp Arg Pro Gly Gly Tyr Gly Asp Phe
        195                 200                 205

gat ctt tgg atc tcg gtt cgt aat cca aaa aac gga agt tgg tcc caa      672

Asp Leu Trp Ile Ser Val Arg Asn Pro Lys Asn Gly Ser Trp Ser Gln
    210                 215                 220

cct aaa aat tta ggt tct ccc ctc aac tct tcc gaa agt gaa att ctt      720
Pro Lys Asn Leu Gly Ser Pro Leu Asn Ser Ser Glu Ser Glu Ile Leu
225                 230                 235                 240

cct ttc att cat caa gac gga gaa caa ctt tat ttc agt tct aat cga      768
Pro Phe Ile His Gln Asp Gly Glu Gln Leu Tyr Phe Ser Ser Asn Arg
                245                 250                 255

gaa gac gaa aga aaa aaa ttt aag att ttt aga ata ttc tta aaa tat      816
Glu Asp Glu Arg Lys Lys Phe Lys Ile Phe Arg Ile Phe Leu Lys Tyr
            260                 265                 270

aaa tct gct cta gac aac atg tta gaa gac gaa gaa gaa acc gaa gaa      864
Lys Ser Ala Leu Asp Asn Met Leu Glu Asp Glu Glu Glu Thr Glu Glu
        275                 280                 285

act cct aca acc aaa ccg act gaa att tta att cct aaa att gat caa      912
Thr Pro Thr Thr Lys Pro Thr Glu Ile Leu Ile Pro Lys Ile Asp Gln
    290                 295                 300

tct tct tta tta ctt ctt ccc aaa ccg ttt aac act gat aag tgg gaa      960
Ser Ser Leu Leu Leu Leu Pro Lys Pro Phe Asn Thr Asp Lys Trp Glu
305                 310                 315                 320

ggt ttt gat aac gaa gga atc agt ttt gac aaa gac ggt atc tgg gct     1008
Gly Phe Asp Asn Glu Gly Ile Ser Phe Asp Lys Asp Gly Ile Trp Ala
                325                 330                 335

tat att tct tcc aat cgt tct ggt gga gaa ggt caa ttt gac att ttc     1056
Tyr Ile Ser Ser Asn Arg Ser Gly Gly Glu Gly Gln Phe Asp Ile Phe
            340                 345                 350

cgc ttt caa gtt ccg gaa tct att cgc aac tcc tat act tta aac ttc     1104
Arg Phe Gln Val Pro Glu Ser Ile Arg Asn Ser Tyr Thr Leu Asn Phe
        355                 360                 365

aaa ggt cta gtt ttg gat ggt tcc gaa aag acg atg att gga tta gat     1152
Lys Gly Leu Val Leu Asp Gly Ser Glu Lys Thr Met Ile Gly Leu Asp
    370                 375                 380

tct act tta aaa att tat gat gga act aaa ccg gct aac gta atc act     1200
Ser Thr Leu Lys Ile Tyr Asp Gly Thr Lys Pro Ala Asn Val Ile Thr
385                 390                 395                 400

tcg aaa aga atc gga gga gat ctt acc aaa gga aaa cct tct aat ttt     1248
Ser Lys Arg Ile Gly Gly Asp Leu Thr Lys Gly Lys Pro Ser Asn Phe
                405                 410                 415

gca aca act ctt caa acc gga aag gtt tat aaa ata gaa att agt tct     1296
Ala Thr Thr Leu Gln Thr Gly Lys Val Tyr Lys Ile Glu Ile Ser Ser
            420                 425                 430

cct ggc ttt cat cct caa gag gat att ttg gat tta aga gga aac ata     1344
Pro Gly Phe His Pro Gln Glu Asp Ile Leu Asp Leu Arg Gly Asn Ile
        435                 440                 445

ggt aaa aat cga aaa gtt tat aga acc tac gtt ctt tta cca att caa     1392
Gly Lys Asn Arg Lys Val Tyr Arg Thr Tyr Val Leu Leu Pro Ile Gln
    450                 455                 460

gtt gaa gaa ggt aaa acg gaa gaa aca aaa ata gaa caa ccc ata gag     1440
Val Glu Glu Gly Lys Thr Glu Glu Thr Lys Ile Glu Gln Pro Ile Glu

465                 470                 475                 480

aat caa aaa cca aat tct gcc gcg ttg aaa gtc atc gta gca gac gca       1488
Asn Gln Lys Pro Asn Ser Ala Ala Leu Lys Val Ile Val Ala Asp Ala
                485                 490                 495

tct aca aaa caa atc ata cca gac gct aaa gtt act ctt ttt aca cct       1536
Ser Thr Lys Gln Ile Ile Pro Asp Ala Lys Val Thr Leu Phe Thr Pro
            500                 505                 510

atg aac cgc aaa gga gaa tct ctt gtc caa gat gcg gat aaa aaa tct       1584
Met Asn Arg Lys Gly Glu Ser Leu Val Gln Asp Ala Asp Lys Lys Ser
        515                 520                 525

ttt ctt att aaa aaa tta cca gat aac gat ttt gaa tta ttt gca aca       1632
Phe Leu Ile Lys Lys Leu Pro Asp Asn Asp Phe Glu Leu Phe Ala Thr
    530                 535                 540

gct tcg aaa tat att tct gaa agt atc aat att att caa aaa aat att       1680
Ala Ser Lys Tyr Ile Ser Glu Ser Ile Asn Ile Ile Gln Lys Asn Ile
545                 550                 555                 560

tcc aaa aat gga act gta aca att tat ctg aaa gca gaa agc gac gta       1728
Ser Lys Asn Gly Thr Val Thr Ile Tyr Leu Lys Ala Glu Ser Asp Val
                565                 570                 575

gat ccg gtt tat aat cta cga gtt tat ttc gaa ttt aat aaa aca aaa       1776
Asp Pro Val Tyr Asn Leu Arg Val Tyr Phe Glu Phe Asn Lys Thr Lys
            580                 585                 590

atc aca gaa gag aat aaa aag ttg cta gat cct ctt gta ggc tat ctt       1824
Ile Thr Glu Glu Asn Lys Lys Leu Leu Asp Pro Leu Val Gly Tyr Leu
        595                 600                 605

ttg aaa aat gcg tcc gat aaa att gaa att ggg gga cat acc gac aac       1872
Leu Lys Asn Ala Ser Asp Lys Ile Glu Ile Gly Gly His Thr Asp Asn
    610                 615                 620

gta gcc tct aag gaa tat aac aca aga ttg agt gcc aaa aga gcc cgt       1920
Val Ala Ser Lys Glu Tyr Asn Thr Arg Leu Ser Ala Lys Arg Ala Arg
625                 630                 635                 640

aac gtt tac gag tat ctt ctt tca aaa gga att ccg gag aaa aga atg       1968
Asn Val Tyr Glu Tyr Leu Leu Ser Lys Gly Ile Pro Glu Lys Arg Met
                645                 650                 655

aga atc agg gct tat tgg tat tca caa ccg gat gca gac aat gag aca       2016
Arg Ile Arg Ala Tyr Trp Tyr Ser Gln Pro Asp Ala Asp Asn Glu Thr
            660                 665                 670

gaa acc gga aga gca aaa aat aga aga gtt ggt ttt aga aag ctc tga       2064
Glu Thr Gly Arg Ala Lys Asn Arg Arg Val Gly Phe Arg Lys Leu
        675                 680                 685


<210>  53
<211>  687
<212>  PRT
<213>  Leptospira interrogans serovar Pomona

<400>  53

Met Arg Pro Phe Ser Lys Leu Ile Phe Ile Leu Ala Phe Cys Ile Phe
1               5                   10                  15

Leu Pro Val Phe Ser Gln Pro Leu Pro Asp Leu Pro Glu Lys Gln Phe
            20                  25                  30

Gly Gln Pro Leu Asn Thr Gln Asn Asp Glu Tyr Asn Pro Ile Val Ser
        35                  40                  45

Pro Asp Gly Arg Tyr Ile Val Phe Gln Ser Asn Arg Pro Gly Gly Glu
    50                  55                  60

Gly Gly Met Asp Ile Trp Ile Ser Glu Asn Ile Arg Phe Leu Asp Lys
65                  70                  75                  80

Glu Ile Pro Ala Glu Trp Thr Lys Pro Val Asn Met Asn Gln Asn Ile
                85                  90                  95

Trp Glu Glu Leu Lys Arg Pro Pro Ala Ala Gly Val Arg Lys Pro Asn
            100                 105                 110

Leu Phe Asn Ser Asn Ala Phe Glu Gly Gly Ile Ser Ile Leu Phe Asp
        115                 120                 125

Ser Asn Asn Ala Pro Ser Glu Ile Tyr Phe Thr Ser Thr Ile Asn Leu
    130                 135                 140

Ala Val Gly Arg Ser Gly Phe Glu Gly Leu Asn Ile Tyr Arg Thr Ile
145                 150                 155                 160

Lys Asp Lys Lys Thr Gly Arg Trp Thr Asp Pro Glu His Leu Ser Glu
                165                 170                 175

Ile Asn Ser Asn Phe Asn Asp Lys Met Pro Ala Ile Ser Pro Asp Gly
            180                 185                 190

Asn Phe Leu Ile Phe Ser Ser Asp Arg Pro Gly Gly Tyr Gly Asp Phe
        195                 200                 205

Asp Leu Trp Ile Ser Val Arg Asn Pro Lys Asn Gly Ser Trp Ser Gln
    210                 215                 220

Pro Lys Asn Leu Gly Ser Pro Leu Asn Ser Ser Glu Ser Glu Ile Leu
225                 230                 235                 240

Pro Phe Ile His Gln Asp Gly Glu Gln Leu Tyr Phe Ser Ser Asn Arg
                245                 250                 255

Glu Asp Glu Arg Lys Lys Phe Lys Ile Phe Arg Ile Phe Leu Lys Tyr
            260                 265                 270

Lys Ser Ala Leu Asp Asn Met Leu Glu Asp Glu Glu Glu Thr Glu Glu
        275                 280                 285

Thr Pro Thr Thr Lys Pro Thr Glu Ile Leu Ile Pro Lys Ile Asp Gln
    290                 295                 300

Ser Ser Leu Leu Leu Leu Pro Lys Pro Phe Asn Thr Asp Lys Trp Glu
305                 310                 315                 320

Gly Phe Asp Asn Glu Gly Ile Ser Phe Asp Lys Asp Gly Ile Trp Ala
                325                 330                 335

Tyr Ile Ser Ser Asn Arg Ser Gly Gly Glu Gly Gln Phe Asp Ile Phe
            340                 345                 350

Arg Phe Gln Val Pro Glu Ser Ile Arg Asn Ser Tyr Thr Leu Asn Phe
        355                 360                 365

Lys Gly Leu Val Leu Asp Gly Ser Glu Lys Thr Met Ile Gly Leu Asp
    370                 375                 380

Ser Thr Leu Lys Ile Tyr Asp Gly Thr Lys Pro Ala Asn Val Ile Thr
385                 390                 395                 400

Ser Lys Arg Ile Gly Gly Asp Leu Thr Lys Gly Lys Pro Ser Asn Phe
                405                 410                 415

Ala Thr Thr Leu Gln Thr Gly Lys Val Tyr Lys Ile Glu Ile Ser Ser
            420                 425                 430

Pro Gly Phe His Pro Gln Glu Asp Ile Leu Asp Leu Arg Gly Asn Ile
        435                 440                 445

Gly Lys Asn Arg Lys Val Tyr Arg Thr Tyr Val Leu Leu Pro Ile Gln
    450                 455                 460

Val Glu Glu Gly Lys Thr Glu Glu Thr Lys Ile Glu Gln Pro Ile Glu
465                 470                 475                 480

Asn Gln Lys Pro Asn Ser Ala Ala Leu Lys Val Ile Val Ala Asp Ala
                485                 490                 495

Ser Thr Lys Gln Ile Ile Pro Asp Ala Lys Val Thr Leu Phe Thr Pro
            500                 505                 510

Met Asn Arg Lys Gly Glu Ser Leu Val Gln Asp Ala Asp Lys Lys Ser
        515                 520                 525

Phe Leu Ile Lys Lys Leu Pro Asp Asn Asp Phe Glu Leu Phe Ala Thr

530                 535                 540

Ala Ser Lys Tyr Ile Ser Glu Ser Ile Asn Ile Ile Gln Lys Asn Ile
545                 550                 555                 560

Ser Lys Asn Gly Thr Val Thr Ile Tyr Leu Lys Ala Glu Ser Asp Val
                565                 570                 575

Asp Pro Val Tyr Asn Leu Arg Val Tyr Phe Glu Phe Asn Lys Thr Lys
            580                 585                 590

Ile Thr Glu Glu Asn Lys Lys Leu Leu Asp Pro Leu Val Gly Tyr Leu
        595                 600                 605

Leu Lys Asn Ala Ser Asp Lys Ile Glu Ile Gly Gly His Thr Asp Asn
    610                 615                 620

Val Ala Ser Lys Glu Tyr Asn Thr Arg Leu Ser Ala Lys Arg Ala Arg
625                 630                 635                 640

Asn Val Tyr Glu Tyr Leu Leu Ser Lys Gly Ile Pro Glu Lys Arg Met
                645                 650                 655

Arg Ile Arg Ala Tyr Trp Tyr Ser Gln Pro Asp Ala Asp Asn Glu Thr
            660                 665                 670

Glu Thr Gly Arg Ala Lys Asn Arg Arg Val Gly Phe Arg Lys Leu
        675                 680                 685

<210>  54
<211>  1983
<212>  DNA
<213>  Leptospira interrogans serovar Pomona

<220>
<221>  CDS
<222>  (1)..(1983)

<400>  54
atg cat acc ctg ctg act ctg atc tta agc ctt ttg ctt ttc tct ggt      48
Met His Thr Leu Leu Thr Leu Ile Leu Ser Leu Leu Leu Phe Ser Gly
1               5                   10                  15

ctt cag tct gag aat aaa aat tct tcc tca aaa aaa cta agc gac tct      96
Leu Gln Ser Glu Asn Lys Asn Ser Ser Ser Lys Lys Leu Ser Asp Ser
            20                  25                  30

gct tct tgg att cct aaa gaa aat ttt act caa ctc gca tca caa aga      144
Ala Ser Trp Ile Pro Lys Glu Asn Phe Thr Gln Leu Ala Ser Gln Arg
        35                  40                  45

gaa aaa ttc aaa aac gtt tct cat aac gaa aca tta aaa cta gaa att      192
Glu Lys Phe Lys Asn Val Ser His Asn Glu Thr Leu Lys Leu Glu Ile
    50                  55                  60

ggc tat aaa tta tta act gga aaa att ctt cag ttt gga aaa tta ctt 240
Gly Tyr Lys Leu Leu Thr Gly Lys Ile Leu Gln Phe Gly Lys Leu Leu
65                  70                  75                  80

agt acc gaa aac gca tac gtc ccc atc gaa tcc aac gaa att act tcc 288
Ser Thr Glu Asn Ala Tyr Val Pro Ile Glu Ser Asn Glu Ile Thr Ser
                85                  90                  95

gaa ctt tct caa ctc aac gac aag acc gta aga att tta tgc agt atg 336
Glu Leu Ser Gln Leu Asn Asp Lys Thr Val Arg Ile Leu Cys Ser Met
            100                 105                 110

aaa ggt tcc acc tgc aat cct ata cgt tat gaa att tat ccc ttt tgg 384
Lys Gly Ser Thr Cys Asn Pro Ile Arg Tyr Glu Ile Tyr Pro Phe Trp
        115                 120                 125

tat tcg aaa gaa atc aaa cct tgg acg atc aaa aaa att cct gat tac 432
Tyr Ser Lys Glu Ile Lys Pro Trp Thr Ile Lys Lys Ile Pro Asp Tyr
    130                 135                 140

gta aat cat aat att ttt gca ttc aat cct aca gtt tcc cca gac ggt 480
Val Asn His Asn Ile Phe Ala Phe Asn Pro Thr Val Ser Pro Asp Gly
145                 150                 155                 160

aaa tat ctt ttt tgg acc gct tat gtc aaa cga ggt aaa tcc gga act 528
Lys Tyr Leu Phe Trp Thr Ala Tyr Val Lys Arg Gly Lys Ser Gly Thr
                165                 170                 175

caa aag att tgg tat tcc aaa tta gac gaa aaa ggt ttt tgg gaa gat 576
Gln Lys Ile Trp Tyr Ser Lys Leu Asp Glu Lys Gly Phe Trp Glu Asp
            180                 185                 190

ggt aag gaa atg aac gct cct tta aat aat gaa atg cct tcc gct gtt 624
Gly Lys Glu Met Asn Ala Pro Leu Asn Asn Glu Met Pro Ser Ala Val
        195                 200                 205

atc tct gca ctt cct ggt ggt aac gaa ctt ttc gtc ttt gga acg ttt 672
Ile Ser Ala Leu Pro Gly Gly Asn Glu Leu Phe Val Phe Gly Thr Phe
    210                 215                 220

ggc gaa aaa gaa ctt tta gac gaa ctc agt aaa gat ttt gaa acg aag 720
Gly Glu Lys Glu Leu Leu Asp Glu Leu Ser Lys Asp Phe Glu Thr Lys
225                 230                 235                 240

gca gac ttg gcc gct cgt tct tct aaa aat tca aac gta tat aga aaa 768
Ala Asp Leu Ala Ala Arg Ser Ser Lys Asn Ser Asn Val Tyr Arg Lys
                245                 250                 255

aag ata gaa gaa ctt aga gca gaa tat gac gaa aaa acg aaa caa att 816
Lys Ile Glu Glu Leu Arg Ala Glu Tyr Asp Glu Lys Thr Lys Gln Ile
            260                 265                 270

tca agc aga gtt cct ctt tat aaa agt ttt aag gaa aaa gat tcc tgg 864
Ser Ser Arg Val Pro Leu Tyr Lys Ser Phe Lys Glu Lys Asp Ser Trp
        275                 280                 285

tct aaa cca agc ata tta aac ttt cct aat ttt tat aat ctt tat aga 912
Ser Lys Pro Ser Ile Leu Asn Phe Pro Asn Phe Tyr Asn Leu Tyr Arg
    290                 295                 300

aag aga aac gat tca agc caa gaa att ttc ggt gga tct act ctt tct 960
Lys Arg Asn Asp Ser Ser Gln Glu Ile Phe Gly Gly Ser Thr Leu Ser
305                 310                 315                 320

tct tca ggg aga att tta atc tat tct tct caa cat aag gat tcc aaa 1008
Ser Ser Gly Arg Ile Leu Ile Tyr Ser Ser Gln His Lys Asp Ser Lys
                325                 330                 335

gga aaa ttg gat ctt tat gtt agc aaa atg tta aac gac gga acc ttt 1056
Gly Lys Leu Asp Leu Tyr Val Ser Lys Met Leu Asn Asp Gly Thr Phe
            340                 345                 350

cct tta ggt aca aac tta gga gat gtg atc aac aca act cat gaa gag 1104
Pro Leu Gly Thr Asn Leu Gly Asp Val Ile Asn Thr Thr His Glu Glu
        355                 360                 365

atg gct cct ttt tta gca agc gat gat aga acc ctt tat ttt tct agc 1152
Met Ala Pro Phe Leu Ala Ser Asp Asp Arg Thr Leu Tyr Phe Ser Ser
    370                 375                 380

gac ggt cat aaa ggg ctc tct att tat atg aca aaa aga atc gga gaa 1200
Asp Gly His Lys Gly Leu Ser Ile Tyr Met Thr Lys Arg Ile Gly Glu
385                 390                 395                 400

ggt tgg gat caa tgg acc aaa cct gtc gag gtt tcc gaa aat cta aaa 1248
Gly Trp Asp Gln Trp Thr Lys Pro Val Glu Val Ser Glu Asn Leu Lys
                405                 410                 415

ggt gta aat ttt ttt tca att ccc gcg aat agc gac tgg gcc tat ata 1296
Gly Val Asn Phe Phe Ser Ile Pro Ala Asn Ser Asp Trp Ala Tyr Ile
            420                 425                 430

agc aaa gac ggc cga ttg ttt atg gct tat ctt cct aaa gaa atg cgc 1344
Ser Lys Asp Gly Arg Leu Phe Met Ala Tyr Leu Pro Lys Glu Met Arg
        435                 440                 445

cca gag aag gtc gtg atc ata aac gga aaa gtt tta gat acg gat ggt 1392
Pro Glu Lys Val Val Ile Ile Asn Gly Lys Val Leu Asp Thr Asp Gly
    450                 455                 460

aac cct ctt tct gct gac ata cat tat gaa tct tta aag tct cac gaa 1440
Asn Pro Leu Ser Ala Asp Ile His Tyr Glu Ser Leu Lys Ser His Glu
465                 470                 475                 480

aag atc ggt agc gcc aaa agc gat cct tct aat ggt aat ttt tcg atc 1488
Lys Ile Gly Ser Ala Lys Ser Asp Pro Ser Asn Gly Asn Phe Ser Ile
                485                 490                 495

att ctc ccc ttc ggc gaa aac tac ggt ttt tac gcc cag aaa aaa ggt 1536
Ile Leu Pro Phe Gly Glu Asn Tyr Gly Phe Tyr Ala Gln Lys Lys Gly
            500                 505                 510

tat ctt cca gta tca caa aat ttg aat cta agt tct aaa aag aaa ttc 1584
Tyr Leu Pro Val Ser Gln Asn Leu Asn Leu Ser Ser Lys Lys Lys Phe
        515                 520                 525

tct gaa aaa gtg gaa gta att tta caa ctt cct ccg atc cga gaa aga 1632
Ser Glu Lys Val Glu Val Ile Leu Gln Leu Pro Pro Ile Arg Glu Arg
    530                 535                 540

ggt tcc att caa atc aat aat tta ttt ttt gag tcc aag agt ttt caa 1680
Gly Ser Ile Gln Ile Asn Asn Leu Phe Phe Glu Ser Lys Ser Phe Gln
545                 550                 555                 560

atc gct ccg gaa tcc gcc cca gaa ctg gat cgt ctc gcg gaa atc gta 1728
Ile Ala Pro Glu Ser Ala Pro Glu Leu Asp Arg Leu Ala Glu Ile Val
                565                 570                 575

aag gaa aat cca gat att gaa att cag att gaa ggt cat acc gac aac 1776

Lys Glu Asn Pro Asp Ile Glu Ile Gln Ile Glu Gly His Thr Asp Asn
            580                 585                 590

att ggc aag aaa aaa gac aat cta att ctt tcc gaa aaa aga gcg gca      1824
Ile Gly Lys Lys Lys Asp Asn Leu Ile Leu Ser Glu Lys Arg Ala Ala
        595                 600                 605

gcg gtc gca gaa tat ctt ttt caa aaa cat tct att tct aag act aga      1872
Ala Val Ala Glu Tyr Leu Phe Gln Lys His Ser Ile Ser Lys Thr Arg
    610                 615                 620

atc caa acc aaa ggt ttt ggg gac agt gtt cct ttg agt aaa aat gat      1920
Ile Gln Thr Lys Gly Phe Gly Asp Ser Val Pro Leu Ser Lys Asn Asp
625                 630                 635                 640

tcc gaa gaa gca cgc aaa aaa aac aga agg gtc aat ttt acg att ttg      1968
Ser Glu Glu Ala Arg Lys Lys Asn Arg Arg Val Asn Phe Thr Ile Leu
                645                 650                 655

aaa aaa agt aaa taa                                                  1983
Lys Lys Ser Lys
            660


<210>  55
<211>  660
<212>  PRT
<213>  Leptospira interrogans serovar Pomona

<400>  55

Met His Thr Leu Leu Thr Leu Ile Leu Ser Leu Leu Leu Phe Ser Gly
1               5                   10                  15


Leu Gln Ser Glu Asn Lys Asn Ser Ser Ser Lys Lys Leu Ser Asp Ser
            20                  25                  30


Ala Ser Trp Ile Pro Lys Glu Asn Phe Thr Gln Leu Ala Ser Gln Arg
        35                  40                  45


Glu Lys Phe Lys Asn Val Ser His Asn Glu Thr Leu Lys Leu Glu Ile
    50                  55                  60


Gly Tyr Lys Leu Leu Thr Gly Lys Ile Leu Gln Phe Gly Lys Leu Leu
65                  70                  75                  80


Ser Thr Glu Asn Ala Tyr Val Pro Ile Glu Ser Asn Glu Ile Thr Ser
                85                  90                  95


Glu Leu Ser Gln Leu Asn Asp Lys Thr Val Arg Ile Leu Cys Ser Met
            100                 105                 110


Lys Gly Ser Thr Cys Asn Pro Ile Arg Tyr Glu Ile Tyr Pro Phe Trp
        115                 120                 125


Tyr Ser Lys Glu Ile Lys Pro Trp Thr Ile Lys Lys Ile Pro Asp Tyr
    130                 135                 140

Val Asn His Asn Ile Phe Ala Phe Asn Pro Thr Val Ser Pro Asp Gly
145                 150                 155                 160

Lys Tyr Leu Phe Trp Thr Ala Tyr Val Lys Arg Gly Lys Ser Gly Thr
                165                 170                 175

Gln Lys Ile Trp Tyr Ser Lys Leu Asp Glu Lys Gly Phe Trp Glu Asp
            180                 185                 190

Gly Lys Glu Met Asn Ala Pro Leu Asn Asn Glu Met Pro Ser Ala Val
        195                 200                 205

Ile Ser Ala Leu Pro Gly Gly Asn Glu Leu Phe Val Phe Gly Thr Phe
    210                 215                 220

Gly Glu Lys Glu Leu Leu Asp Glu Leu Ser Lys Asp Phe Glu Thr Lys
225                 230                 235                 240

Ala Asp Leu Ala Ala Arg Ser Ser Lys Asn Ser Asn Val Tyr Arg Lys
                245                 250                 255

Lys Ile Glu Glu Leu Arg Ala Glu Tyr Asp Glu Lys Thr Lys Gln Ile
            260                 265                 270

Ser Ser Arg Val Pro Leu Tyr Lys Ser Phe Lys Glu Lys Asp Ser Trp
        275                 280                 285

Ser Lys Pro Ser Ile Leu Asn Phe Pro Asn Phe Tyr Asn Leu Tyr Arg
    290                 295                 300

Lys Arg Asn Asp Ser Ser Gln Glu Ile Phe Gly Gly Ser Thr Leu Ser
305                 310                 315                 320

Ser Ser Gly Arg Ile Leu Ile Tyr Ser Ser Gln His Lys Asp Ser Lys
                325                 330                 335

Gly Lys Leu Asp Leu Tyr Val Ser Lys Met Leu Asn Asp Gly Thr Phe
            340                 345                 350

Pro Leu Gly Thr Asn Leu Gly Asp Val Ile Asn Thr Thr His Glu Glu
        355                 360                 365

Met Ala Pro Phe Leu Ala Ser Asp Asp Arg Thr Leu Tyr Phe Ser Ser
    370                 375                 380

Asp Gly His Lys Gly Leu Ser Ile Tyr Met Thr Lys Arg Ile Gly Glu
385                 390                 395                 400

Gly Trp Asp Gln Trp Thr Lys Pro Val Glu Val Ser Glu Asn Leu Lys
                405                 410                 415

Gly Val Asn Phe Phe Ser Ile Pro Ala Asn Ser Asp Trp Ala Tyr Ile
            420                 425                 430

Ser Lys Asp Gly Arg Leu Phe Met Ala Tyr Leu Pro Lys Glu Met Arg
        435                 440                 445

Pro Glu Lys Val Val Ile Ile Asn Gly Lys Val Leu Asp Thr Asp Gly
    450                 455                 460

Asn Pro Leu Ser Ala Asp Ile His Tyr Glu Ser Leu Lys Ser His Glu
465                 470                 475                 480

Lys Ile Gly Ser Ala Lys Ser Asp Pro Ser Asn Gly Asn Phe Ser Ile
                485                 490                 495

Ile Leu Pro Phe Gly Glu Asn Tyr Gly Phe Tyr Ala Gln Lys Lys Gly
            500                 505                 510

Tyr Leu Pro Val Ser Gln Asn Leu Asn Leu Ser Ser Lys Lys Lys Phe
        515                 520                 525

Ser Glu Lys Val Glu Val Ile Leu Gln Leu Pro Pro Ile Arg Glu Arg
    530                 535                 540

Gly Ser Ile Gln Ile Asn Asn Leu Phe Phe Glu Ser Lys Ser Phe Gln
545                 550                 555                 560

Ile Ala Pro Glu Ser Ala Pro Glu Leu Asp Arg Leu Ala Glu Ile Val
                565                 570                 575

Lys Glu Asn Pro Asp Ile Glu Ile Gln Ile Glu Gly His Thr Asp Asn
            580                 585                 590

Ile Gly Lys Lys Lys Asp Asn Leu Ile Leu Ser Glu Lys Arg Ala Ala
        595                 600                 605

Ala Val Ala Glu Tyr Leu Phe Gln Lys His Ser Ile Ser Lys Thr Arg
    610                 615                 620

Ile Gln Thr Lys Gly Phe Gly Asp Ser Val Pro Leu Ser Lys Asn Asp
625                 630                 635                 640

Ser Glu Glu Ala Arg Lys Lys Asn Arg Arg Val Asn Phe Thr Ile Leu
                645                 650                 655

Lys Lys Ser Lys
            660

<210>  56
<211>  1428
<212>  DNA
<213>  Leptospira interrogans serovar Pomona

<220>
<221>  CDS
<222>  (1)..(1428)

<400>  56
ttg gaa agt ttt tta cac ttt cct tat gtt tta aag gaa tct tac gaa          48
Met Glu Ser Phe Leu His Phe Pro Tyr Val Leu Lys Glu Ser Tyr Glu
1               5                   10                  15

act aaa act atg ggg aaa ttt tcc aat caa cct tca aaa tcc aaa atc          96
Thr Lys Thr Met Gly Lys Phe Ser Asn Gln Pro Ser Lys Ser Lys Ile
            20                  25                  30

ata aac aga cgt tta cga aga att gta tca tac gtt ttt att att tct         144
Ile Asn Arg Arg Leu Arg Arg Ile Val Ser Tyr Val Phe Ile Ile Ser
        35                  40                  45

aca ttg atc ggt ttt act tct act ttt cca gaa gaa tcg gac aaa gtt         192
Thr Leu Ile Gly Phe Thr Ser Thr Phe Pro Glu Glu Ser Asp Lys Val
    50                  55                  60

ctc ttt cgt tgg aaa cta aaa cca gga gag aat gta gaa tta aac gaa         240
Leu Phe Arg Trp Lys Leu Lys Pro Gly Glu Asn Val Glu Leu Asn Glu
65                  70                  75                  80

tat cat cga gta caa tta gta agt atg gga aag aaa att aga aga gaa         288
Tyr His Arg Val Gln Leu Val Ser Met Gly Lys Lys Ile Arg Arg Glu
                85                  90                  95

gat aaa aat cga atc cta tta caa acc ctt tcc tgc gaa aat aaa gaa         336
Asp Lys Asn Arg Ile Leu Leu Gln Thr Leu Ser Cys Glu Asn Lys Glu
            100                 105                 110

tgt acg tta gaa gga ttt ttt gat act tac act cgt ttt ccc gaa gtt         384
Cys Thr Leu Glu Gly Phe Phe Asp Thr Tyr Thr Arg Phe Pro Glu Val
        115                 120                 125

gat cct gcg ttt cgt aaa gat aaa acg ttt aag agc aga ttt caa ata         432
Asp Pro Ala Phe Arg Lys Asp Lys Thr Phe Lys Ser Arg Phe Gln Ile
    130                 135                 140

aca gat tta ggt caa tat aaa gtg cca cag gaa tac agt atg cct aat         480
Thr Asp Leu Gly Gln Tyr Lys Val Pro Gln Glu Tyr Ser Met Pro Asn
145                 150                 155                 160

ctc cgc tct ctt cct agt ttt tcc gaa aaa cca att tcc att gga gaa         528
Leu Arg Ser Leu Pro Ser Phe Ser Glu Lys Pro Ile Ser Ile Gly Glu
                165                 170                 175

gaa tgg aca caa ccc gct acg gaa agt ttt caa ttt cca gga gga agg         576
Glu Trp Thr Gln Pro Ala Thr Glu Ser Phe Gln Phe Pro Gly Gly Arg
            180                 185                 190

gtt atg atc acg gtt ttt gcg aaa tat aag tat cac ggt gta gac gaa         624
Val Met Ile Thr Val Phe Ala Lys Tyr Lys Tyr His Gly Val Asp Glu

195                 200                 205

tgg gaa tat caa aag ttg tcc ggt aaa gga gat cgt atc gaa tac aat       672
Trp Glu Tyr Gln Lys Leu Ser Gly Lys Gly Asp Arg Ile Glu Tyr Asn
    210                 215                 220

tat aat tta tat tat gat tct caa atg aac aga acc ggg gtt cca ttt       720
Tyr Asn Leu Tyr Tyr Asp Ser Gln Met Asn Arg Thr Gly Val Pro Phe
225                 230                 235                 240

aag atc tac gga ttt gcg cga gga atg gta ttt ttt gat cgc gaa ctt       768
Lys Ile Tyr Gly Phe Ala Arg Gly Met Val Phe Phe Asp Arg Glu Leu
                245                 250                 255

gga att cca caa tat aaa agg gtt cag ttg tcg tac act ttt gtc tat       816
Gly Ile Pro Gln Tyr Lys Arg Val Gln Leu Ser Tyr Thr Phe Val Tyr
            260                 265                 270

gaa aat gga atg gcg caa gag atg tcg ttc gac att cac gga gta tat       864
Glu Asn Gly Met Ala Gln Glu Met Ser Phe Asp Ile His Gly Val Tyr
        275                 280                 285

aat aaa aac gta aaa ctt acg gat caa gac aag gac aag ttt gcg gaa       912
Asn Lys Asn Val Lys Leu Thr Asp Gln Asp Lys Asp Lys Phe Ala Glu
    290                 295                 300

gaa atc cgt aaa att tta gga gga gaa ctt cca act gga att gaa cca       960
Glu Ile Arg Lys Ile Leu Gly Gly Glu Leu Pro Thr Gly Ile Glu Pro
305                 310                 315                 320

gat tcc gat tca aaa aag aat tta aaa aaa ccg aaa aag gaa acc att      1008
Asp Ser Asp Ser Lys Lys Asn Leu Lys Lys Pro Lys Lys Glu Thr Ile
                325                 330                 335

tca tgg ccg gaa gaa gag gat aat caa gcc gca cca gaa agt tcg ccg      1056
Ser Trp Pro Glu Glu Glu Asp Asn Gln Ala Ala Pro Glu Ser Ser Pro
            340                 345                 350

gta gaa att aga aga aca gaa gaa gga atc gct att tct tta aat tca      1104
Val Glu Ile Arg Arg Thr Glu Glu Gly Ile Ala Ile Ser Leu Asn Ser
        355                 360                 365

gtg ttg ttt gat cac aac agc tca gaa ctt aaa caa gaa gca aaa tta      1152
Val Leu Phe Asp His Asn Ser Ser Glu Leu Lys Gln Glu Ala Lys Leu
    370                 375                 380

gaa tta aaa aga atc gca tcc gta ttg aaa aaa tat gca gat aga gaa      1200
Glu Leu Lys Arg Ile Ala Ser Val Leu Lys Lys Tyr Ala Asp Arg Glu
385                 390                 395                 400

atc aga att agc ggg cat act gac aat tca ggc ggg gaa gaa tat aat      1248
Ile Arg Ile Ser Gly His Thr Asp Asn Ser Gly Gly Glu Glu Tyr Asn
                405                 410                 415

cga aaa ctt tcc aga gaa aga gca ctt tcc gtt ttg aag gaa ctc aga      1296
Arg Lys Leu Ser Arg Glu Arg Ala Leu Ser Val Leu Lys Glu Leu Arg
            420                 425                 430

gat gaa caa ggt ttg gaa gaa aaa aga atg tct tac gaa gga tat ggg      1344
Asp Glu Gln Gly Leu Glu Glu Lys Arg Met Ser Tyr Glu Gly Tyr Gly
        435                 440                 445

aaa tca aaa ccg att gca gat aac tct acg att caa ggt agg caa aaa      1392
Lys Ser Lys Pro Ile Ala Asp Asn Ser Thr Ile Gln Gly Arg Gln Lys
    450                 455                 460

aat cgt agg gta gat att act att gta atg gaa taa 1428
Asn Arg Arg Val Asp Ile Thr Ile Val Met Glu
465                 470                 475

<210> 57
<211> 475
<212> PRT
<213> Leptospira interrogans serovar Pomona

<400> 57

Met Glu Ser Phe Leu His Phe Pro Tyr Val Leu Lys Glu Ser Tyr Glu
1               5                   10                  15

Thr Lys Thr Met Gly Lys Phe Ser Asn Gln Pro Ser Lys Ser Lys Ile
            20                  25                  30

Ile Asn Arg Arg Leu Arg Arg Ile Val Ser Tyr Val Phe Ile Ile Ser
        35                  40                  45

Thr Leu Ile Gly Phe Thr Ser Thr Phe Pro Glu Glu Ser Asp Lys Val
    50                  55                  60

Leu Phe Arg Trp Lys Leu Lys Pro Gly Glu Asn Val Glu Leu Asn Glu
65                  70                  75                  80

Tyr His Arg Val Gln Leu Val Ser Met Gly Lys Lys Ile Arg Arg Glu
                85                  90                  95

Asp Lys Asn Arg Ile Leu Leu Gln Thr Leu Ser Cys Glu Asn Lys Glu
            100                 105                 110

Cys Thr Leu Glu Gly Phe Phe Asp Thr Tyr Thr Arg Phe Pro Glu Val
        115                 120                 125

Asp Pro Ala Phe Arg Lys Asp Lys Thr Phe Lys Ser Arg Phe Gln Ile
    130                 135                 140

Thr Asp Leu Gly Gln Tyr Lys Val Pro Gln Glu Tyr Ser Met Pro Asn
145                 150                 155                 160

Leu Arg Ser Leu Pro Ser Phe Ser Glu Lys Pro Ile Ser Ile Gly Glu
                165                 170                 175

Glu Trp Thr Gln Pro Ala Thr Glu Ser Phe Gln Phe Pro Gly Gly Arg
            180                 185                 190

Val Met Ile Thr Val Phe Ala Lys Tyr Lys Tyr His Gly Val Asp Glu
        195                 200                 205

Trp Glu Tyr Gln Lys Leu Ser Gly Lys Gly Asp Arg Ile Glu Tyr Asn
    210                 215                 220

Tyr Asn Leu Tyr Tyr Asp Ser Gln Met Asn Arg Thr Gly Val Pro Phe
225                 230                 235                 240

Lys Ile Tyr Gly Phe Ala Arg Gly Met Val Phe Phe Asp Arg Glu Leu
                245                 250                 255

Gly Ile Pro Gln Tyr Lys Arg Val Gln Leu Ser Tyr Thr Phe Val Tyr
            260                 265                 270

Glu Asn Gly Met Ala Gln Glu Met Ser Phe Asp Ile His Gly Val Tyr
        275                 280                 285

Asn Lys Asn Val Lys Leu Thr Asp Gln Asp Lys Asp Lys Phe Ala Glu
    290                 295                 300

Glu Ile Arg Lys Ile Leu Gly Gly Glu Leu Pro Thr Gly Ile Glu Pro
305                 310                 315                 320

Asp Ser Asp Ser Lys Lys Asn Leu Lys Lys Pro Lys Lys Glu Thr Ile
                325                 330                 335

Ser Trp Pro Glu Glu Glu Asp Asn Gln Ala Ala Pro Glu Ser Ser Pro
            340                 345                 350

Val Glu Ile Arg Arg Thr Glu Glu Gly Ile Ala Ile Ser Leu Asn Ser
        355                 360                 365

Val Leu Phe Asp His Asn Ser Ser Glu Leu Lys Gln Glu Ala Lys Leu
    370                 375                 380

Glu Leu Lys Arg Ile Ala Ser Val Leu Lys Lys Tyr Ala Asp Arg Glu
385                 390                 395                 400

Ile Arg Ile Ser Gly His Thr Asp Asn Ser Gly Gly Glu Glu Tyr Asn
                405                 410                 415

Arg Lys Leu Ser Arg Glu Arg Ala Leu Ser Val Leu Lys Glu Leu Arg
            420                 425                 430

Asp Glu Gln Gly Leu Glu Glu Lys Arg Met Ser Tyr Glu Gly Tyr Gly
        435                 440                 445

Lys Ser Lys Pro Ile Ala Asp Asn Ser Thr Ile Gln Gly Arg Gln Lys
    450                 455                 460

Asn Arg Arg Val Asp Ile Thr Ile Val Met Glu

465                 470                 475

<210>  58
<211>  1683
<212>  DNA
<213>  Leptospira interrogans serovar Pomona

<220>
<221>  CDS
<222>  (1)..(1683)

<400>  58
atg aaa caa ttt tta ttt tta ctg tgt gta ttt cta ctc ttt caa aat       48
Met Lys Gln Phe Leu Phe Leu Leu Cys Val Phe Leu Leu Phe Gln Asn
1               5                   10                  15

tgt tct gtc aag ccc acg gaa aat ccg tgc gat acg agt act tct ctt       96
Cys Ser Val Lys Pro Thr Glu Asn Pro Cys Asp Thr Ser Thr Ser Leu
            20                  25                  30

ttt tat aaa tct ttg ttc tta aat ttt att tta act tcc ggc aaa acg      144
Phe Tyr Lys Ser Leu Phe Leu Asn Phe Ile Leu Thr Ser Gly Lys Thr
        35                  40                  45

agt att tgc ggt atg cac acg tcg gtt cag ttg cct ccc cct acg att      192
Ser Ile Cys Gly Met His Thr Ser Val Gln Leu Pro Pro Pro Thr Ile
    50                  55                  60

ctc aac ata aaa tca aaa agt act ttg aat aca gga ttt tta att ggt      240
Leu Asn Ile Lys Ser Lys Ser Thr Leu Asn Thr Gly Phe Leu Ile Gly
65                  70                  75                  80

gaa atg gat gag tct gct tcc gga gtt caa att tct tta gat tcg ggt      288
Glu Met Asp Glu Ser Ala Ser Gly Val Gln Ile Ser Leu Asp Ser Gly
                85                  90                  95

cca ttt atg gac gct caa act tct ggc aat cag tgg aaa ttt caa ctt      336
Pro Phe Met Asp Ala Gln Thr Ser Gly Asn Gln Trp Lys Phe Gln Leu
            100                 105                 110

cct gcc gca ggt gtt tct act aca att cct tca agt gga att tgg aga      384
Pro Ala Ala Gly Val Ser Thr Thr Ile Pro Ser Ser Gly Ile Trp Arg
        115                 120                 125

gac tgg agt tta cat aca att tcg gtt cga tct act tct aag gaa tca      432
Asp Trp Ser Leu His Thr Ile Ser Val Arg Ser Thr Ser Lys Glu Ser
    130                 135                 140

aat tct att ccg att aca atc act gtt caa aaa ggt tca aac aag gat      480
Asn Ser Ile Pro Ile Thr Ile Thr Val Gln Lys Gly Ser Asn Lys Asp
145                 150                 155                 160

att aac ggc gac ggt tat cca gat gca ttg att ggt tct caa gct gca      528
Ile Asn Gly Asp Gly Tyr Pro Asp Ala Leu Ile Gly Ser Gln Ala Ala
                165                 170                 175

aat cga gtt agg gct tat ctt tct ctt ggg aaa gca aag ggt tta gat      576
Asn Arg Val Arg Ala Tyr Leu Ser Leu Gly Lys Ala Lys Gly Leu Asp
            180                 185                 190

ttg gtt ccg gtc act tta cta aat ggg gca ggt gga ttt ggt tat tcc      624
Leu Val Pro Val Thr Leu Leu Asn Gly Ala Gly Gly Phe Gly Tyr Ser
        195                 200                 205

gtc aag tta ggc gac atc gat gga gat gga tac gcc gat gca gtt gta 672
Val Lys Leu Gly Asp Ile Asp Gly Asp Gly Tyr Ala Asp Ala Val Val
    210                 215                 220

gga agt gca aca aat aca ttc gct att tat tta gga tca atc ggt ggg 720
Gly Ser Ala Thr Asn Thr Phe Ala Ile Tyr Leu Gly Ser Ile Gly Gly
225                 230                 235                 240

ctt tcg aca acg gcg att aat tat atc cct att ggt gtt ggt ggt tta 768
Leu Ser Thr Thr Ala Ile Asn Tyr Ile Pro Ile Gly Val Gly Gly Leu
                245                 250                 255

ttg aat gtg gat gta gga gat ata aac gga gac gga ttt tca gac gtt 816
Leu Asn Val Asp Val Gly Asp Ile Asn Gly Asp Gly Phe Ser Asp Val
            260                 265                 270

ttg att ggt gta cct tat gac gta gga aat att ggg cgt gtt tat tct 864
Leu Ile Gly Val Pro Tyr Asp Val Gly Asn Ile Gly Arg Val Tyr Ser
        275                 280                 285

tat ttt tca aat gga gtt atg ggg caa ggt gtt acg ttt ggt caa caa 912
Tyr Phe Ser Asn Gly Val Met Gly Gln Gly Val Thr Phe Gly Gln Gln
    290                 295                 300

ttg aac aat cct gga aat gca ggt agt gct gca ttt tac gga tat gcc 960
Leu Asn Asn Pro Gly Asn Ala Gly Ser Ala Ala Phe Tyr Gly Tyr Ala
305                 310                 315                 320

att acg tta ggt gat att aat gga gat ggt aaa tcg gat gcg att ata 1008
Ile Thr Leu Gly Asp Ile Asn Gly Asp Gly Lys Ser Asp Ala Ile Ile
                325                 330                 335

gga gcg gtc ggt tct gga cag gtc ggc gct tct ttt att tat ctt gcg 1056
Gly Ala Val Gly Ser Gly Gln Val Gly Ala Ser Phe Ile Tyr Leu Ala
            340                 345                 350

caa gca gca ggg gct ttt gct gct tat tct caa aca att acg gga tcg 1104
Gln Ala Ala Gly Ala Phe Ala Ala Tyr Ser Gln Thr Ile Thr Gly Ser
        355                 360                 365

gct gca aat gaa tgg tat gca aat tca gca att gct acc gat ata aac 1152
Ala Ala Asn Glu Trp Tyr Ala Asn Ser Ala Ile Ala Thr Asp Ile Asn
    370                 375                 380

cgg gat gga ttt gcg gat tta ttt gtg ggg gct tat caa gaa tcg ggc 1200
Arg Asp Gly Phe Ala Asp Leu Phe Val Gly Ala Tyr Gln Glu Ser Gly
385                 390                 395                 400

ggc gtt gga aga gta cat tta tat tta tct aat tta gga att ctt gtg 1248
Gly Val Gly Arg Val His Leu Tyr Leu Ser Asn Leu Gly Ile Leu Val
                405                 410                 415

aat acc gcc aac ggt cca att tca gga ttg gtt ggg tca caa act gga 1296
Asn Thr Ala Asn Gly Pro Ile Ser Gly Leu Val Gly Ser Gln Thr Gly
            420                 425                 430

act tct gtg gca act gga gat gta aac ggt gat gga ttt tta gat ctt 1344
Thr Ser Val Ala Thr Gly Asp Val Asn Gly Asp Gly Phe Leu Asp Leu
        435                 440                 445

ctt acc gga ggt tat tct tac act tct acg ttt cca aat caa ggt tat 1392
Leu Thr Gly Gly Tyr Ser Tyr Thr Ser Thr Phe Pro Asn Gln Gly Tyr
    450                 455                 460

gcg att act cat tta act acg ggt gat aca atg ggt tta act gtg aac 1440
Ala Ile Thr His Leu Thr Thr Gly Asp Thr Met Gly Leu Thr Val Asn
465                 470                 475                 480

cct tta aat ctt cta acg gtt cca gtg aat tta gga gat atg gga aat 1488
Pro Leu Asn Leu Leu Thr Val Pro Val Asn Leu Gly Asp Met Gly Asn
                485                 490                 495

tca att gct tcg gta gat att aac ggt gac ggt tta agt gac gtt ctt 1536
Ser Ile Ala Ser Val Asp Ile Asn Gly Asp Gly Leu Ser Asp Val Leu
            500                 505                 510

gtg gga gct cct agt tcc gta ggt gga aca aat gtt gga aac gtt tac 1584
Val Gly Ala Pro Ser Ser Val Gly Gly Thr Asn Val Gly Asn Val Tyr
        515                 520                 525

ctt tat ata tca aac gga ttg gac gga tat act tcg gcc cca caa att 1632
Leu Tyr Ile Ser Asn Gly Leu Asp Gly Tyr Thr Ser Ala Pro Gln Ile
    530                 535                 540

ttc gtc gaa cca gat gta aac gga acg ttt ggg act tcc gtg gat tta 1680
Phe Val Glu Pro Asp Val Asn Gly Thr Phe Gly Thr Ser Val Asp Leu
545                 550                 555                 560

tga 1683

<210> 59
<211> 560
<212> PRT
<213> Leptospira interrogans serovar Pomona

<400> 59

Met Lys Gln Phe Leu Phe Leu Leu Cys Val Phe Leu Leu Phe Gln Asn
1               5                   10                  15

Cys Ser Val Lys Pro Thr Glu Asn Pro Cys Asp Thr Ser Thr Ser Leu
            20                  25                  30

Phe Tyr Lys Ser Leu Phe Leu Asn Phe Ile Leu Thr Ser Gly Lys Thr
        35                  40                  45

Ser Ile Cys Gly Met His Thr Ser Val Gln Leu Pro Pro Pro Thr Ile
    50                  55                  60

Leu Asn Ile Lys Ser Lys Ser Thr Leu Asn Thr Gly Phe Leu Ile Gly
65                  70                  75                  80

Glu Met Asp Glu Ser Ala Ser Gly Val Gln Ile Ser Leu Asp Ser Gly
                85                  90                  95

Pro Phe Met Asp Ala Gln Thr Ser Gly Asn Gln Trp Lys Phe Gln Leu
            100                 105                 110

Pro Ala Ala Gly Val Ser Thr Thr Ile Pro Ser Ser Gly Ile Trp Arg
        115                 120                 125

Asp Trp Ser Leu His Thr Ile Ser Val Arg Ser Thr Ser Lys Glu Ser
    130                 135                 140

Asn Ser Ile Pro Ile Thr Ile Thr Val Gln Lys Gly Ser Asn Lys Asp
145                 150                 155                 160

Ile Asn Gly Asp Gly Tyr Pro Asp Ala Leu Ile Gly Ser Gln Ala Ala
                165                 170                 175

Asn Arg Val Arg Ala Tyr Leu Ser Leu Gly Lys Ala Lys Gly Leu Asp
            180                 185                 190

Leu Val Pro Val Thr Leu Leu Asn Gly Ala Gly Gly Phe Gly Tyr Ser
        195                 200                 205

Val Lys Leu Gly Asp Ile Asp Gly Asp Gly Tyr Ala Asp Ala Val Val
    210                 215                 220

Gly Ser Ala Thr Asn Thr Phe Ala Ile Tyr Leu Gly Ser Ile Gly Gly
225                 230                 235                 240

Leu Ser Thr Thr Ala Ile Asn Tyr Ile Pro Ile Gly Val Gly Gly Leu
                245                 250                 255

Leu Asn Val Asp Val Gly Asp Ile Asn Gly Asp Gly Phe Ser Asp Val
            260                 265                 270

Leu Ile Gly Val Pro Tyr Asp Val Gly Asn Ile Gly Arg Val Tyr Ser
        275                 280                 285

Tyr Phe Ser Asn Gly Val Met Gly Gln Gly Val Thr Phe Gly Gln Gln
    290                 295                 300

Leu Asn Asn Pro Gly Asn Ala Gly Ser Ala Ala Phe Tyr Gly Tyr Ala
305                 310                 315                 320

Ile Thr Leu Gly Asp Ile Asn Gly Asp Gly Lys Ser Asp Ala Ile Ile
                325                 330                 335

Gly Ala Val Gly Ser Gly Gln Val Gly Ala Ser Phe Ile Tyr Leu Ala
            340                 345                 350

Gln Ala Ala Gly Ala Phe Ala Ala Tyr Ser Gln Thr Ile Thr Gly Ser
        355                 360                 365

Ala Ala Asn Glu Trp Tyr Ala Asn Ser Ala Ile Ala Thr Asp Ile Asn
    370                 375                 380

Arg Asp Gly Phe Ala Asp Leu Phe Val Gly Ala Tyr Gln Glu Ser Gly
385                 390                 395                 400

Gly Val Gly Arg Val His Leu Tyr Leu Ser Asn Leu Gly Ile Leu Val
                405                 410                 415

Asn Thr Ala Asn Gly Pro Ile Ser Gly Leu Val Gly Ser Gln Thr Gly
            420                 425                 430

Thr Ser Val Ala Thr Gly Asp Val Asn Gly Asp Gly Phe Leu Asp Leu
        435                 440                 445

Leu Thr Gly Gly Tyr Ser Tyr Thr Ser Thr Phe Pro Asn Gln Gly Tyr
    450                 455                 460

Ala Ile Thr His Leu Thr Thr Gly Asp Thr Met Gly Leu Thr Val Asn
465                 470                 475                 480

Pro Leu Asn Leu Leu Thr Val Pro Val Asn Leu Gly Asp Met Gly Asn
                485                 490                 495

Ser Ile Ala Ser Val Asp Ile Asn Gly Asp Gly Leu Ser Asp Val Leu
            500                 505                 510

Val Gly Ala Pro Ser Ser Val Gly Gly Thr Asn Val Gly Asn Val Tyr
        515                 520                 525

Leu Tyr Ile Ser Asn Gly Leu Asp Gly Tyr Thr Ser Ala Pro Gln Ile
    530                 535                 540

Phe Val Glu Pro Asp Val Asn Gly Thr Phe Gly Thr Ser Val Asp Leu
545                 550                 555                 560

<210>  60
<211>  3492
<212>  DNA
<213>  Leptospira interrogans serovar Pomona

<220>
<221>  CDS
<222>  (1)..(3492)

<400>  60
ttg aac aca tcc atc caa gtt ggt ttt agt caa aaa ttg gat tct tct      48
Met Asn Thr Ser Ile Gln Val Gly Phe Ser Gln Lys Leu Asp Ser Ser
1               5                   10                  15

agt atc caa tct caa tcg atc caa cta aca caa gga aat acg atc att      96
Ser Ile Gln Ser Gln Ser Ile Gln Leu Thr Gln Gly Asn Thr Ile Ile
            20                  25                  30

ccg gga aat ttc act tct aca gaa aaa act ctt tta ttc aat cca acg      144
Pro Gly Asn Phe Thr Ser Thr Glu Lys Thr Leu Leu Phe Asn Pro Thr

35                  40                  45

tct tcc ttg gct gca tca act gta tat tcc gtt agc att tcc aaa gac          192
Ser Ser Leu Ala Ala Ser Thr Val Tyr Ser Val Ser Ile Ser Lys Asp
    50                  55                  60

ata aaa tca atg gac ggc tcc tct ctt tca gaa gac tat act tgg agc          240
Ile Lys Ser Met Asp Gly Ser Ser Leu Ser Glu Asp Tyr Thr Trp Ser
65                  70                  75                  80

ttt acc acc aac acg att gtt gat tta gtc gcg cca gac gta tct tta          288
Phe Thr Thr Asn Thr Ile Val Asp Leu Val Ala Pro Asp Val Ser Leu
                85                  90                  95

aga acc cct acc ata ggt gcc aat tta gtt cct aat aat act tcc gtt          336
Arg Thr Pro Thr Ile Gly Ala Asn Leu Val Pro Asn Asn Thr Ser Val
            100                 105                 110

caa att gcg ttt act gaa aca atg aat tgc act tcc atc aac att gta          384
Gln Ile Ala Phe Thr Glu Thr Met Asn Cys Thr Ser Ile Asn Ile Val
        115                 120                 125

aat ttt aca tta aaa aat aat gtt acc aat gta tta gaa cct agt aat          432
Asn Phe Thr Leu Lys Asn Asn Val Thr Asn Val Leu Glu Pro Ser Asn
    130                 135                 140

gta gtt tgt tta gga tcc gtt gcc acc ctt acc cct aat aat cct ctt          480
Val Val Cys Leu Gly Ser Val Ala Thr Leu Thr Pro Asn Asn Pro Leu
145                 150                 155                 160

gct ttt aat act gtt tat cgt gtg gat att ctt tct aca gca aaa gat          528
Ala Phe Asn Thr Val Tyr Arg Val Asp Ile Leu Ser Thr Ala Lys Asp
                165                 170                 175

ctt gct aat aat cct ctt gta aac gca tat aac tgg act ttt aca acc          576
Leu Ala Asn Asn Pro Leu Val Asn Ala Tyr Asn Trp Thr Phe Thr Thr
            180                 185                 190

gga gtt gcc ccc gat tta acg gtt cca acc gtc tcc ttt gta aat ccc          624
Gly Val Ala Pro Asp Leu Thr Val Pro Thr Val Ser Phe Val Asn Pro
        195                 200                 205

act ccc aat gca caa aac gtt cct atc aac gaa aca atc agt att gcc          672
Thr Pro Asn Ala Gln Asn Val Pro Ile Asn Glu Thr Ile Ser Ile Ala
    210                 215                 220

ttt agt gaa cct atc aat tgt gct acg atc atc gga agt att gtc tta          720
Phe Ser Glu Pro Ile Asn Cys Ala Thr Ile Ile Gly Ser Ile Val Leu
225                 230                 235                 240

gat gat aat ata ctt att cct gga agc gta aac ggg aat ccg ggt tgt          768
Asp Asp Asn Ile Leu Ile Pro Gly Ser Val Asn Gly Asn Pro Gly Cys
                245                 250                 255

aca ggc aca acc gca tcc ttt act cct tta ggc aat ttg aca cca aat          816
Thr Gly Thr Thr Ala Ser Phe Thr Pro Leu Gly Asn Leu Thr Pro Asn
            260                 265                 270

acg aat tat acg gtt acc gtc tca aat gcg att acg gat ttg caa aac          864
Thr Asn Tyr Thr Val Thr Val Ser Asn Ala Ile Thr Asp Leu Gln Asn
        275                 280                 285

aat cca ctt aca cct tct acc tgg agt ttt acg acg gct gct gca gtc          912
Asn Pro Leu Thr Pro Ser Thr Trp Ser Phe Thr Thr Ala Ala Ala Val
    290                 295                 300

gat cag act caa ccc acg gtt aca ttt act gta ccc tcc gcc aac gca 960
Asp Gln Thr Gln Pro Thr Val Thr Phe Thr Val Pro Ser Ala Asn Ala
305                 310                 315                 320

aac ggc gta ggg acc aat gtc aac cct atg gta gtt ttt agc gaa cct 1008
Asn Gly Val Gly Thr Asn Val Asn Pro Met Val Val Phe Ser Glu Pro
                325                 330                 335

atg tct tgc gct tcc gtg acc tcc gca tcc ttt cga tta aag agg cag 1056
Met Ser Cys Ala Ser Val Thr Ser Ala Ser Phe Arg Leu Lys Arg Gln
            340                 345                 350

gca acc ggg gtt tat ctt ata gga agt gta aat tgt ttt gga act tcc 1104
Ala Thr Gly Val Tyr Leu Ile Gly Ser Val Asn Cys Phe Gly Thr Ser
        355                 360                 365

gct acc tgg acc cca gat cct gta aat ccg ctt gcc ttt aat act act 1152
Ala Thr Trp Thr Pro Asp Pro Val Asn Pro Leu Ala Phe Asn Thr Thr
    370                 375                 380

tac acc gta gaa atc gat cag ggt gca tta gat act ttt aat aat cct 1200
Tyr Thr Val Glu Ile Asp Gln Gly Ala Leu Asp Thr Phe Asn Asn Pro
385                 390                 395                 400

ctc att cca ata aat tgg aac ttt act aca ggc cca gga ccc gat cta 1248
Leu Ile Pro Ile Asn Trp Asn Phe Thr Thr Gly Pro Gly Pro Asp Leu
                405                 410                 415

act cct ccc agt gtt gcc gtc gtt act ccc gca aac gct gcg ata ggc 1296
Thr Pro Pro Ser Val Ala Val Val Thr Pro Ala Asn Ala Ala Ile Gly
            420                 425                 430

gtt ccg acc aac gga ggt gta agt atc gct ttt agt gaa gcc atg aat 1344
Val Pro Thr Asn Gly Gly Val Ser Ile Ala Phe Ser Glu Ala Met Asn
        435                 440                 445

tgt gga agt att tta gga gga atc acc ctg gac gat gat cct aca act 1392
Cys Gly Ser Ile Leu Gly Gly Ile Thr Leu Asp Asp Asp Pro Thr Thr
    450                 455                 460

ccg ggt act gtg att cca atc aat atc aat tgt aac gga aat act gtc 1440
Pro Gly Thr Val Ile Pro Ile Asn Ile Asn Cys Asn Gly Asn Thr Val
465                 470                 475                 480

tct ttt gcc ccg aca atc cct cca ctt gcc ttt aat aca acc tac aca 1488
Ser Phe Ala Pro Thr Ile Pro Pro Leu Ala Phe Asn Thr Thr Tyr Thr
                485                 490                 495

gta acg att tta aat acg gtt acg gac agt aat aat aat gca cta aac 1536
Val Thr Ile Leu Asn Thr Val Thr Asp Ser Asn Asn Asn Ala Leu Asn
            500                 505                 510

gga gga aat tac gct tgg tct ttt aca aca gga gtg gca cca gac cta 1584
Gly Gly Asn Tyr Ala Trp Ser Phe Thr Thr Gly Val Ala Pro Asp Leu
        515                 520                 525

gtt cct cct caa gtt tct ctt gta agc cct ctc tcg gga gca gta ggt 1632
Val Pro Pro Gln Val Ser Leu Val Ser Pro Leu Ser Gly Ala Val Gly
    530                 535                 540

gtc gca aca aat gca aac att aca gtt gca ttt aat gaa acc atc aat 1680
Val Ala Thr Asn Ala Asn Ile Thr Val Ala Phe Asn Glu Thr Ile Asn
545                 550                 555                 560

tgt tcc acc ttg aat ttt acg gtt aat aac gga atc aat ggt act gtg 1728
Cys Ser Thr Leu Asn Phe Thr Val Asn Asn Gly Ile Asn Gly Thr Val
                565                 570                 575

aac tgt tca gga tcc tct gct act ttt att cct agc gct tta acc cct 1776
Asn Cys Ser Gly Ser Ser Ala Thr Phe Ile Pro Ser Ala Leu Thr Pro
            580                 585                 590

cta aat gcc ggt aca aat tat aca gca aca atc cta aca gta aat gat 1824
Leu Asn Ala Gly Thr Asn Tyr Thr Ala Thr Ile Leu Thr Val Asn Asp
        595                 600                 605

att gta gga aat cca atc ggt gct gcg ttt ggt tgg agt ttt aca acc 1872
Ile Val Gly Asn Pro Ile Gly Ala Ala Phe Gly Trp Ser Phe Thr Thr
    610                 615                 620

gga gcc gct cca gat gtg act cct cct gtc gtt acg atc caa aat ctt 1920
Gly Ala Ala Pro Asp Val Thr Pro Pro Val Val Thr Ile Gln Asn Leu
625                 630                 635                 640

aga aat aat tct atc gta gaa act ggt ttc gta att gga acg gca aca 1968
Arg Asn Asn Ser Ile Val Glu Thr Gly Phe Val Ile Gly Thr Ala Thr
                645                 650                 655

gat gca ggc aca att act tct gta gaa gtt tct ttg gac aat ggc gct 2016
Asp Ala Gly Thr Ile Thr Ser Val Glu Val Ser Leu Asp Asn Gly Ala
            660                 665                 670

ttt gtg ccc gca acc gga acc aat cct tgg aaa ttt aaa ctt cct tcg 2064
Phe Val Pro Ala Thr Gly Thr Asn Pro Trp Lys Phe Lys Leu Pro Ser
        675                 680                 685

gat ata aat act tgg aag caa aac tct caa cat acg atc att gca aga 2112
Asp Ile Asn Thr Trp Lys Gln Asn Ser Gln His Thr Ile Ile Ala Arg
    690                 695                 700

gct aag gac tta gcc aat aac ctt aca act aca gca gcc att tcg gtt 2160
Ala Lys Asp Leu Ala Asn Asn Leu Thr Thr Thr Ala Ala Ile Ser Val
705                 710                 715                 720

cga aaa gga aac aat aaa gat ata aac gga gac gga tac gtt gat ctt 2208
Arg Lys Gly Asn Asn Lys Asp Ile Asn Gly Asp Gly Tyr Val Asp Leu
                725                 730                 735

gta tct gca gaa tac gga cag ggt tta ctt tat att ttt cat tct tcc 2256
Val Ser Ala Glu Tyr Gly Gln Gly Leu Leu Tyr Ile Phe His Ser Ser
            740                 745                 750

ggg aac gct gga atg aca att aca aac gca caa tcc gca agt aag atc 2304
Gly Asn Ala Gly Met Thr Ile Thr Asn Ala Gln Ser Ala Ser Lys Ile
        755                 760                 765

att gta gga gtc gcg gcg gaa gaa ttt gga aga act gtt tct atg gga 2352
Ile Val Gly Val Ala Ala Glu Glu Phe Gly Arg Thr Val Ser Met Gly
    770                 775                 780

gat tta aac gga gat gga ttt gca gac gta att tct ggt gct cct ggt 2400
Asp Leu Asn Gly Asp Gly Phe Ala Asp Val Ile Ser Gly Ala Pro Gly
785                 790                 795                 800

tgg aac ggt gcc caa ggt agg gtt tat ata ttt cat tct tcc gga aac 2448
Trp Asn Gly Ala Gln Gly Arg Val Tyr Ile Phe His Ser Ser Gly Asn
                805                 810                 815

gct gga gtc aat att tcc ttt tcc ggt ttt gcc act aaa acg atc agt 2496

Ala Gly Val Asn Ile Ser Phe Ser Gly Phe Ala Thr Lys Thr Ile Ser
            820                 825                 830

gga gcc aat gcg gga gct aga ttt gga gat agt att gtt aca ggt gat        2544
Gly Ala Asn Ala Gly Ala Arg Phe Gly Asp Ser Ile Val Thr Gly Asp
        835                 840                 845

tta aac gga gac ggc tac gca gac cta gca tca gga gaa ccc gtt ttt        2592
Leu Asn Gly Asp Gly Tyr Ala Asp Leu Ala Ser Gly Glu Pro Val Phe
    850                 855                 860

aat ggt tct cag ggt aga gtc tat gta ttt cat tcc gct gga gcc gct        2640
Asn Gly Ser Gln Gly Arg Val Tyr Val Phe His Ser Ala Gly Ala Ala
865                 870                 875                 880

gga gtt aca caa ata aat tcg gct gct gca aat tct aca ctc aca ggt        2688
Gly Val Thr Gln Ile Asn Ser Ala Ala Ala Asn Ser Thr Leu Thr Gly
                885                 890                 895

gaa aac gca act gat cgt ttc gga tat tcg ttg agc act gga aat atg        2736
Glu Asn Ala Thr Asp Arg Phe Gly Tyr Ser Leu Ser Thr Gly Asn Met
            900                 905                 910

aac gga gat aat ttt gca gat ctt gca att gga gca cct ggt tat ggc        2784
Asn Gly Asp Asn Phe Ala Asp Leu Ala Ile Gly Ala Pro Gly Tyr Gly
        915                 920                 925

gct ggc gta ggt gga gga ttt gta gta aac caa ggt aaa gtt tat ata        2832
Ala Gly Val Gly Gly Gly Phe Val Val Asn Gln Gly Lys Val Tyr Ile
    930                 935                 940

cat cac ggg gcg gct ggt gga ttg gga gga gta att aca act ctt acc        2880
His His Gly Ala Ala Gly Gly Leu Gly Gly Val Ile Thr Thr Leu Thr
945                 950                 955                 960

aat gat agc gct gga aat gct ggg gaa ttt ggt atc agt tta ttt aca        2928
Asn Asp Ser Ala Gly Asn Ala Gly Glu Phe Gly Ile Ser Leu Phe Thr
                965                 970                 975

gcc gat ttt aac gga gac ggt aat tcc gat ctt gca att gga agt cca        2976
Ala Asp Phe Asn Gly Asp Gly Asn Ser Asp Leu Ala Ile Gly Ser Pro
            980                 985                 990

aat tta ggg ggt gga act gga aga  gtt tcc gta ttt acg  tct gca gga        3024
Asn Leu Gly Gly Gly Thr Gly Arg  Val Ser Val Phe Thr  Ser Ala Gly
        995                 1000                1005

ggt gta  ggt ata aat aca tct  acg att gga aac gct  ccg ctt atg        3069
Gly Val  Gly Ile Asn Thr Ser  Thr Ile Gly Asn Ala  Pro Leu Met
    1010                 1015                 1020

atc aat  gga acg gct gtg ggt  aat gca ttt gga att  tct tta acc        3114
Ile Asn  Gly Thr Ala Val Gly  Asn Ala Phe Gly Ile  Ser Leu Thr
    1025                 1030                 1035

gca cag  gat tta aat tta gat  gga aga ccg gat tta  att tcc gca        3159
Ala Gln  Asp Leu Asn Leu Asp  Gly Arg Pro Asp Leu  Ile Ser Ala
    1040                 1045                 1050

acc gta  att ccc aat agg gta  ttc gtt ttc cac atg  cca ggt gca        3204
Thr Val  Ile Pro Asn Arg Val  Phe Val Phe His Met  Pro Gly Ala
    1055                 1060                 1065

gga gct  atc ggt gga ttt cta  acc act ggt aat gca  act aca caa        3249
Gly Ala  Ile Gly Gly Phe Leu  Thr Thr Gly Asn Ala  Thr Thr Gln

1070                1075                1080

atc acg  agt gca ttt gca gga  atc ggg gtt tcc cca  aat gca cct        3294
Ile Thr  Ser Ala Phe Ala Gly  Ile Gly Val Ser Pro  Asn Ala Pro
    1085                1090                1095

aaa act  cct att tcc ggc gga  gac atc aac gga gac  gga ttt cca        3339
Lys Thr  Pro Ile Ser Gly Gly  Asp Ile Asn Gly Asp  Gly Phe Pro
    1100                1105                1110

gac tta  ttt gtc ggg gga agt  tct gat aat att ttt  att ttc cat        3384
Asp Leu  Phe Val Gly Gly Ser  Ser Asp Asn Ile Phe  Ile Phe His
    1115                1120                1125

tct tca  acg gtt gga act ggt  tta tta aca aat act  act gca acc        3429
Ser Ser  Thr Val Gly Thr Gly  Leu Leu Thr Asn Thr  Thr Ala Thr
    1130                1135                1140

gct gca  ggc gca att acc agt  tcc gga ctt gcc aat  ggc ttt ttt        3474
Ala Ala  Gly Ala Ile Thr Ser  Ser Gly Leu Ala Asn  Gly Phe Phe
    1145                1150                1155

gga tgt  agc gtt tat tga                                              3492
Gly Cys  Ser Val Tyr
    1160

<210>  61
<211>  1163
<212>  PRT
<213>  Leptospira interrogans serovar Pomona

<400>  61

Met Asn Thr Ser Ile Gln Val Gly Phe Ser Gln Lys Leu Asp Ser Ser
1               5                   10                  15

Ser Ile Gln Ser Gln Ser Ile Gln Leu Thr Gln Gly Asn Thr Ile Ile
            20                  25                  30

Pro Gly Asn Phe Thr Ser Thr Glu Lys Thr Leu Leu Phe Asn Pro Thr
        35                  40                  45

Ser Ser Leu Ala Ala Ser Thr Val Tyr Ser Val Ser Ile Ser Lys Asp
    50                  55                  60

Ile Lys Ser Met Asp Gly Ser Ser Leu Ser Glu Asp Tyr Thr Trp Ser
65                  70                  75                  80

Phe Thr Thr Asn Thr Ile Val Asp Leu Val Ala Pro Asp Val Ser Leu
                85                  90                  95

Arg Thr Pro Thr Ile Gly Ala Asn Leu Val Pro Asn Asn Thr Ser Val
            100                 105                 110

Gln Ile Ala Phe Thr Glu Thr Met Asn Cys Thr Ser Ile Asn Ile Val
        115                 120                 125

Asn Phe Thr Leu Lys Asn Asn Val Thr Asn Val Leu Glu Pro Ser Asn
    130                 135                 140

Val Val Cys Leu Gly Ser Val Ala Thr Leu Thr Pro Asn Asn Pro Leu
145                 150                 155                 160

Ala Phe Asn Thr Val Tyr Arg Val Asp Ile Leu Ser Thr Ala Lys Asp
                165                 170                 175

Leu Ala Asn Asn Pro Leu Val Asn Ala Tyr Asn Trp Thr Phe Thr Thr
            180                 185                 190

Gly Val Ala Pro Asp Leu Thr Val Pro Thr Val Ser Phe Val Asn Pro
        195                 200                 205

Thr Pro Asn Ala Gln Asn Val Pro Ile Asn Glu Thr Ile Ser Ile Ala
    210                 215                 220

Phe Ser Glu Pro Ile Asn Cys Ala Thr Ile Ile Gly Ser Ile Val Leu
225                 230                 235                 240

Asp Asp Asn Ile Leu Ile Pro Gly Ser Val Asn Gly Asn Pro Gly Cys
                245                 250                 255

Thr Gly Thr Thr Ala Ser Phe Thr Pro Leu Gly Asn Leu Thr Pro Asn
            260                 265                 270

Thr Asn Tyr Thr Val Thr Val Ser Asn Ala Ile Thr Asp Leu Gln Asn
        275                 280                 285

Asn Pro Leu Thr Pro Ser Thr Trp Ser Phe Thr Thr Ala Ala Ala Val
    290                 295                 300

Asp Gln Thr Gln Pro Thr Val Thr Phe Thr Val Pro Ser Ala Asn Ala
305                 310                 315                 320

Asn Gly Val Gly Thr Asn Val Asn Pro Met Val Val Phe Ser Glu Pro
                325                 330                 335

Met Ser Cys Ala Ser Val Thr Ser Ala Ser Phe Arg Leu Lys Arg Gln
            340                 345                 350

Ala Thr Gly Val Tyr Leu Ile Gly Ser Val Asn Cys Phe Gly Thr Ser
        355                 360                 365

Ala Thr Trp Thr Pro Asp Pro Val Asn Pro Leu Ala Phe Asn Thr Thr
    370                 375                 380

Tyr Thr Val Glu Ile Asp Gln Gly Ala Leu Asp Thr Phe Asn Asn Pro
385                 390                 395                 400

Leu Ile Pro Ile Asn Trp Asn Phe Thr Thr Gly Pro Gly Pro Asp Leu
                405                 410                 415

Thr Pro Pro Ser Val Ala Val Val Thr Pro Ala Asn Ala Ala Ile Gly
            420                 425                 430

Val Pro Thr Asn Gly Gly Val Ser Ile Ala Phe Ser Glu Ala Met Asn
        435                 440                 445

Cys Gly Ser Ile Leu Gly Gly Ile Thr Leu Asp Asp Asp Pro Thr Thr
    450                 455                 460

Pro Gly Thr Val Ile Pro Ile Asn Ile Asn Cys Asn Gly Asn Thr Val
465                 470                 475                 480

Ser Phe Ala Pro Thr Ile Pro Pro Leu Ala Phe Asn Thr Thr Tyr Thr
                485                 490                 495

Val Thr Ile Leu Asn Thr Val Thr Asp Ser Asn Asn Asn Ala Leu Asn
            500                 505                 510

Gly Gly Asn Tyr Ala Trp Ser Phe Thr Thr Gly Val Ala Pro Asp Leu
        515                 520                 525

Val Pro Pro Gln Val Ser Leu Val Ser Pro Leu Ser Gly Ala Val Gly
    530                 535                 540

Val Ala Thr Asn Ala Asn Ile Thr Val Ala Phe Asn Glu Thr Ile Asn
545                 550                 555                 560

Cys Ser Thr Leu Asn Phe Thr Val Asn Asn Gly Ile Asn Gly Thr Val
                565                 570                 575

Asn Cys Ser Gly Ser Ser Ala Thr Phe Ile Pro Ser Ala Leu Thr Pro
            580                 585                 590

Leu Asn Ala Gly Thr Asn Tyr Thr Ala Thr Ile Leu Thr Val Asn Asp
        595                 600                 605

Ile Val Gly Asn Pro Ile Gly Ala Ala Phe Gly Trp Ser Phe Thr Thr
    610                 615                 620

Gly Ala Ala Pro Asp Val Thr Pro Pro Val Val Thr Ile Gln Asn Leu
625                 630                 635                 640

Arg Asn Asn Ser Ile Val Glu Thr Gly Phe Val Ile Gly Thr Ala Thr

                645                 650                 655
Asp Ala Gly Thr Ile Thr Ser Val Glu Val Ser Leu Asp Asn Gly Ala
            660                 665                 670
Phe Val Pro Ala Thr Gly Thr Asn Pro Trp Lys Phe Lys Leu Pro Ser
        675                 680                 685
Asp Ile Asn Thr Trp Lys Gln Asn Ser Gln His Thr Ile Ile Ala Arg
    690                 695                 700
Ala Lys Asp Leu Ala Asn Asn Leu Thr Thr Thr Ala Ala Ile Ser Val
705                 710                 715                 720
Arg Lys Gly Asn Asn Lys Asp Ile Asn Gly Asp Gly Tyr Val Asp Leu
                725                 730                 735
Val Ser Ala Glu Tyr Gly Gln Gly Leu Leu Tyr Ile Phe His Ser Ser
            740                 745                 750
Gly Asn Ala Gly Met Thr Ile Thr Asn Ala Gln Ser Ala Ser Lys Ile
        755                 760                 765
Ile Val Gly Val Ala Ala Glu Glu Phe Gly Arg Thr Val Ser Met Gly
    770                 775                 780
Asp Leu Asn Gly Asp Gly Phe Ala Asp Val Ile Ser Gly Ala Pro Gly
785                 790                 795                 800
Trp Asn Gly Ala Gln Gly Arg Val Tyr Ile Phe His Ser Ser Gly Asn
                805                 810                 815
Ala Gly Val Asn Ile Ser Phe Ser Gly Phe Ala Thr Lys Thr Ile Ser
            820                 825                 830
Gly Ala Asn Ala Gly Ala Arg Phe Gly Asp Ser Ile Val Thr Gly Asp
        835                 840                 845
Leu Asn Gly Asp Gly Tyr Ala Asp Leu Ala Ser Gly Glu Pro Val Phe
    850                 855                 860
Asn Gly Ser Gln Gly Arg Val Tyr Val Phe His Ser Ala Gly Ala Ala
865                 870                 875                 880
Gly Val Thr Gln Ile Asn Ser Ala Ala Ala Asn Ser Thr Leu Thr Gly
                885                 890                 895
Glu Asn Ala Thr Asp Arg Phe Gly Tyr Ser Leu Ser Thr Gly Asn Met
            900                 905                 910

Asn Gly Asp Asn Phe Ala Asp Leu Ala Ile Gly Ala Pro Gly Tyr Gly
        915                 920                 925

Ala Gly Val Gly Gly Gly Phe Val Val Asn Gln Gly Lys Val Tyr Ile
    930                 935                 940

His His Gly Ala Ala Gly Gly Leu Gly Gly Val Ile Thr Thr Leu Thr
945                 950                 955                 960

Asn Asp Ser Ala Gly Asn Ala Gly Glu Phe Gly Ile Ser Leu Phe Thr
                965                 970                 975

Ala Asp Phe Asn Gly Asp Gly Asn Ser Asp Leu Ala Ile Gly Ser Pro
            980                 985                 990

Asn Leu Gly Gly Gly Thr Gly Arg Val Ser Val Phe Thr Ser Ala Gly
        995                 1000                1005

Gly Val Gly Ile Asn Thr Ser Thr Ile Gly Asn Ala Pro Leu Met
    1010                1015                1020

Ile Asn Gly Thr Ala Val Gly Asn Ala Phe Gly Ile Ser Leu Thr
    1025                1030                1035

Ala Gln Asp Leu Asn Leu Asp Gly Arg Pro Asp Leu Ile Ser Ala
    1040                1045                1050

Thr Val Ile Pro Asn Arg Val Phe Val Phe His Met Pro Gly Ala
    1055                1060                1065

Gly Ala Ile Gly Gly Phe Leu Thr Thr Gly Asn Ala Thr Thr Gln
    1070                1075                1080

Ile Thr Ser Ala Phe Ala Gly Ile Gly Val Ser Pro Asn Ala Pro
    1085                1090                1095

Lys Thr Pro Ile Ser Gly Gly Asp Ile Asn Gly Asp Gly Phe Pro
    1100                1105                1110

Asp Leu Phe Val Gly Gly Ser Ser Asp Asn Ile Phe Ile Phe His
    1115                1120                1125

Ser Ser Thr Val Gly Thr Gly Leu Leu Thr Asn Thr Thr Ala Thr
    1130                1135                1140

Ala Ala Gly Ala Ile Thr Ser Ser Gly Leu Ala Asn Gly Phe Phe
    1145                1150                1155

Gly Cys Ser Val Tyr
    1160

<210> 62
<211> 1287
<212> DNA
<213> Leptospira interrogans serovar Pomona

<220>
<221> CDS
<222> (1)..(1287)

<400> 62
atg agc cta aaa aat aaa aat tat gtt tta tca aaa aaa acc ata ttg      48
Met Ser Leu Lys Asn Lys Asn Tyr Val Leu Ser Lys Lys Thr Ile Leu
1               5                   10                  15

att cta ttt tta gta tat ttt gtt ttt ata att tct ttt ttt tct att      96
Ile Leu Phe Leu Val Tyr Phe Val Phe Ile Ile Ser Phe Phe Ser Ile
            20                  25                  30

tac tct cag gat tta agt ata aat caa aat cct aaa cca gaa aag tta      144
Tyr Ser Gln Asp Leu Ser Ile Asn Gln Asn Pro Lys Pro Glu Lys Leu
        35                  40                  45

aaa ggt tct atc aat acg agt ctc aat gag ttt gga atc agt ctt act      192
Lys Gly Ser Ile Asn Thr Ser Leu Asn Glu Phe Gly Ile Ser Leu Thr
    50                  55                  60

gat gac gga aat att tta tat ttc tat tct aaa aga caa aat tca aat      240
Asp Asp Gly Asn Ile Leu Tyr Phe Tyr Ser Lys Arg Gln Asn Ser Asn
65                  70                  75                  80

tat aca gac att tat aag tca act cga acg aaa gat gaa tgg aca caa      288
Tyr Thr Asp Ile Tyr Lys Ser Thr Arg Thr Lys Asp Glu Trp Thr Gln
                85                  90                  95

gga gag gaa att gaa gtt cta aat tca aac ttt gac gac caa agt cct      336
Gly Glu Glu Ile Glu Val Leu Asn Ser Asn Phe Asp Asp Gln Ser Pro
            100                 105                 110

ttc att tta aac cga gaa gaa gga att ctt ttt tca tct aat aga gat      384
Phe Ile Leu Asn Arg Glu Glu Gly Ile Leu Phe Ser Ser Asn Arg Asp
        115                 120                 125

ggt gcg acc gaa ttc caa ttt gca aat gga aaa atc gga gtt tct aga      432
Gly Ala Thr Glu Phe Gln Phe Ala Asn Gly Lys Ile Gly Val Ser Arg
    130                 135                 140

gat att tat ttt tct aaa aaa ata aat tct tct tgg aca gaa ccg gtt      480
Asp Ile Tyr Phe Ser Lys Lys Ile Asn Ser Ser Trp Thr Glu Pro Val
145                 150                 155                 160

ctt ctt cct aga gcc gtg aat acg gaa gaa atc gaa gaa aat ccg ttt      528
Leu Leu Pro Arg Ala Val Asn Thr Glu Glu Ile Glu Glu Asn Pro Phe
                165                 170                 175

cta ttt aat aat aga ttg tat ttt acc cgt tat cct ttt ggg caa gtt      576
Leu Phe Asn Asn Arg Leu Tyr Phe Thr Arg Tyr Pro Phe Gly Gln Val
            180                 185                 190

tca gaa gcg gac att ttc gtt tct gtt tat aaa aat aac act tgg gaa      624

Ser Glu Ala Asp Ile Phe Val Ser Val Tyr Lys Asn Asn Thr Trp Glu
        195                 200                 205

aaa gca atg agc ctc cct gat ccg att aac acc gtt tat tcg gaa att        672
Lys Ala Met Ser Leu Pro Asp Pro Ile Asn Thr Val Tyr Ser Glu Ile
    210                 215                 220

gcg gct aca att agt aaa gat gga aag acg att tat ttt tct tct aac        720
Ala Ala Thr Ile Ser Lys Asp Gly Lys Thr Ile Tyr Phe Ser Ser Asn
225                 230                 235                 240

cgt ccg ggg ggt ttt ggc ggt tat gat ttg tat aag tct act tta ctt        768
Arg Pro Gly Gly Phe Gly Gly Tyr Asp Leu Tyr Lys Ser Thr Leu Leu
                245                 250                 255

gaa aac gga aat tat tcc gaa ccg att aat ctt gga ccc gaa att aac        816
Glu Asn Gly Asn Tyr Ser Glu Pro Ile Asn Leu Gly Pro Glu Ile Asn
            260                 265                 270

act acc gga gat gag gct ttt ttt ctg gaa aca aac gat aga aag aca        864
Thr Thr Gly Asp Glu Ala Phe Phe Leu Glu Thr Asn Asp Arg Lys Thr
        275                 280                 285

ttc tat ttt tgt aga agg aaa gaa cgc gat tat gat att tat tct atc        912
Phe Tyr Phe Cys Arg Arg Lys Glu Arg Asp Tyr Asp Ile Tyr Ser Ile
    290                 295                 300

gtt tct aac ccg ttt caa gaa cta gaa aaa gga aaa tct att tct ttg        960
Val Ser Asn Pro Phe Gln Glu Leu Glu Lys Gly Lys Ser Ile Ser Leu
305                 310                 315                 320

gat agt atc cat ttt tct ttg ggc tct tat gaa att ctc gaa aat tct       1008
Asp Ser Ile His Phe Ser Leu Gly Ser Tyr Glu Ile Leu Glu Asn Ser
                325                 330                 335

ttt tca att tta gat aat ttg aat tct tat ctt aag gaa aat tta aat       1056
Phe Ser Ile Leu Asp Asn Leu Asn Ser Tyr Leu Lys Glu Asn Leu Asn
            340                 345                 350

ata aaa atc aaa atc acc ggc cat acc gat ctt aat gga gat tcc cag       1104
Ile Lys Ile Lys Ile Thr Gly His Thr Asp Leu Asn Gly Asp Ser Gln
        355                 360                 365

gac aac ctt att ctc agc cgt aat cgt gca aat gca gta aag gat tat       1152
Asp Asn Leu Ile Leu Ser Arg Asn Arg Ala Asn Ala Val Lys Asp Tyr
    370                 375                 380

tta gtt aaa agg gga atc gat tct caa aga att atc acg gat ggg aaa       1200
Leu Val Lys Arg Gly Ile Asp Ser Gln Arg Ile Ile Thr Asp Gly Lys
385                 390                 395                 400

ggt agt tcg gag cca att gtt cct atg aaa aat cca gag acg gat tat       1248
Gly Ser Ser Glu Pro Ile Val Pro Met Lys Asn Pro Glu Thr Asp Tyr
                405                 410                 415

aaa aat aga aga acc gaa ttt cag att atc agt cgt tag                   1287
Lys Asn Arg Arg Thr Glu Phe Gln Ile Ile Ser Arg
            420                 425

<210>  63
<211>  428
<212>  PRT
<213>  Leptospira interrogans serovar Pomona

<400>  63

Met Ser Leu Lys Asn Lys Asn Tyr Val Leu Ser Lys Lys Thr Ile Leu
1               5                   10                  15

Ile Leu Phe Leu Val Tyr Phe Val Phe Ile Ile Ser Phe Phe Ser Ile
            20                  25                  30

Tyr Ser Gln Asp Leu Ser Ile Asn Gln Asn Pro Lys Pro Glu Lys Leu
        35                  40                  45

Lys Gly Ser Ile Asn Thr Ser Leu Asn Glu Phe Gly Ile Ser Leu Thr
    50                  55                  60

Asp Asp Gly Asn Ile Leu Tyr Phe Tyr Ser Lys Arg Gln Asn Ser Asn
65                  70                  75                  80

Tyr Thr Asp Ile Tyr Lys Ser Thr Arg Thr Lys Asp Glu Trp Thr Gln
                85                  90                  95

Gly Glu Glu Ile Glu Val Leu Asn Ser Asn Phe Asp Asp Gln Ser Pro
            100                 105                 110

Phe Ile Leu Asn Arg Glu Glu Gly Ile Leu Phe Ser Ser Asn Arg Asp
        115                 120                 125

Gly Ala Thr Glu Phe Gln Phe Ala Asn Gly Lys Ile Gly Val Ser Arg
    130                 135                 140

Asp Ile Tyr Phe Ser Lys Lys Ile Asn Ser Ser Trp Thr Glu Pro Val
145                 150                 155                 160

Leu Leu Pro Arg Ala Val Asn Thr Glu Glu Ile Glu Glu Asn Pro Phe
                165                 170                 175

Leu Phe Asn Asn Arg Leu Tyr Phe Thr Arg Tyr Pro Phe Gly Gln Val
            180                 185                 190

Ser Glu Ala Asp Ile Phe Val Ser Val Tyr Lys Asn Asn Thr Trp Glu
        195                 200                 205

Lys Ala Met Ser Leu Pro Asp Pro Ile Asn Thr Val Tyr Ser Glu Ile
    210                 215                 220

Ala Ala Thr Ile Ser Lys Asp Gly Lys Thr Ile Tyr Phe Ser Ser Asn
225                 230                 235                 240

Arg Pro Gly Gly Phe Gly Gly Tyr Asp Leu Tyr Lys Ser Thr Leu Leu
                245                 250                 255

Glu Asn Gly Asn Tyr Ser Glu Pro Ile Asn Leu Gly Pro Glu Ile Asn
            260                 265                 270

Thr Thr Gly Asp Glu Ala Phe Phe Leu Glu Thr Asn Asp Arg Lys Thr
        275                 280                 285

Phe Tyr Phe Cys Arg Arg Lys Glu Arg Asp Tyr Asp Ile Tyr Ser Ile
    290                 295                 300

Val Ser Asn Pro Phe Gln Glu Leu Glu Lys Gly Lys Ser Ile Ser Leu
305                 310                 315                 320

Asp Ser Ile His Phe Ser Leu Gly Ser Tyr Glu Ile Leu Glu Asn Ser
                325                 330                 335

Phe Ser Ile Leu Asp Asn Leu Asn Ser Tyr Leu Lys Glu Asn Leu Asn
            340                 345                 350

Ile Lys Ile Lys Ile Thr Gly His Thr Asp Leu Asn Gly Asp Ser Gln
        355                 360                 365

Asp Asn Leu Ile Leu Ser Arg Asn Arg Ala Asn Ala Val Lys Asp Tyr
    370                 375                 380

Leu Val Lys Arg Gly Ile Asp Ser Gln Arg Ile Ile Thr Asp Gly Lys
385                 390                 395                 400

Gly Ser Ser Glu Pro Ile Val Pro Met Lys Asn Pro Glu Thr Asp Tyr
                405                 410                 415

Lys Asn Arg Arg Thr Glu Phe Gln Ile Ile Ser Arg
            420                 425

<210>  64
<211>  1152
<212>  DNA
<213>  Leptospira interrogans serovar Pomona

<220>
<221>  CDS
<222>  (1)..(1152)

<400>  64
atg gcg aag aaa gaa aac tac tat att act atc aaa ggt aga aaa tat        48
Met Ala Lys Lys Glu Asn Tyr Tyr Ile Thr Ile Lys Gly Arg Lys Tyr
1               5                   10                  15

gat cgt aag ttg atc cag ctc gcg gaa gag ttc act tcc ggt aaa cgg        96
Asp Arg Lys Leu Ile Gln Leu Ala Glu Glu Phe Thr Ser Gly Lys Arg
            20                  25                  30

gac ggt aag att tcg atc aac gac gca aaa cgt ctt tta aaa att gtc       144

Asp Gly Lys Ile Ser Ile Asn Asp Ala Lys Arg Leu Leu Lys Ile Val
        35                  40                  45

aag gat aac aac gct tat acg gat ata gaa aaa cat acg atc gaa tac 192
Lys Asp Asn Asn Ala Tyr Thr Asp Ile Glu Lys His Thr Ile Glu Tyr
    50                  55                  60

att cgt gaa aac tat aag ttt acc gaa aaa tcg gac gaa tgg ttc cgt 240
Ile Arg Glu Asn Tyr Lys Phe Thr Glu Lys Ser Asp Glu Trp Phe Arg
65                  70                  75                  80

tca gaa atc cgt aaa tgg gcc gct aaa aaa gtg caa gaa gca aaa aag 288
Ser Glu Ile Arg Lys Trp Ala Ala Lys Lys Val Gln Glu Ala Lys Lys
                85                  90                  95

aaa agt gat gtg gaa tcc atc cta gtc gat gat tct gaa gcg ccg gaa 336
Lys Ser Asp Val Glu Ser Ile Leu Val Asp Asp Ser Glu Ala Pro Glu
            100                 105                 110

ata aat ttt cct tcc agt tgg gga gaa gac aaa acc gaa gtt gtt gaa 384
Ile Asn Phe Pro Ser Ser Trp Gly Glu Asp Lys Thr Glu Val Val Glu
        115                 120                 125

att aca caa acc tct aaa ata gat tgg aga gaa aat tca aat ttt tca 432
Ile Thr Gln Thr Ser Lys Ile Asp Trp Arg Glu Asn Ser Asn Phe Ser
    130                 135                 140

tcc gca acg tct cac tct aaa aaa aat aaa aag atc att cca act tta 480
Ser Ala Thr Ser His Ser Lys Lys Asn Lys Lys Ile Ile Pro Thr Leu
145                 150                 155                 160

att ttt ctt tct ggt ttt ttg att ctt gta ggt tta gtt tat ttt ttt 528
Ile Phe Leu Ser Gly Phe Leu Ile Leu Val Gly Leu Val Tyr Phe Phe
                165                 170                 175

cga acc tta ttt tat aag gaa gac ttg gaa caa gta gta aaa acg aat 576
Arg Thr Leu Phe Tyr Lys Glu Asp Leu Glu Gln Val Val Lys Thr Asn
            180                 185                 190

tct gag att gtc tct aat tca aaa gaa aaa caa tcc gac gtt tcg ata 624
Ser Glu Ile Val Ser Asn Ser Lys Glu Lys Gln Ser Asp Val Ser Ile
        195                 200                 205

gaa aaa gca gaa tct aca aag gaa gtt cga aag aaa aac gta aga tct 672
Glu Lys Ala Glu Ser Thr Lys Glu Val Arg Lys Lys Asn Val Arg Ser
    210                 215                 220

aaa aaa gaa gaa tcg gaa att cca aaa aat gct ctt aca att cta aaa 720
Lys Lys Glu Glu Ser Glu Ile Pro Lys Asn Ala Leu Thr Ile Leu Lys
225                 230                 235                 240

cct caa acc gga aag aag tta gaa tct aaa tcc tta ttc tct tcg ttg 768
Pro Gln Thr Gly Lys Lys Leu Glu Ser Lys Ser Leu Phe Ser Ser Leu
                245                 250                 255

aca aat caa aat tcc aca gaa gaa ttt tct tcc aat cct caa ttt aga 816
Thr Asn Gln Asn Ser Thr Glu Glu Phe Ser Ser Asn Pro Gln Phe Arg
            260                 265                 270

gaa att gaa tcc aat gta att cgt ttt gaa aaa aac agc att caa att 864
Glu Ile Glu Ser Asn Val Ile Arg Phe Glu Lys Asn Ser Ile Gln Ile
        275                 280                 285

cat aaa gaa tcg aga cca agt ctc aac cgt ttg gct cgc tgg atg aaa 912
His Lys Glu Ser Arg Pro Ser Leu Asn Arg Leu Ala Arg Trp Met Lys

290                 295                 300

cag gat tct tcg atc cga gtc aaa gtt atc ggt cat act tct tta gag         960
Gln Asp Ser Ser Ile Arg Val Lys Val Ile Gly His Thr Ser Leu Glu
305                 310                 315                 320

ggt agc gaa gac gcc aat caa aaa gtt tct ctt ctt cgt gca caa acg         1008
Gly Ser Glu Asp Ala Asn Gln Lys Val Ser Leu Leu Arg Ala Gln Thr
                325                 330                 335

gtt cga aat tat atc gcc ggg aat ggc att tcc aaa gat cgt ttt gag         1056
Val Arg Asn Tyr Ile Ala Gly Asn Gly Ile Ser Lys Asp Arg Phe Glu
            340                 345                 350

att att ccc aaa ggc gca agc gtt cct att ggc gat aat tct aaa gaa         1104
Ile Ile Pro Lys Gly Ala Ser Val Pro Ile Gly Asp Asn Ser Lys Glu
        355                 360                 365

gag ggg aag gaa atg aat cgt aga gtg gaa ctt aga atc tat aat tga         1152
Glu Gly Lys Glu Met Asn Arg Arg Val Glu Leu Arg Ile Tyr Asn
    370                 375                 380


<210>  65
<211>  383
<212>  PRT
<213>  Leptospira interrogans serovar Pomona

<400>  65

Met Ala Lys Lys Glu Asn Tyr Tyr Ile Thr Ile Lys Gly Arg Lys Tyr
1               5                   10                  15


Asp Arg Lys Leu Ile Gln Leu Ala Glu Glu Phe Thr Ser Gly Lys Arg
            20                  25                  30


Asp Gly Lys Ile Ser Ile Asn Asp Ala Lys Arg Leu Leu Lys Ile Val
        35                  40                  45


Lys Asp Asn Asn Ala Tyr Thr Asp Ile Glu Lys His Thr Ile Glu Tyr
    50                  55                  60


Ile Arg Glu Asn Tyr Lys Phe Thr Glu Lys Ser Asp Glu Trp Phe Arg
65                  70                  75                  80


Ser Glu Ile Arg Lys Trp Ala Ala Lys Lys Val Gln Glu Ala Lys Lys
                85                  90                  95


Lys Ser Asp Val Glu Ser Ile Leu Val Asp Asp Ser Glu Ala Pro Glu
            100                 105                 110


Ile Asn Phe Pro Ser Ser Trp Gly Glu Asp Lys Thr Glu Val Val Glu
        115                 120                 125


Ile Thr Gln Thr Ser Lys Ile Asp Trp Arg Glu Asn Ser Asn Phe Ser
    130                 135                 140

Ser Ala Thr Ser His Ser Lys Lys Asn Lys Lys Ile Ile Pro Thr Leu
145                 150                 155                 160

Ile Phe Leu Ser Gly Phe Leu Ile Leu Val Gly Leu Val Tyr Phe Phe
                165                 170                 175

Arg Thr Leu Phe Tyr Lys Glu Asp Leu Glu Gln Val Val Lys Thr Asn
            180                 185                 190

Ser Glu Ile Val Ser Asn Ser Lys Glu Lys Gln Ser Asp Val Ser Ile
        195                 200                 205

Glu Lys Ala Glu Ser Thr Lys Glu Val Arg Lys Lys Asn Val Arg Ser
    210                 215                 220

Lys Lys Glu Glu Ser Glu Ile Pro Lys Asn Ala Leu Thr Ile Leu Lys
225                 230                 235                 240

Pro Gln Thr Gly Lys Lys Leu Glu Ser Lys Ser Leu Phe Ser Ser Leu
                245                 250                 255

Thr Asn Gln Asn Ser Thr Glu Glu Phe Ser Ser Asn Pro Gln Phe Arg
            260                 265                 270

Glu Ile Glu Ser Asn Val Ile Arg Phe Glu Lys Asn Ser Ile Gln Ile
        275                 280                 285

His Lys Glu Ser Arg Pro Ser Leu Asn Arg Leu Ala Arg Trp Met Lys
    290                 295                 300

Gln Asp Ser Ser Ile Arg Val Lys Val Ile Gly His Thr Ser Leu Glu
305                 310                 315                 320

Gly Ser Glu Asp Ala Asn Gln Lys Val Ser Leu Leu Arg Ala Gln Thr
                325                 330                 335

Val Arg Asn Tyr Ile Ala Gly Asn Gly Ile Ser Lys Asp Arg Phe Glu
            340                 345                 350

Ile Ile Pro Lys Gly Ala Ser Val Pro Ile Gly Asp Asn Ser Lys Glu
        355                 360                 365

Glu Gly Lys Glu Met Asn Arg Arg Val Glu Leu Arg Ile Tyr Asn
    370                 375                 380

<210>  66
<211>  549
<212>  DNA
<213>  Leptospira interrogans serovar Pomona

<220>
<221>  CDS
<222>  (1)..(549)

<400>  66
atg aaa tat aaa ata att tta att tta tca cta atg tta ttt ctt ttc      48
Met Lys Tyr Lys Ile Ile Leu Ile Leu Ser Leu Met Leu Phe Leu Phe
1               5                   10                  15

gtt tcc tgt ccg gat gaa aaa aaa gaa aat gaa ttg agt act tat att      96
Val Ser Cys Pro Asp Glu Lys Lys Glu Asn Glu Leu Ser Thr Tyr Ile
            20                  25                  30

tta tat agt gtt ctc ata aac gca act act caa tac gat tgt gtt act      144
Leu Tyr Ser Val Leu Ile Asn Ala Thr Thr Gln Tyr Asp Cys Val Thr
        35                  40                  45

agt tcg gaa gta gta tca gac tct tat aac aaa aca aca ata acc ttc      192
Ser Ser Glu Val Val Ser Asp Ser Tyr Asn Lys Thr Thr Ile Thr Phe
    50                  55                  60

gaa aat aaa cct caa tat tac aat tca ccc agt gga aat gta gtt cca      240
Glu Asn Lys Pro Gln Tyr Tyr Asn Ser Pro Ser Gly Asn Val Val Pro
65                  70                  75                  80

aaa gca att atg ccg att ttg att aaa aag ggg cag aca att caa gta      288
Lys Ala Ile Met Pro Ile Leu Ile Lys Lys Gly Gln Thr Ile Gln Val
                85                  90                  95

tcc agt ata acg act aac gtt aag tat gaa gcg aca aac caa gac tta      336
Ser Ser Ile Thr Thr Asn Val Lys Tyr Glu Ala Thr Asn Gln Asp Leu
            100                 105                 110

act ttt ctt ttt aga aaa gat ggt tgt cac ggt aca aac tcc gaa att      384
Thr Phe Leu Phe Arg Lys Asp Gly Cys His Gly Thr Asn Ser Glu Ile
        115                 120                 125

gca acc tat gca gga gct act aat aca aat gtt ttt tta gga aac aca      432
Ala Thr Tyr Ala Gly Ala Thr Asn Thr Asn Val Phe Leu Gly Asn Thr
    130                 135                 140

aat act gtt agc tta act caa ttt aaa ttt acc gcc gac tat aat ggg      480
Asn Thr Val Ser Leu Thr Gln Phe Lys Phe Thr Ala Asp Tyr Asn Gly
145                 150                 155                 160

att ata cta atc gtt ggg aaa aac cta ggt gca agt tta cct gga gat      528
Ile Ile Leu Ile Val Gly Lys Asn Leu Gly Ala Ser Leu Pro Gly Asp
                165                 170                 175

att cgt gtg aat gta ttt taa                                          549
Ile Arg Val Asn Val Phe
            180


<210>  67
<211>  182
<212>  PRT
<213>  Leptospira interrogans serovar Pomona

<400>  67

Met Lys Tyr Lys Ile Ile Leu Ile Leu Ser Leu Met Leu Phe Leu Phe
1               5                   10                  15

Val Ser Cys Pro Asp Glu Lys Lys Glu Asn Glu Leu Ser Thr Tyr Ile
            20                  25                  30

Leu Tyr Ser Val Leu Ile Asn Ala Thr Thr Gln Tyr Asp Cys Val Thr
        35                  40                  45

Ser Ser Glu Val Val Ser Asp Ser Tyr Asn Lys Thr Thr Ile Thr Phe
    50                  55                  60

Glu Asn Lys Pro Gln Tyr Tyr Asn Ser Pro Ser Gly Asn Val Val Pro
65                  70                  75                  80

Lys Ala Ile Met Pro Ile Leu Ile Lys Lys Gly Gln Thr Ile Gln Val
                85                  90                  95

Ser Ser Ile Thr Thr Asn Val Lys Tyr Glu Ala Thr Asn Gln Asp Leu
            100                 105                 110

Thr Phe Leu Phe Arg Lys Asp Gly Cys His Gly Thr Asn Ser Glu Ile
        115                 120                 125

Ala Thr Tyr Ala Gly Ala Thr Asn Thr Asn Val Phe Leu Gly Asn Thr
    130                 135                 140

Asn Thr Val Ser Leu Thr Gln Phe Lys Phe Thr Ala Asp Tyr Asn Gly
145                 150                 155                 160

Ile Ile Leu Ile Val Gly Lys Asn Leu Gly Ala Ser Leu Pro Gly Asp
                165                 170                 175

Ile Arg Val Asn Val Phe
            180